(19) Europäisches Patentamt
European Patent Office
Office européen des brevets

(11) EP 3 788 039 B1

(12) EUROPEAN PATENT SPECIFICATION

(45) Date of publication and mention of the grant of the patent:
**11.02.2026 Bulletin 2026/07**

(21) Application number: **19721369.7**

(22) Date of filing: **29.04.2019**

(51) International Patent Classification (IPC):
***C07D 403/12*** (2006.01) ***C07D 401/14*** (2006.01)
***C07D 401/12*** (2006.01) ***C07D 403/14*** (2006.01)
***C07D 405/12*** (2006.01) ***C07D 241/04*** (2006.01)
***C07D 471/04*** (2006.01) ***C07D 211/14*** (2006.01)
***A61P 7/02*** (2006.01) ***A61K 31/496*** (2006.01)

(52) Cooperative Patent Classification (CPC):
**C07D 241/04; A61P 7/02; C07D 211/14; C07D 401/12; C07D 401/14; C07D 403/12; C07D 403/14; C07D 405/12; C07D 471/04**

(86) International application number:
**PCT/GB2019/051180**

(87) International publication number:
**WO 2019/211585 (07.11.2019 Gazette 2019/45)**

(54) **FACTOR XIIA INHIBITORS**

FAKTOR-XIIA-INHIBITOREN

INHIBITEURS DU FACTEUR XIIA

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2018 GB 201807014**

(43) Date of publication of application:
**10.03.2021 Bulletin 2021/10**

(73) Proprietor: **University of Leeds**
**Leeds LS2 9JT (GB)**

(72) Inventors:
- **PHILIPPOU, Helen**
  **Leeds LS2 9JT (GB)**
- **FOSTER, Richard**
  **Leeds LS2 9JT (GB)**
- **FISHWICK, Colin**
  **Leeds LS2 9JT (GB)**
- **REVILL, Charlotte**
  **Leeds LS2 9JT (GB)**
- **YULE, Ian**
  **Leeds LS2 3AA (GB)**
- **TAYLOR, Roger**
  **Leeds LS2 3AA (GB)**
- **NAYLOR, Alan**
  **Cambridgeshire CB22 7QJ (GB)**
- **FALLON, Philip Spencer**
  **Saffron Walden Essex CB10 1XL (GB)**
- **CROSBY, Stuart**
  **Saffron Walden Essex CB10 1XL (GB)**
- **HOPKINS, Anna**
  **Saffron Walden Essex CB10 1XL (GB)**
- **STEWART, Mark Richard**
  **Saffron Walden Essex CB10 1XL (GB)**
- **WINFIELD, Natalie Louise**
  **Saffron Walden Essex CB10 1XL (GB)**

(74) Representative: **HGF**
**HGF Limited**
**4th Floor, 1 City Square**
**Leeds LS1 2AL (GB)**

(56) References cited:
**WO-A1-2012/083436 WO-A1-2018/093695**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

This invention relates to compounds and methods of treatment (or prevention) using the compounds. The invention also relates to processes and methods for producing the compounds of the invention. The compounds of the invention are modulators of Factor XII (e.g. Factor XIIa). In particular, the compounds are inhibitors of Factor XIIa and may be useful as anticoagulants. The references to method of treatment in this description are to be interpreted as references to compounds, pharmaceutical compositions and medicaments of the present invention for use in a method of treatment of the human (or animal) body by therapy (or for diagnosis).

## BACKGROUND

Cardiovascular disease is the leading cause of death in the developed world, affecting millions of people worldwide every year. The disease is generally caused by atherosclerosis of the arterial wall, which develops over many years and is characterised by inflammation of the endothelium, sub-endothelial lipid deposition, macrophage infiltration and plaque development. In the acute phase of the disease, the atherosclerotic plaque becomes unstable and ruptures, triggering thrombosis. The development of a thrombus (blood clot) that occludes the blood vessel and consequently deprives the tissue of oxygen constitutes the main precipitating event leading to morbidity and mortality. Blood clot formation is initiated by activation and aggregation of platelets. The platelet plug is consolidated by the activation of coagulation and formation of a fibrin network. Arterial occlusion by the thrombus leads to tissue death downstream, and, depending upon where this occurs, is associated with the development of myocardial infarction, stroke or claudication.

Thrombosis in the venous circulation has a different aetiology as it does not depend on atherosclerosis, but is triggered by circulatory stasis due to immobilisation and is often associated with naturally occurring deficiencies of coagulation inhibitors (e.g. antithrombin, protein C and S) and with surgical procedures. Venous thrombosis usually occurs in the leg or arm (deep vein thrombosis, DVT) and can lead to emboli (thrombus fragments) blocking downstream smaller vessels particularly in the lung (pulmonary embolism, PE). Other triggers of DVT include cancer, nephrotic syndrome, antiphospholipid syndrome and heart failure.

Thrombosis is a very serious condition and is associated with up to 25,000 and 200,000 fatalities in the UK alone on an annual basis for venous and arterial thrombosis respectively. In January 2010, the UK National Institute for Health and Clinical Excellence (NICE) published new guidelines to increase screening for early signs of thrombosis in patients admitted to hospital.

Current medications to treat or prevent thrombosis target either the platelet or coagulation. Generally, antiplatelet drugs are used in the prevention of arterial disease, whereas anticoagulants are used in the prevention of stroke in patients with atrial fibrillation, DVT and PE. The largest clinical problem associated with current anticoagulant use is the risk of bleeding. As many as 1 to 3% of patients experience major bleeding or 15-18% patients experience minor bleeding whilst on anticoagulation therapy, dependent upon patient group and choice of anticoagulation.

Warfarin and heparin (encompassing all of its derivatives) are the most commonly used anticoagulant drugs. Warfarin, the oldest approved long-term oral anticoagulant, requires regular monitoring via prothrombin time (PT) clotting assays to determine optimal dosage, which places a major burden on the healthcare system and patient quality of life. Warfarin is non-specific and targets several coagulation enzymes, whereas heparin, which is administered subcutaneously or intravenously, targets activated factor X (FXa) and/or thrombin depending on its molecular weight. Furthermore, the new oral anticoagulants (NOACs) on the market or in development that target thrombin or FXa, also carry a significant risk of bleeding which is comparable to that of heparin and warfarin with the exception of intracranial haemorrhage where NOACs have better outcome than warfarin. However, gastrointestinal bleeding is increased with NOACs compared with low-molecular-weight heparin and vitamin K antagonist that encompasses warfarin (New Oral Anticoagulants Increase Risk for Gastrointestinal Bleeding: A Systematic Review and Meta-analysis Holster IL, Valkhoff VE, Kuipers EJ, Tjwa ET Gastroenterology. 2013 Jul;145(1):105-112.

Therefore, there is a large unmet clinical need for a novel anticoagulant that is not associated with bleeding. This goal has been an aspiration for the field for more than 6 decades. However, it was always assumed that anticoagulation leads to an unavoidable risk of bleeding because the mechanisms involved in thrombosis were considered the same as those involved in haemostasis.

Factor XII (FXII) was identified 50 years ago as a coagulation protein in the intrinsic pathway of blood coagulation as FXII deficient patients had marked prolongation of the *in vitro* surface-activated coagulation time. However, series of investigations have convincingly shown that FXII has no role in normal haemostasis. Evidence within the last decade has identified FXII as essential for thrombus formation *in vivo* (Renne T, Pozgajova M, Gruner S, Schuh K, Pauer HU, Burfeind P, Gailani D, Nieswandt B. Defective thrombus formation in mice lacking coagulation factor XII. J Exp Med 2005;202:271-281; Kleinschnitz C, Stoll G, Bendszus M, Schuh K, Pauer HU, Burfeind P, Renne C, Gailani D, Nieswandt B, Renne T. Targeting coagulation factor XII provides protection from pathological thrombosis in cerebral ischemia without interfering with hemostasis. J Exp Med 2006;203:513-518; Renne T, Nieswandt B, Gailani D. The intrinsic pathway of

coagulation is essential for thrombus stability in mice. Blood Cells Mol Dis 2006;36:148-151; Hagedorn I, Schmidbauer S, Pleines I, Kleinschnitz C, Kronthaler U, Stoll G,Dickneite G, Nieswandt B. Factor XIIa inhibitor recombinant human albumin Infestin-4 abolishes occlusive arterial thrombus formation without affecting bleeding. Circulation 2010;121:1510-1517 and Matafonov A, Leung PY, Gailani AE, Grach SL, Puy C, Cheng Q, Sun MF, McCarty OJ, Tucker EI, Kataoka H, Renné T, Morrissey JH, Gruber A, Gailani D. Factor XII inhibition reduces thrombus formation in a primate thrombosis model. Blood. 2014;13;123(11):1739-46). A unique characteristic of FXII is that its deficiency does not incur bleeding, unlike deficiencies in all other coagulation factors. Therefore, FXIIa is a highly attractive target for the discovery of an anticoagulant with the potential for a greatly improved safety profile.

Recent studies have challenged dogma in the haemostasis and thrombosis field by demonstrating novel mechanisms in thrombosis involving FXII. These studies provide clear evidence that FXII is necessary for thrombus development whilst not playing a role in haemostasis. FXII deficient mice were remarkably protected against thrombosis when challenged with collagen and epinephrine infusion, whilst showing no prolongation of bleeding time during surgery or tail-clipping. Similar protection against thrombosis was observed in mesenteric arterioles exposed to $FeCl_3$ and in the aorta after mechanical injury. Infusion of human FXII in these models restored the development of thrombi. The ground breaking nature of these findings is illustrated by the debate on FXII function and the role of the contact coagulation pathway activated by FXIIa that, until recently, dominated the field. This debate was fuelled by the fact that FXII deficiency does not lead to bleeding whereas deficiency in every other coagulation protease does, which led to the belief that FXII was not required for physiological coagulation and that FXII activation was an *in vitro* phenomenon.

However, recent studies have shown that FXII is activated by negatively charged surfaces and the surface of activated platelets (Zakharova et al, PLoS One. 2015 Feb 17;10(2):e0116665). These *in vivo* and *in vitro* studies demonstrate that FXII plays a hitherto unrecognised role in thrombosis. The generation of FXIIa stabilises the thrombus through enhanced thrombin generation, fibrin deposition and direct prothrombotic effects on fibrin structure. This mechanism does not appear to play a role in normal haemostasis, since FXII deficiency is phenotypically silent in humans as well as mice, making FXII an ideal target for the development of a new anticoagulant to treat thrombosis.

The effectiveness of FXII deficiency in reducing thrombosis has been shown in several different *in vivo* thrombosis models. In addition to the models mentioned above, the role of FXII in thrombosis has been demonstrated in a murine model of thrombosis induced by ligation of the carotid artery and a murine model of cerebral microvascular thrombosis secondary to transient occlusion of the middle cerebral artery. Brain infarct sizes were significantly reduced in FXII deficient mice and restored to large infarcts by the infusion of human FXII. Inhibition of FXII has also been shown to reduce risk of venous thrombosis. One study has demonstrated that a Kunitz-type inhibitor of contact activation isolated from the tick salivary glands (Ir-CPI) effectively reduces thrombosis in mouse and rat models of venous thrombosis induced by vessel ligation. This inhibitory protein was also effective in reducing PE in a murine model induced by infusion with collagen and epinephrine, and in a murine model of dorsal skin arteriole thrombosis. Again, there was no effect on bleeding time in the animals treated with Ir-CPI. Inhibition of FXIIa with H-D-Pro-Phe-Arg-chloromethylketone (PCK) has also been shown to protect against thrombosis. These studies provide preclinical proof of concept that inhibition of FXIIa is efficacious in the treatment of thrombosis.

More recently, Magnus Larsson et al., "A Factor XIIa Inhibitory Antibody Provides Thromboprotection in Extracorporeal Circulation Without Increasing Bleeding Risk" Sci Transl Med 6, 222ra17 (2014); demonstrated that recombinant fully human antibody 3F7 binds into the FXIIa enzymatic pocket. 3F7 interfered with FXIIa-mediated coagulation, abolished thrombus formation under flow, and blocked experimental thrombosis in mice and rabbits. In rabbits 3F7 provided thromboprotection as efficiently as heparin, but unlike heparin, 3F7 treatment did not impair the haemostatic capacity and did not increase bleeding from wounds. Larsson concludes that targeting of FXIIa is a safe mode of thromboprotection in bypass systems, and provides a clinically relevant anticoagulation strategy that is not complicated by excess bleeding.

Dabigatran, apixaban and rivaroxaban, are approved for short-term use as oral FXa/thrombin inhibitors, respectively. Dabigatran is 3-({2-[(4-carbamimidoyl- phenylamino)-methyl]-1-methyl-1H-benzoimidazole-5-carbonyl}-pyridin-2-yl-amino)-propionic acid;

Dabigatran is also approved for long term prevention of stroke in patients with atrial

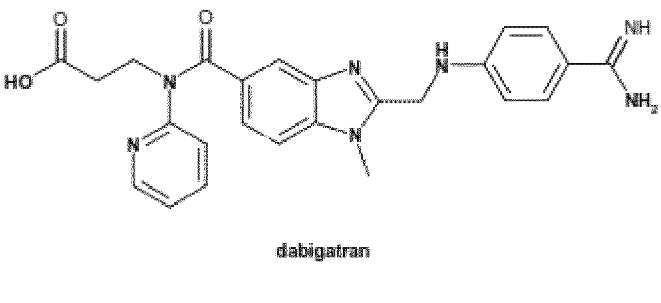

dabigatran

fibrillation (AF) and is described in US Patent No. 6,087,380.

Rivaroxaban is (S)-5-chloro-N-{[2-oxo-3-[4-(3-oxomorpholin-4-yl)phenyl]oxazolidine -5-yl]methyl} thiophene-2-carboxamide;

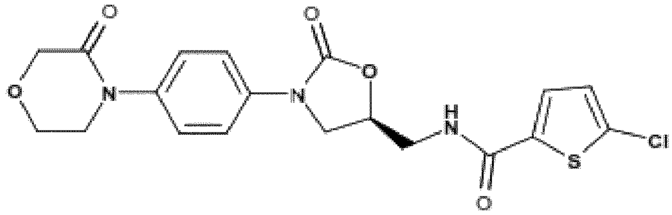

rivaroxaban

Rivaroxaban is also approved for reducing stroke risk in patients with nonvalvular AF. Rivaroxaban has shown superiority of once-daily rivaroxaban over warfarin in protecting AF patients from stroke and non-CNS systemic embolism. Rivaroxaban also demonstrates comparable major and non-major clinically relevant bleeding, as well as significantly lower rates of intracranial haemorrhage vs. warfarin. Rivaroxaban, is described in US Patent No. 7,157,456.

Apixaban is also factor Xa inhibitor approved for use in preventing stroke and systemic embolism in patients with nonvalvular atrial fibrillation.

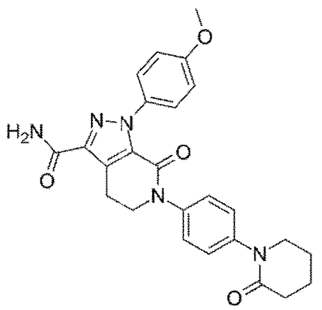

apixaban

Apixaban is 1-(4-methoxyphenyl)-7-oxo-6-[4-(2-oxopiperidin-1-yl)phenyl]-4,5-dihydropyrazolo[5,4-c]pyridine-3-carboxamide:

Apixaban is described in US Patent No. 6,413,980.

Edoxaban is N'-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide;

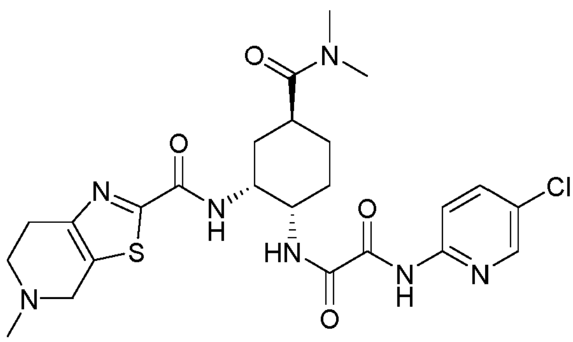

edoxaban

Edoxaban is another factor Xa inhibitor approved for use in preventing stroke and systemic embolism in patients with nonvalvular atrial fibrillation and for the treatment of deep vein thrombosis. Edoxaban is described in US Patent No. 7,365,205.

Betrixaban is N-(5-chloropyridin-2-yl)-2-[4-(N,N-dimethylcarbamimidoyl)benzamido]-5-methoxybenzamide:

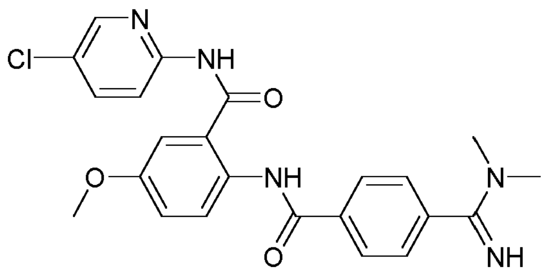

betrixaban

Betrixaban is a factor Xa inhibitor approved for use in preventing venous thromboembolism in patients with moderate to severe restricted mobility. Betrixaban is described in US Patent No. US 6,376,515.

WO 2012/083436 relates to thrombin and factor Xa inhibitors that are useful as anticoagulant agents as a result of their selective dual inhibition of thrombin and prothrombinase.

Recent surveys of the cardiovascular pipelines of major pharmaceutical companies have not revealed any oral inhibitors of FXIIa in development. Infestin-4 is a biological agent produced by CSL Behring that targets FXIIa, and shows efficacy in a FeCl3-induced model of thrombosis in mice and rabbits. Other antibody approaches targeting FXII(a) have also shown *in vivo* efficacy. However, if infestin-4 or the antibody approaches were successful, they would require intravenous administration, which makes them less suitable for long term anticoagulation.

As FXII deficiency in humans is asymptomatic, unlike other coagulation factor deficiencies that cause bleeding and that deficiency or inhibition of the activity of FXII show an anticoagulant effect, a selective FXIIa inhibitor, has the potential to reduce bleeding risk associated with currently available anticoagulant therapies.

It is an aim of aspects of the present invention to at least partially mitigate the problems associated with the prior art.

It is an aim of certain embodiments of the present invention to provide compounds that inhibit FXII activity, in particular FXIIa activity, for example the serine protease activity of FXIIa.

It is an aim of certain embodiments of the present invention to provide compounds that possess physicochemical and pharmacokinetic properties consistent with the potential for oral bioavailability.

It is an aim of certain embodiments of this invention to provide compounds which exhibit reduced cytotoxicity or increased solubility relative to prior art compounds and existing therapies.

Another aim of certain embodiments of this invention is to provide compounds having a convenient pharmacokinetic profile and a suitable duration of action following dosing. A further aim of certain embodiments of this invention is to provide compounds in which the metabolised fragment or fragments of the drug after absorption are GRAS (Generally Regarded As Safe).

Certain embodiments of the present invention satisfy some or all of the above aims.

## BRIEF SUMMARY OF THE DISCLOSURE

In accordance with the present invention there is provided a compound according to formula (I) and pharmaceutically acceptable salts thereof:

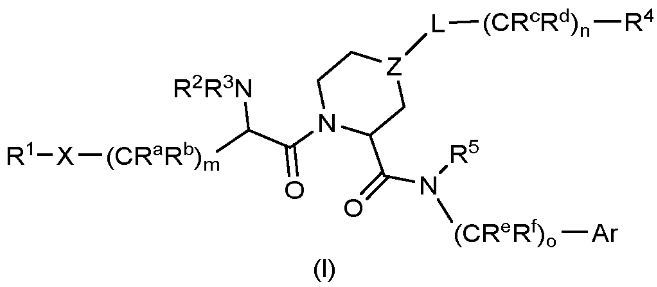

(I)

wherein

Z is either N or $CR^{4a}$;

X is either a bond, -C(O)NH-, -C(O)O- or -C(O)-;

L is selected from: bond, -O-, -C(O)O-, $-NR^6-$, $-C(O)NR^7-$, and $-SO_2-$;

Ar is a substituted or unsubstituted 9 to 10 membered bicyclic heteroaromatic ring system, wherein the bicyclic heteroaromatic ring system is an aromatic hydrocarbon ring system with at least one heteroatom within the ring system selected from O, N and S or a bicyclic ring system which is not completely aromatic but contains an aromatic ring and wherein the at least one heteroatom is present within the aromatic ring or the non-aromatic ring, wherein, when substituted, Ar is substituted with 1, 2, or 3 substituents selected from: halo, $C_{1-6}$ alkyl, $-OR^g$, $-NR^gR^h$ or $C_{1-4}$ alkyl substituted by $-NR^gR^h$;

m is selected from 0, 1, 2, or 3;

n is selected from 0, 1, 2, 3, or 4;

o is selected from 1 or 2;

R' is selected from substituted or unsubstituted: $-NR^8R^9$, 5 to 10 membered carbocyclic ring system or a 5 to 10 membered heterocyclic ring system;
wherein when substituted $R^1$ is substituted with 1, 2, or 3 groups selected from: =O, CN, - OH, or $-O-C_{1-6}$ alkyl, halo, $C_{1-6}$ alkyl and $C_{1-6}$ haloalkyl;

$R^2$ is selected from: H, $C_{1-6}$ alkyl, $C_{1-6}$ haloalkyl, phenyl, benzyl, $-C(O)R^{2a}$, and $-S(O_2)R^{2a}$;
wherein $R^{2a}$ is selected from: $C_{1-6}$ alkyl, phenyl, and benzyl;

$R^3$ is:

(a) H or $C_{1-6}$ alkyl; or

(b) $R^3$ together with one of $R^a$ or $R^b$ forms a bond, $-CH_2-$ or $-CH_2CH_2-$ group resulting in a 4, 5 or 6 membered heterocycloalkyl ring comprising the $-CH_2-$ or $-CH_2CH_2-$ group, the N atom to which $R^3$ is attached, the C atom to which $R^a$ or $R^b$ are attached, and any intervening atoms; or

(c) $R^3$ forms a bond, $-CH_2-$ or $-CH_2CH_2-$ group with an atom of $R^1$ when $R^1$ is a carbocyclic ring system or a heterocyclic ring system;

$R^4$ is selected from: H, $=CH_2$, -CN, halo, $C_{1-4}$ alkyl, $C_{1-4}$ haloalkyl, $-OR^{10}$, $-NR^{10}R^{11}$, 6 to 10 membered aryl, 5 to 10 membered heteroaryl, wherein the 6 to 10 membered aryl or 5 to 10 membered heteroaryl group is unsubstituted or substituted with 1, 2 or 3 $R^{12}$;

$R^{4a}$ is selected from: H, -OH, halo or $C_{1-4}$ alkyl;

$R^5$ is H or $C_{1-6}$ alkyl;

$R^6$ is H, $C_{1-6}$ alkyl or $-C(O)C_{1-6}$ alkyl;

$R^7$ is H or $C_{1-6}$ alkyl;

$R^8$ and $R^9$ are independently at each occurrence selected from: H, $C_{1-6}$ alkyl, $C_{3-6}$ cycloalkyl, phenyl, $C_{1-4}$ alkyl substituted with $-OR^i$, or $C_{1-4}$ alkyl substituted with phenyl, or $R^8$ and $R^9$ taken together with the atom to which they are attached form 3 to 8 membered heterocycloalkyl ring, which is unsubstituted or substituted with: CN, halo, $C_{1-6}$ alkyl or $-OR^i$;

$R^{12}$ is independently at each occurrence selected from: halo, $C_{1-4}$ alkyl, $C_{1-4}$ haloalkyl, $-OR^{13}$, -CN, - $C(O)R^{10}$, =O,

$SO_2R^{10}$, benzyl, phenyl, unsubstituted 5 or 6 membered heteroaryl, or methyl substituted 5 or 6 membered heteroaryl;

$R^{10}$ and $R^{11}$ are independently at each occurrence selected from: H and $C_{1-4}$ alkyl;

$R^{13}$ is selected from: H, $C_{1-4}$ alkyl, $C_{1-4}$ haloalkyl, phenyl or benzyl;

$R^a$ and $R^b$ are independently at each occurrence selected from: H, $C_{1-4}$ alkyl, $-OR^j$ or one of $R^a$ or $R^b$ together with $R^3$ forms a bond, $-CH_2-$ or $-CH_2CH_2-$ group resulting in a 4, 5 or 6 membered heterocycloalkyl ring comprising the $-CH_2-$ or $-CH_2CH_2-$ group, the N atom to which $R^3$ is attached, the C atom to which $R^a$ or $R^b$ are attached, and any intervening atoms; and

$R^c$, $R^d$, $R^e$, $R^f$, $R^g$, $R^h$, $R^i$ and $R^j$ are independently at each occurrence selected from: H and $C_{1-4}$ alkyl.

In embodiments where $R^3$ is option (a) or option (b) then m is not 0 when X is a bond. In embodiments where $R^3$ is option (c) then m may be 0 when X is a bond.
In embodiments where $R^4$ is 6 to 10 membered aryl, or 5 to 10 membered heteroaryl then n is not 0 when L is -C(O)O-. In embodiments n is not 0 when L is -C(O)O-. In embodiments n is 1, 2, 3 or 4.
The compound according to formula (I) may be a compound of formula (Ia) and pharmaceutically acceptable salts thereof:

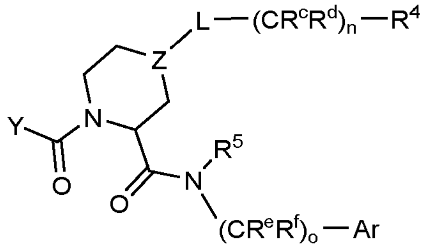

(Ia)

wherein

Y is selected from:

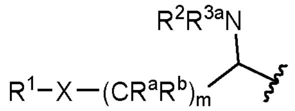

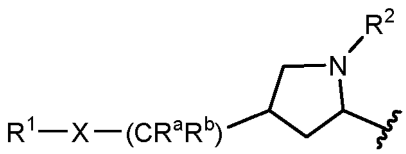

and

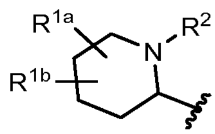

$R^{1a}$ and $R^{1b}$ taken together form a substituted or unsubstituted: 5 or 6 membered heteroaromatic ring or a phenyl ring; wherein when the ring formed from $R^{1a}$ and $R^{1b}$ is substituted it is substituted with 1, 2, or 3 $R^z$ groups wherein $R^z$ is independently selected at each occurrence from: =O, CN, -OH, or $-O-C_{1-6}$ alkyl, halo and $C_{1-6}$ alkyl;

$R^{3a}$ is H or $C_{1-6}$ alkyl; and

m is selected from 1, 2, or 3.

In embodiments $R^{1a}$ and $R^{1b}$ are substituted on adjacent atoms. Accordingly, Y may be selected from:

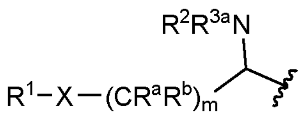

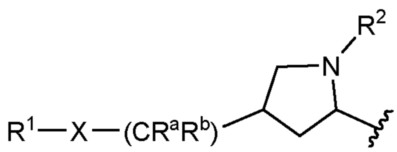

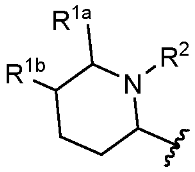
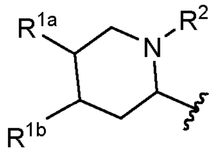
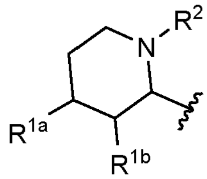

In embodiments Y is selected from:

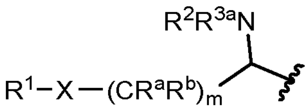
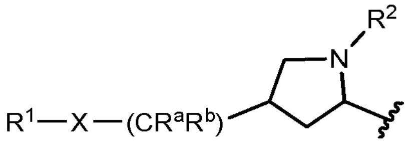
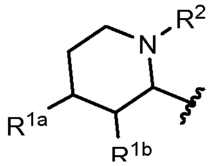

In embodiments $R^3$ is:

(a) H or $C_{1-6}$ alkyl; or

(b) $R^3$ together with one of $R^a$ or $R^b$ forms a bond, $-CH_2-$ or $-CH_2CH_2-$ group resulting in a 4, 5 or 6 membered heterocycloalkyl ring comprising the $-CH_2-$ or $-CH_2CH_2-$ group, the N atom to which $R^3$ is attached, the C atom to which $R^a$ or $R^b$ are attached, and any intervening atoms.

The compound according to formula (I) and pharmaceutically acceptable salts thereof may have the following definition of groups singly or in combination:
wherein

Z is either N or CH;

X is either a bond or -C(O)-;

L is selected from: bond, -O-, or -C(O)O-;

Ar is selected from a substituted or unsubstituted 9 to 10 membered bicyclic heteroaromatic ring system, wherein the bicyclic heteroaromatic ring system is an aromatic hydrocarbon ring system with at least one heteroatom within the ring system selected from O, N and S or a bicyclic ring system which is not completely aromatic but contains an aromatic ring and wherein the at least one heteroatom is present within the aromatic ring or the non-aromatic ring, wherein, when substituted, Ar is substituted with 1, 2, or 3 substituents selected from: halo, $C_{1-6}$ alkyl, $-OR^g$, $-NR^gR^h$ or $C_{1-4}$ alkyl substituted by $-NR^gR^h$;

m is selected from 1, 2, or 3;

n is selected from 0, 1, or 2;

o is selected from 1 or 2;

$R^1$ is selected from substituted or unsubstituted: phenyl or a 5 or 6 membered heterocycloalkyl ring system;
wherein when substituted $R^1$ is substituted with 1, 2, or 3 groups selected from: =O, CN, - OH, or $-O-C_{1-6}$ alkyl, halo, $C_{1-6}$ alkyl and $C_{1-6}$ haloalkyl;

$R^2$ is selected from: H, $C_{1-6}$ alkyl, $C_{1-6}$ haloalkyl, phenyl, benzyl, $-C(O)R^{2a}$, and $-S(O_2)R^{2a}$;
wherein $R^{2a}$ is selected from: $C_{1-6}$ alkyl, phenyl, and benzyl;

$R^3$ is:

(a) H or $C_{1-6}$ alkyl; or

(b) $R^3$ together with one of $R^a$ or $R^b$ forms a bond, $-CH_2-$ or $-CH_2CH_2-$ group resulting in a 4, 5 or 6 membered heterocycloalkyl ring comprising the $-CH_2-$ or $-CH_2CH_2-$ group, the N atom to which $R^3$ is attached, the C atom to which $R^a$ or $R^b$ are attached, and any intervening atoms;

$R^4$ is selected from: $C_{1-4}$ alkyl, 6 to 10 membered aryl, 5 to 10 membered heteroaryl, wherein the aryl or heteroaryl group is unsubstituted or substituted with 1, 2 or 3 $R^{12}$;

$R^{4a}$ is selected from: H, -OH, halo or $C_{1-4}$ alkyl;

$R^5$ is H or $C_{1-6}$ alkyl;

$R^6$ is H, $C_{1-6}$ alkyl or $-C(O)C_{1-6}$ alkyl;

$R^7$ is H or $C_{1-6}$ alkyl;

$R^B$ and $R^9$ are independently at each occurrence selected from: H, $C_{1-6}$ alkyl, $C_{3-6}$ cycloalkyl, phenyl, $C_{1-4}$ alkyl substituted with $-OR^i$, or $C_{1-4}$ alkyl substituted with phenyl, or $R^8$ and $R^9$ taken together with the atom to which they are attached form 3 to 8 membered heterocycloalkyl ring, which is unsubstituted or substituted with: CN, halo, $C_{1-6}$ alkyl or $-OR^i$;

$R^{12}$ is independently at each occurrence selected from: halo, $C_{1-4}$ alkyl, or $-OR^{13}$;

$R^{13}$ is selected from: H, or $C_{1-4}$ alkyl;

$R^a$ and $R^b$ are independently at each occurrence selected from: H, $C_{1-4}$ alkyl, $-OR^j$ or one of $R^a$ or $R^b$ together with $R^3$ forms a bond, $-CH_2-$ or $-CH_2CH_2-$ group resulting in a 4, 5 or 6 membered heterocycloalkyl ring comprising the $-CH_2-$ or $-CH_2CH_2-$ group, the N atom to which $R^3$ is attached, the C atom to which $R^a$ or $R^b$ are attached, and any intervening atoms; and

$R^c$, $R^d$, $R^e$, $R^f$, $R^g$, $R^h$, $R^i$ and $R^i$ are independently at each occurrence selected from: H and $C_{1-4}$ alkyl.

In embodiments Z is N or CH.

In embodiments Y is selected from:

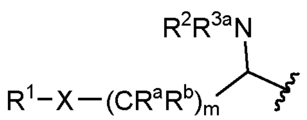

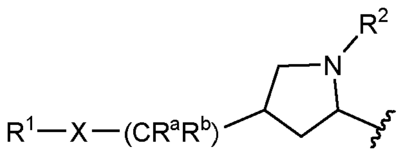
.

In embodiments m is 2 or 3. Alternatively m is 1 or 2.

In embodiments X is either a bond or -C(O)-.

In an embodiment the compound of formula (I) is a compound according to formulae (IIa) or (IIb):

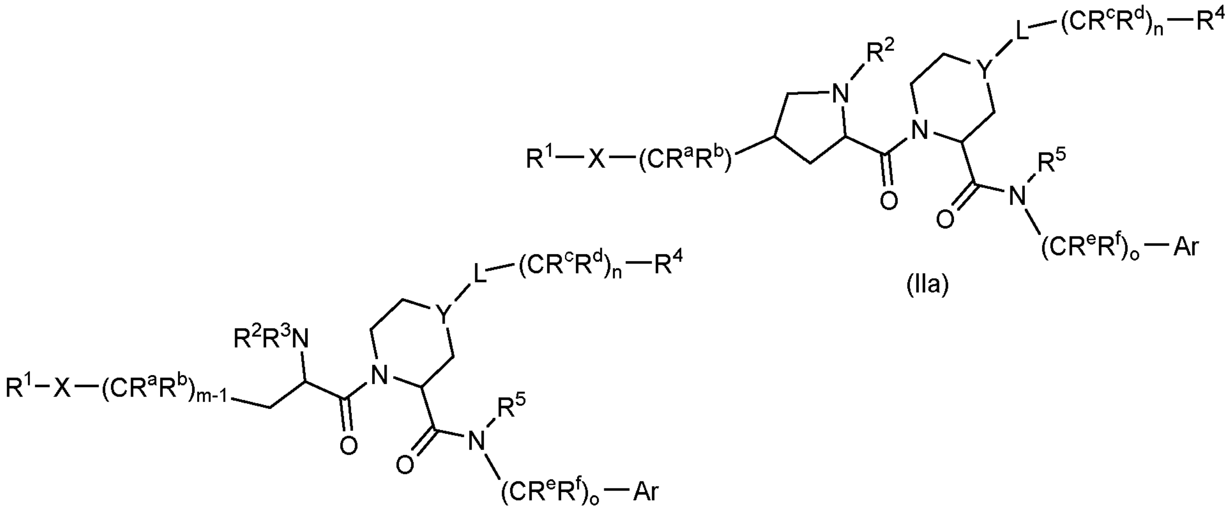

(IIa)

(IIb)

In embodiments of formula (IIa) X is a bond.

Accordingly, in embodiments the compound of formula (I) is a compound according to formula (III):

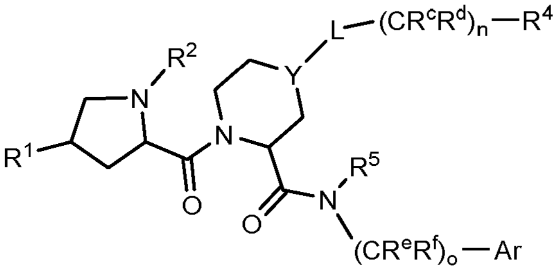

(III)

In embodiments of formula (IIb) m is 1 or 2. In embodiments of formula (IIb) X is either a bond, -C(O)NH-, or -C(O)-, preferably X is -C(O)-.

In embodiments $R^z$ is selected from OH, Cl or OMe.

Accordingly, in embodiments the compound of formula (I) may be a compound according to formula (IV):

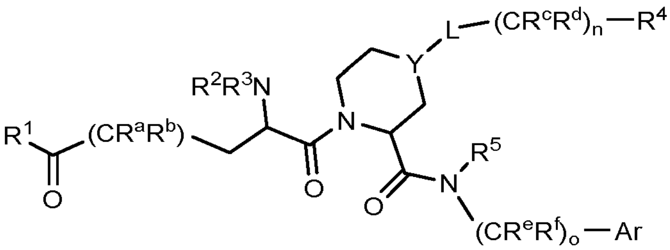

(IV)

In embodiments o is 1. In embodiments $R^e$ and $R^f$ are H.

In preferred embodiments o is 1 and $R^e$ and $R^f$ are H. Accordingly, in an embodiment the compound of formula (I) is a compound according to formula (V) or (Va):

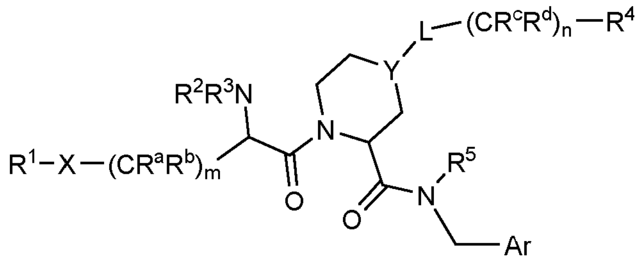

(V)

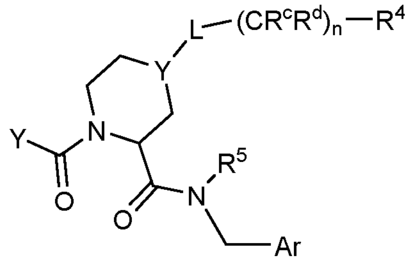

(Va)

In embodiments $R^2$ is H. In embodiments $R^3$ is H. In embodiments $R^{4a}$ is H. In embodiments $R^5$ is H. In embodiments $R^2$, $R^3$, $R^{4a}$ and $R^5$ are each H. In embodiments $R^a$ and $R^b$ are each H. In embodiments $R^2$, $R^3$, $R^5$, $R^{4a}$, $R^a$ and $R^b$ are each H.

In embodiments the compound of formula (I) may be a compound according to formulae (VIa) or (VIb):

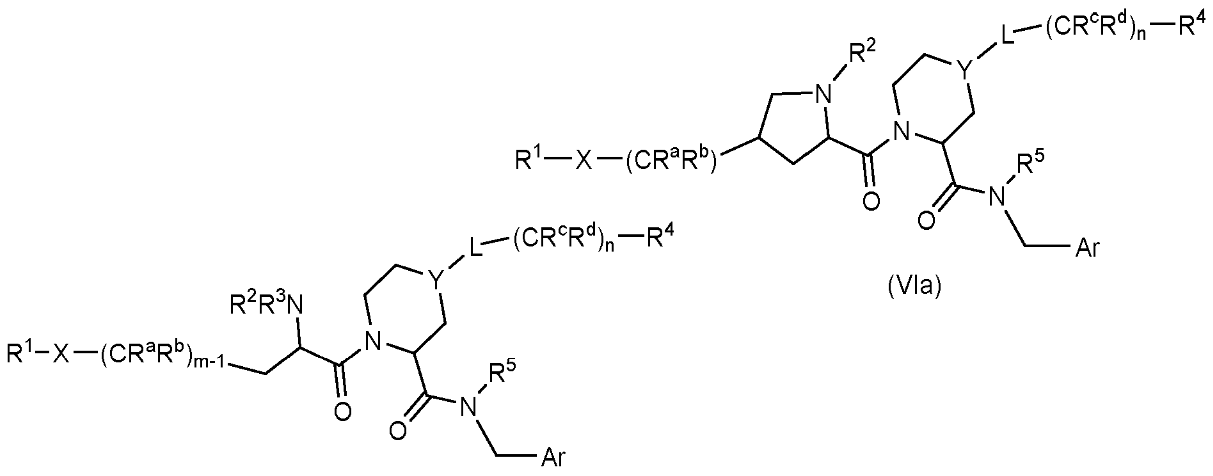

(VIa)

(VIb)

In embodiments of compounds of formulae (VIa) $R^2$ and $R^5$ are each H. In embodiments of compounds of formulae (VIa) $R^a$ and $R^b$ are each H. In embodiments of compounds of formulae (VIa) $R^2$, $R^5$, $R^a$ and $R^b$ are each H.

In embodiments of compounds of formulae (VIb) $R^2$, $R^3$ and $R^5$ are each H. In embodiments of compounds of formulae (VIb) $R^a$ and $R^b$ are each H. In embodiments of compounds of formulae (VIb) $R^2$, $R^3$, $R^5$, $R^a$ and $R^b$ are each H.

In embodiments the compound of formula (I) may be a compound according to formulae (VIIa) or (VIIb):

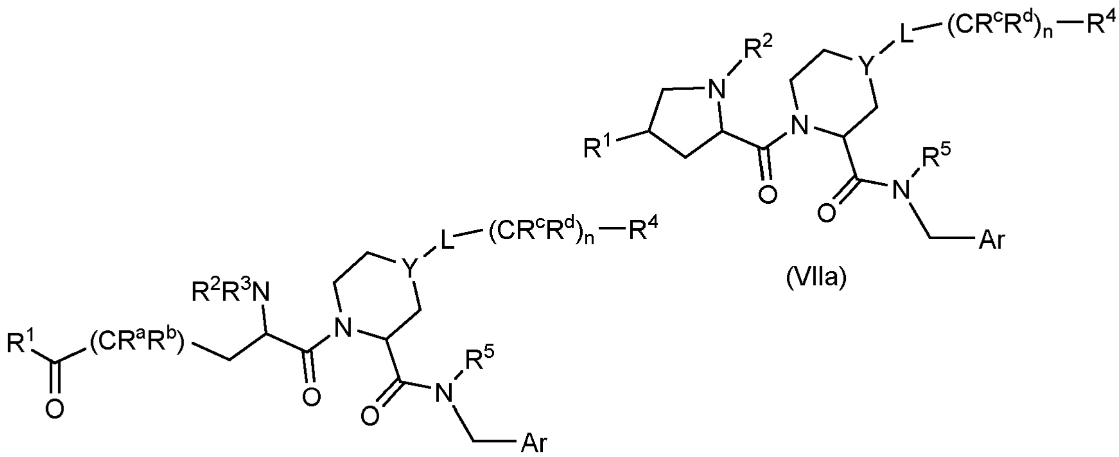

(VIIa)

(VIIb)

In embodiments of compounds of formulae (VIIa) $R^2$ and $R^5$ are each H.

In embodiments of compounds of formulae (VIIb) $R^2$, $R^3$ and $R^5$ are each H. In embodiments of compounds of

formulae (VIIb) $R^a$ and $R^b$ are each H. In embodiments of compounds of formulae (VIIb) $R^2$, $R^3$, $R^5$, $R^a$ and $R^b$ are each H.

In embodiments $R^{4a}$ may be selected from H, OH or F. Preferably, $R^{4a}$ is H.

In embodiments the compound of formula (I) is a compound according to formulae (VIIIa) or (VIIIb):

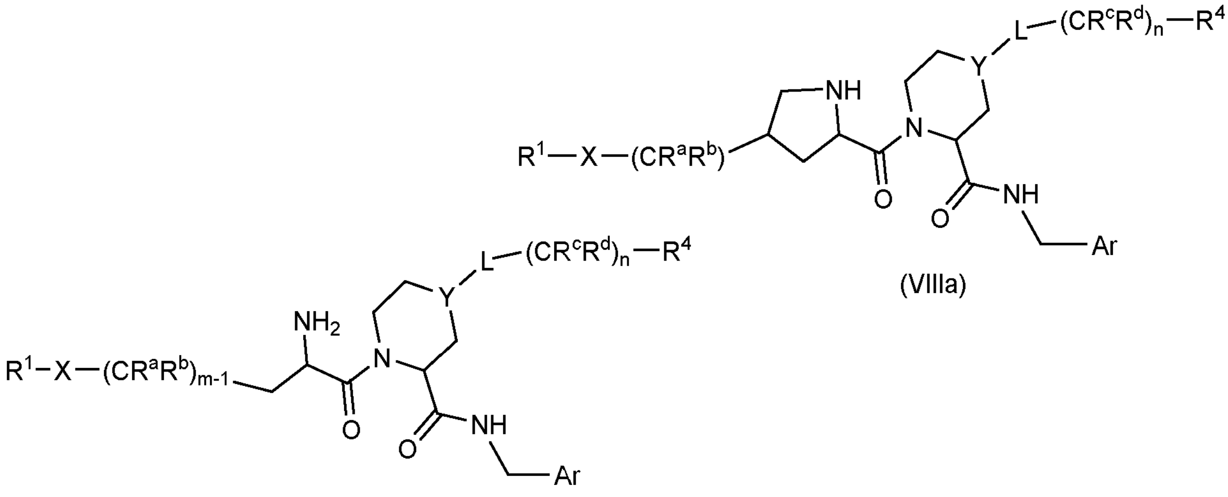

(VIIIa)

(VIIIb)

In embodiments Ar is unsubstituted or substituted with methyl, chloro, -OMe, $-NH_2$ or $-CH_2NH_2$.

$R^g$ and $R^h$ may be independently at each occurrence selected from: H and methyl.

In embodiments Ar is selected from benzotriazole, imidazopyridine, pyridofuran, pyrrolopyridine, azaindole, benzopyrazole, pyridoazathiophene, benzoxazole, benzimidiazole, quinoline, and isoquinoline wherein Ar is unsubstituted or substituted with methyl, chloro, -OMe, $-NH_2$ or $-CH_2NH_2$.

In embodiments Ar is selected from benzotriazole, imidazopyridine, pyridofuran, pyrrolopyridine, azaindole, benzopyrazole, pyridoazathiophene, benzoxazole, wherein Ar is unsubstituted or substituted with methyl, chloro, -OMe, $-NH_2$ or $-CH_2NH_2$.

In embodiments Ar is selected from phenyl and pyridyl and from:

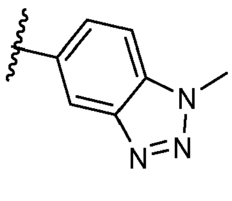 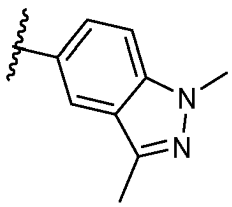 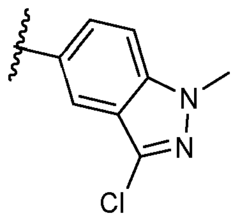 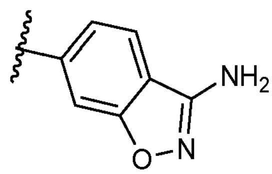

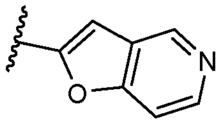 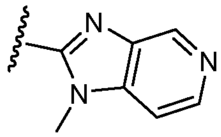 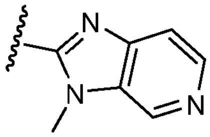 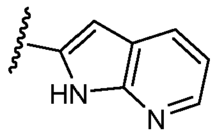

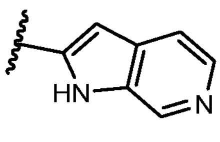 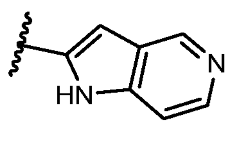 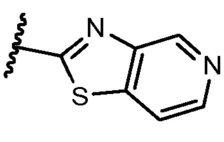 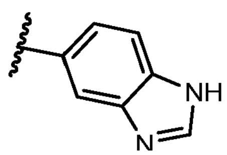 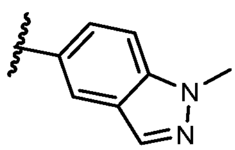

In preferred embodiments Ar is pyrrolopyridine, azaindole, benzotriazole or N-methyl benzotriazole.

In preferred embodiments Ar is:

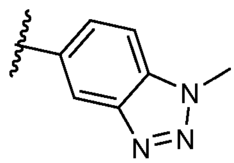

or

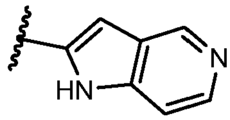

In embodiments the compound of formula (I) is a compound according to formulae (IXa) or (IXb):

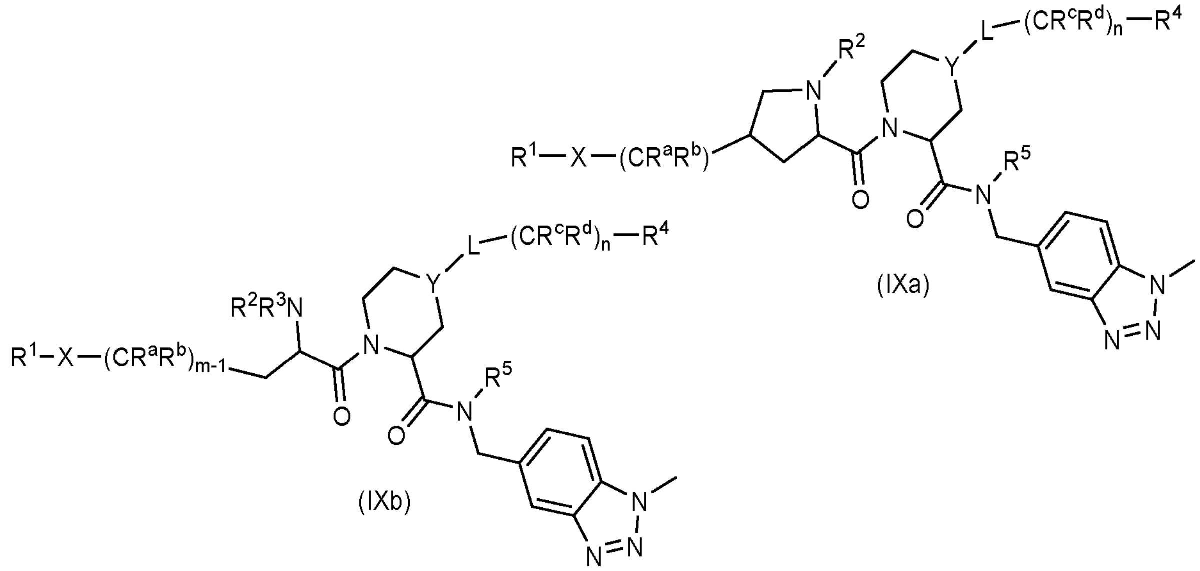

(IXa)

(IXb)

In embodiments of compounds of formulae (IXa) $R^2$ and $R^5$ are each H. In embodiments of compounds of formulae (IXa) $R^a$ and $R^b$ are each H. In embodiments of compounds of formulae (IXa) $R^2$, $R^5$, $R^a$ and $R^b$ are each H.

In embodiments of compounds of formulae (IXb) $R^2$, $R^3$ and $R^5$ are each H. In embodiments of compounds of formulae (IXb) $R^a$ and $R^b$ are each H. In embodiments of compounds of formulae (IXb) $R^2$, $R^3$, $R^5$, $R^a$ and $R^b$ are each H.

$R^6$ may be H, Me or -C(O)Me. $R^7$ may be H. In embodiments $R^6$ is H.

In embodiments L is selected from: bond, -C(O)O-, and -O-. In embodiments L is -C(O)O-.

In embodiments n is 0, 1, 2 or 3. In embodiments n is 0 or 1. In embodiments n is not 0.

$R^c$ and $R^d$ are independently at each occurrence selected from H and methyl. Preferably, $R^c$ and $R^d$ are H.

In embodiments $-L-(CR^cR^d)_n-$ is selected from: a bond, $CH_2$, -NH-, $-NHCH_2-$, $-NH(CH_2)_2-$, $- NH(CH_2)_3-$, -N(Me)-, $-N(C(O)Me)CH_2-$, $-NHC(O)CH_2-$, $-NHC(O)(CH_2)_2-$, or $NHC(O)(CH_2)_3-$.

In embodiments $-L-(CR^cR^d)_n-$ is selected from: a bond, -C(O)O-, $-C(O)OCH_2-$, or -O-.

$R^4$ may be selected from: $C_{1-4}$ alkyl, 6 to 10 membered aryl, 5 to 10 membered heteroaryl, wherein the aryl or heteroaryl group is unsubstituted or substituted with 1, 2 or 3 $R^{12}$.

The 6 to 10 membered aryl of $R^4$ may be selected from phenyl or napthalenyl. The 5 to 10 membered heteroaryl of $R^4$ may be selected from pyridinyl, pyrazinyl, pyrazolyl, imidazolyl, dihydrobenzofuran, benzodioxolanyl or isoindolinyl (optionally pyridinyl, pyrazinyl, pyrazolyl, imidazolyl, dihydrobenzofuran, benzodioxolanyl or isoindolinyl). Any of the 6 to 10 membered aryl or 5 to 10 membered heteroaryl groups may be unsubstituted or substituted with 1, 2, or 3 $R^{12}$.

In embodiments $R^4$ is selected from: $=CH_2$, -CN, halo, $C_{1-4}$ alkyl, $C_{1-4}$ haloalkyl, $-OR^{10}$, $- NR^{10}R^{11}$, phenyl or napthalenyl, pyridinyl, pyrazinyl, pyrazolyl, imidazolyl, dihydrobenzofuran, benzodioxolanyl or isoindolinyl; wherein any group that is cyclic is unsubstituted or substituted with 1, 2, or 3 $R^{12}$.

In embodiments $R^4$ is selected from: phenyl, methoxyphenyl, methylphenyl, chlorphenyl, methyl, pyrimidine.

$R^{12}$ is independently selected from: halo, $C_{1-4}$ alkyl, $C_{1-4}$ haloalkyl, $-OR^{13}$, -CN, $-C(O)R^{10}$, =O, $SO_2R^{10}$, benzyl, phenyl, unsubstituted 5 or 6 membered heteroaryl, or methyl substituted 5 or 6 membered heteroaryl. Optionally, $R^{12}$ is independently selected from: Cl, Br, F, $CF_3$, OMe, OEt, OPh, CN, $SO_2Me$, methyl, pyridinyl, or methylpyrazole.

In embodiments $R^{4a}$ is H, OH or F (preferably H) and $-L-(CR^cR^d)_n-R^4$ is selected from: $-CF_3$, - OH, $-NH_2$, $=CH_2$, -CN, -NHC(O)Me, phenyl, isoindoline, benzyl, $-CH_2$phenyl, $-CH_2$pyridinyl, - $CH_2$pyrazolyl, $-CH_2$dihydrobenzofuran, $-CH_2$imidazolyl, $-CH_2$benzodioxolanyl, -NHpyrazinyl, - $NHCH_2Ph$, - $-NHCH_2CH_2Ph$, and $-NHCH_2CH_2CH_2Ph$;
wherein any of the above cyclic groups is unsubstituted or substituted with 1, 2 or 3 groups selected from: Cl, Br, F, $CF_3$, OMe, OEt, -O-phenyl, -O-benzyl, CN, $SO_2Me$, methyl, pyridinyl, or methylpyrazole.

In embodiments $R^{4a}$ is H, OH or F (preferably H) and $-L-(CR^cR^d)_n-R^4$ is selected from: $-CF_3$, - OH, $-NH_2$, $=CH_2$,

-CN, -NHC(O)Me, phenyl, isoindoline, -NHpyrazinyl, $-NHCH_2Ph$, $-NHCH_2CH_2Ph$, and $-NHCH_2CH_2CH_2Ph$;

wherein any of the above cyclic groups is unsubstituted or substituted with 1, 2 or 3 groups selected from: Cl, Br, F, $CF_3$, OMe, OEt, -O-phenyl, -O-benzyl, CN, $SO_2Me$, methyl, pyridinyl, or methylpyrazole.

In embodiments $R^{4a}$ is H and $-L-(CR^cR^d)_n-R^4$ is selected from: $-C(O)OCH_2$phenyl, - $C(O)OCH_3$,

In embodiments $R^{4a}$ is H and $-L-(CR^cR^d)_n-R^4$ is:

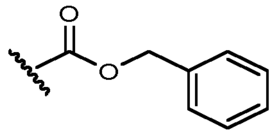

In embodiments the compound of formula (I) is a compound according to formula (Xa) or (Xb):

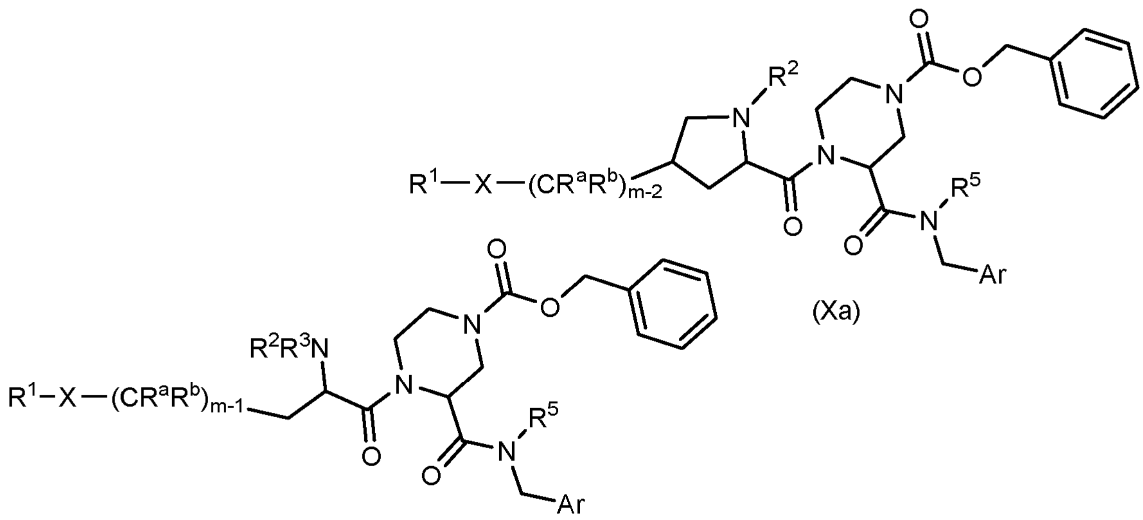

(Xa)

(Xb)

In embodiments the compound of formula (I) is a compound according to formula (XIa) or (XIb):

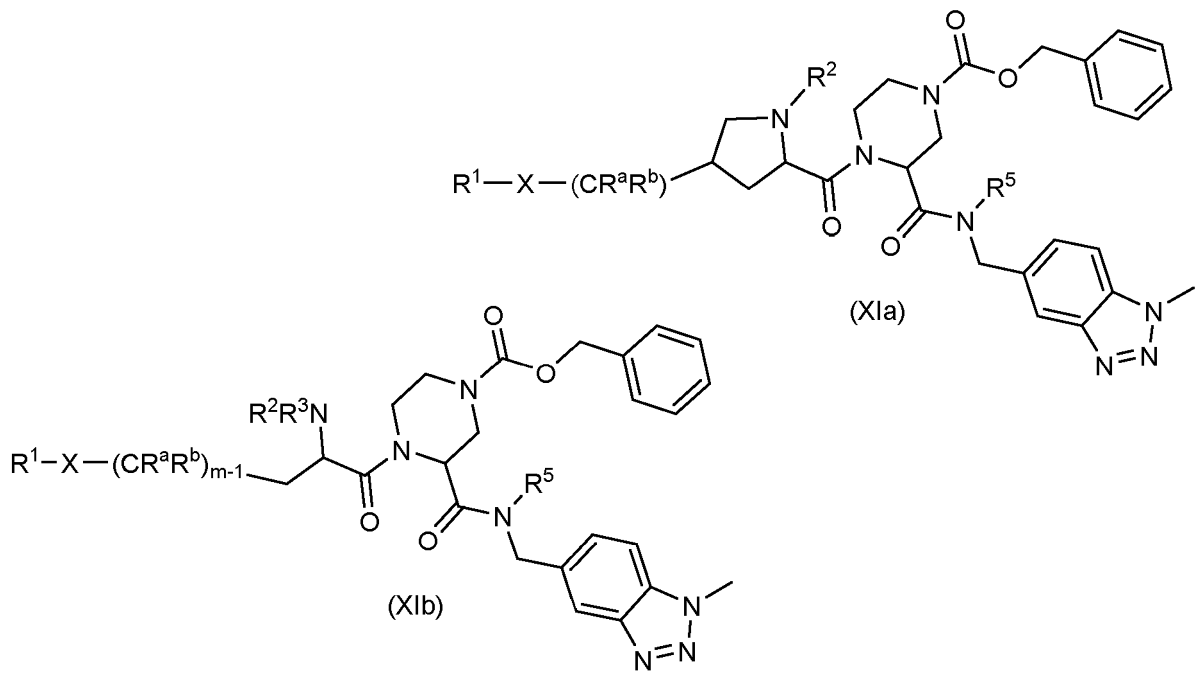

(XIa)

(XIb)

In embodiments $R^1$ is selected from substituted or unsubstituted: phenyl, or 5, 6 membered heteroaryl; wherein when substituted $R^1$ is substituted with 1, 2, or 3 groups selected from: =O, CN, - OH, or -O-$C_{1-6}$ alkyl, halo and $C_{1-6}$ alkyl. Preferably, $R^1$ is unsubstituted.

R' may be selected from: -$NMe_2$, -N(Me)i-Pr, -NH-cyclopropyl, cyclopropyl, phenyl, pyridinyl, pyridinonyl, pyrimidinyl, imidazolyl, pyrazolyl, oxazolyl, pyrollidinyl, fluoropyrollidinyl, azetidinyl, piperidinyl, piperazinyl, azepanyl, indoline, tetrahydronapthalenyl, or

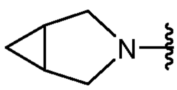 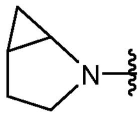 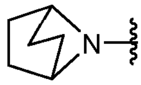 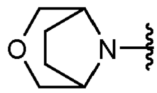 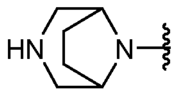

$R^1$ may be substituted with a group selected from: F, CN, =O, -OH, -$OCF_3$, -OMe, Me, i-Pr, or -$CF_3$.

Preferably $R^1$ may be selected from: phenyl, pyridinyl, or pyrollidinyl, wherein $R^1$ is unsubstituted or substituted with a group selected from: F, CN, -OH, -$OCF_3$, -OMe, Me, i-Pr, or -$CF_3$.

Preferably $R^1$ may be phenyl or pyrrolidinyl.

In embodiments the compounds of formula (I) are selected from:

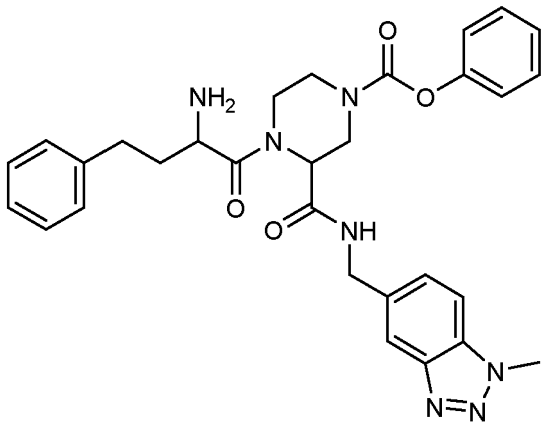

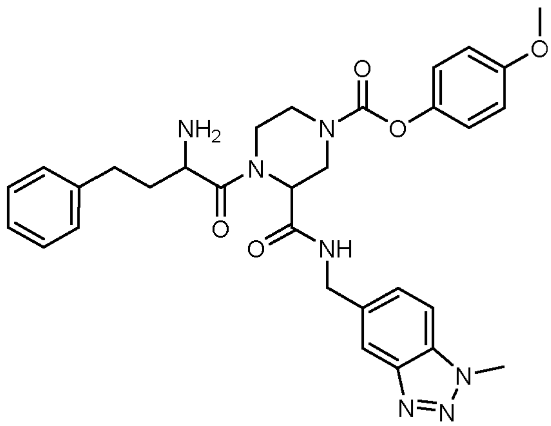

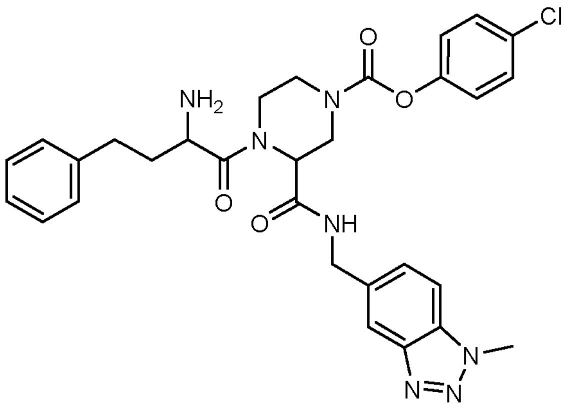

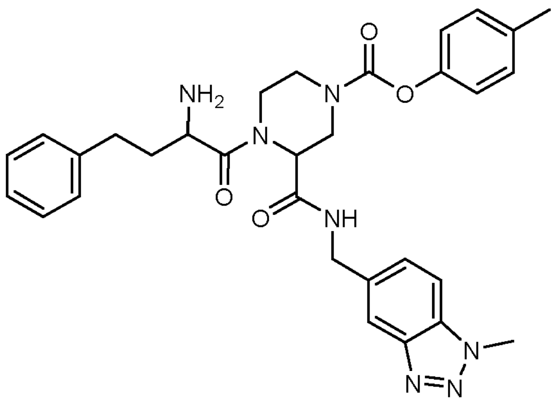

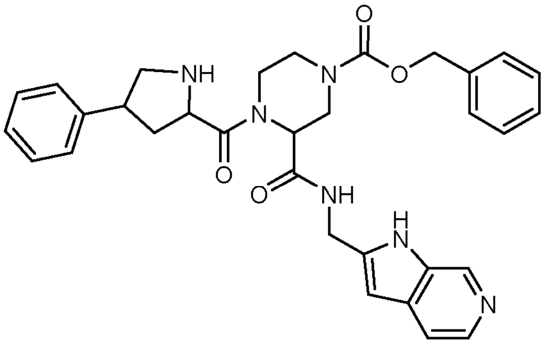

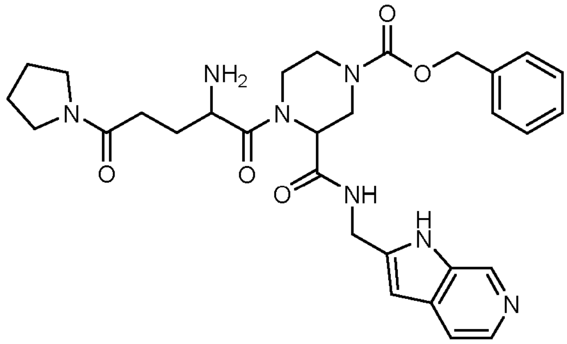

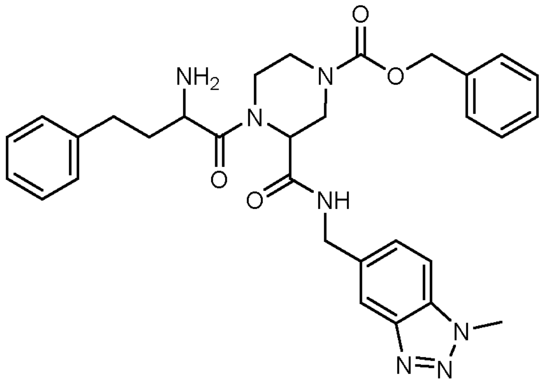

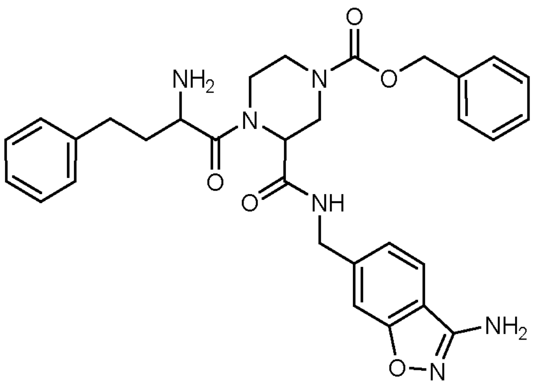

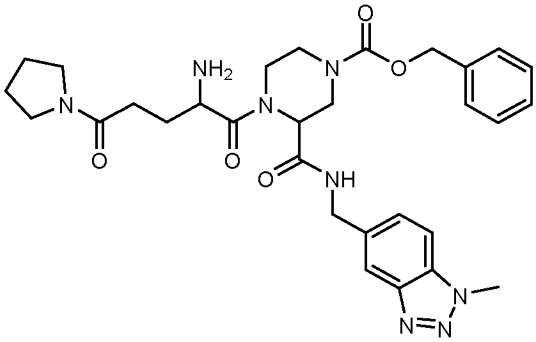

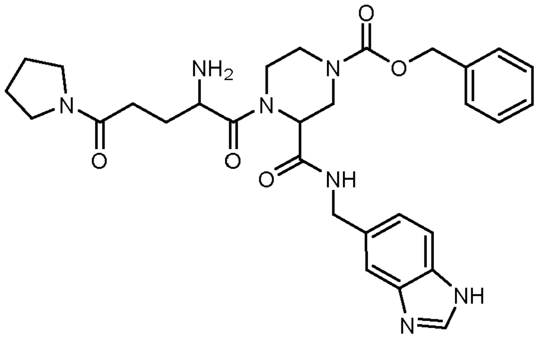

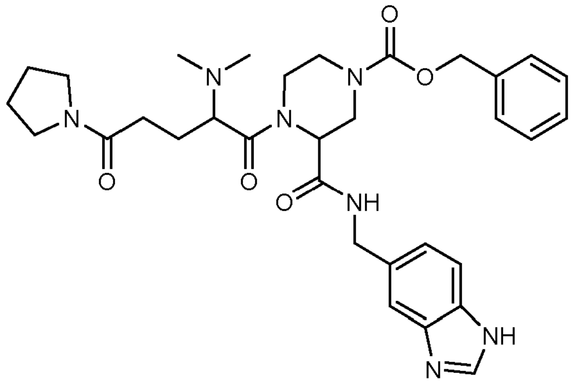

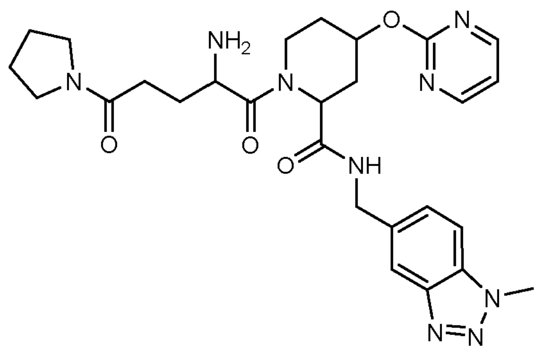

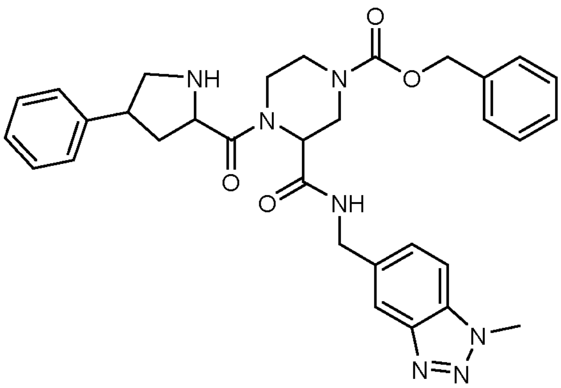

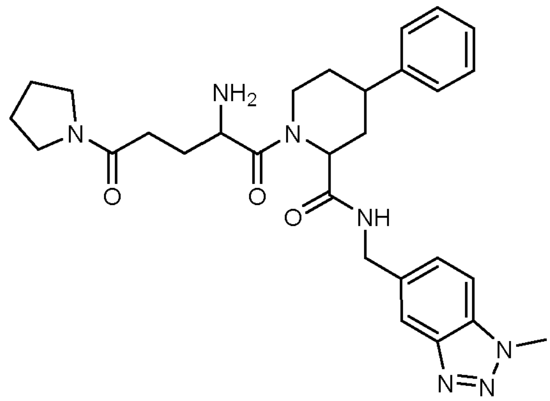

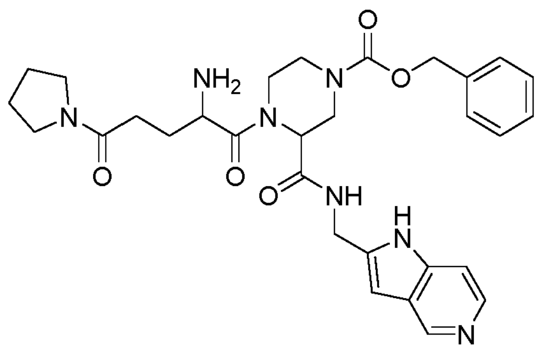

In embodiments the compounds of formula (I) may be selected from:

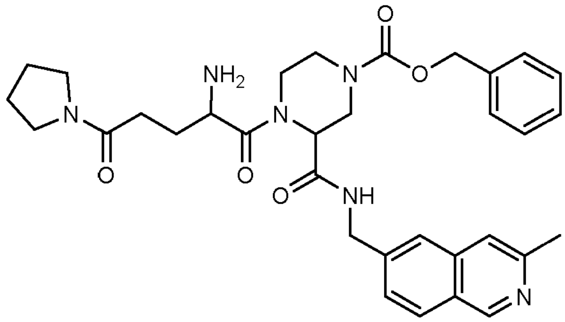

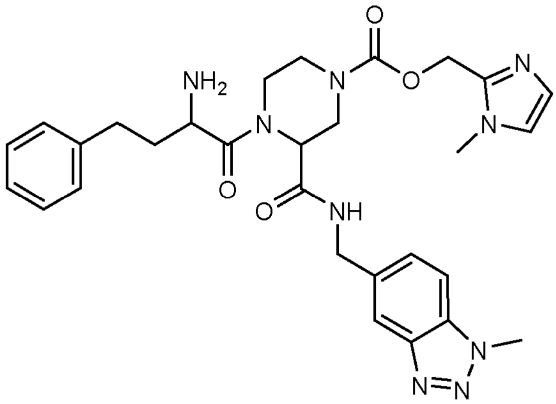

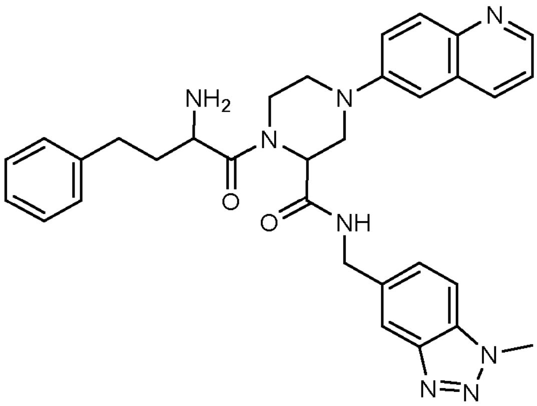

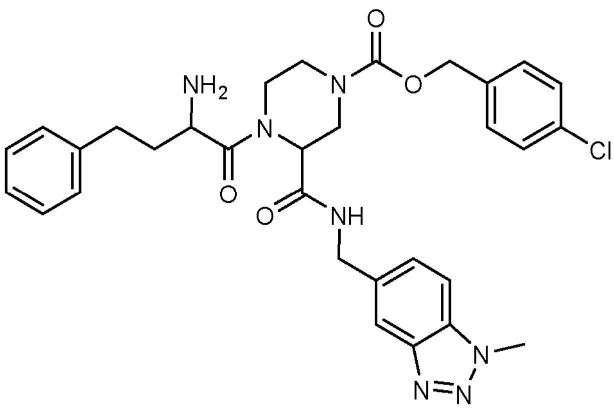

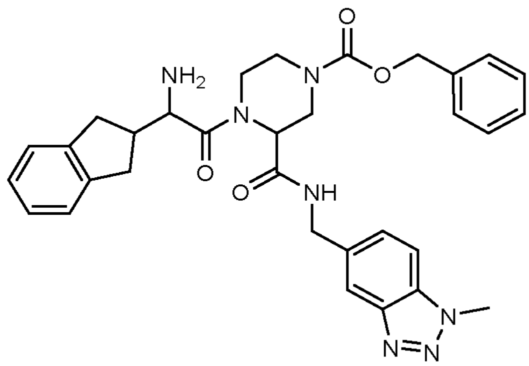

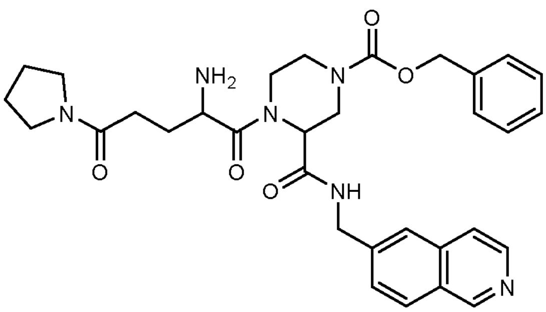

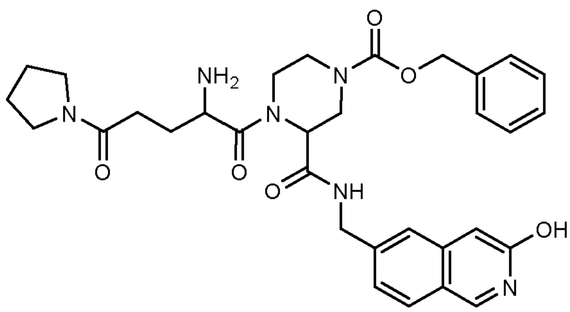

More preferred compounds of the invention have a Ki of less than 0.2 μM (for a disclosure of the test methodology for determining Ki see Determination of Factor XIIa Inhibition in the Examples and Synthesis section). Less preferred compounds have a Ki value of greater than 100 μM measured at concentrations utilised in the test method. In embodiments, compounds with a Ki value of greater than 100 μM do not form part of the invention. Accordingly, in certain embodiments the compound of the invention is not a compound with a Ki value of greater than 100 μM. Equally, in embodiments, the compound of the invention is a compound with a Ki of less than 100 μM. Certain less preferred compounds having a Ki value of greater than 100 μM are shown immediately below. In certain embodiments the

compound of the invention is not a compound selected from:

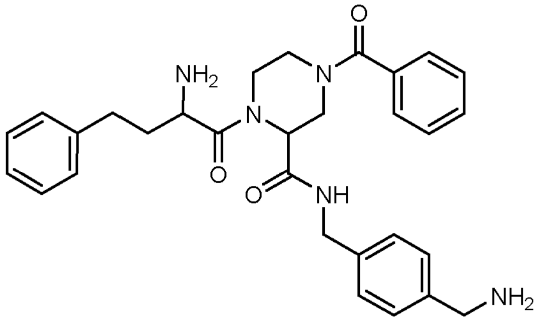

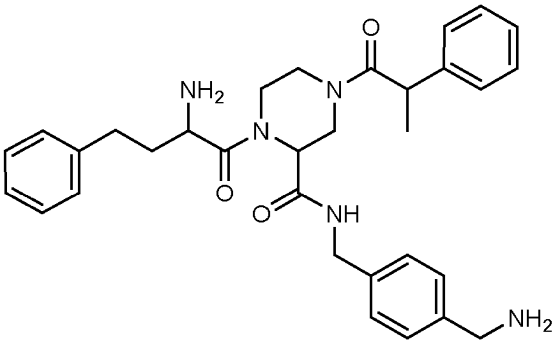

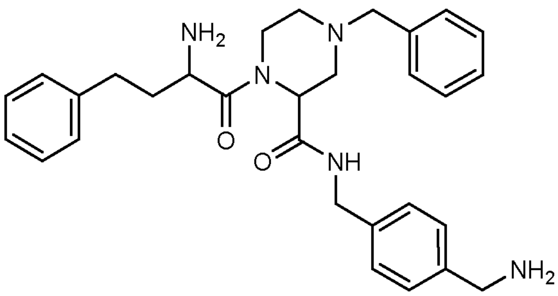

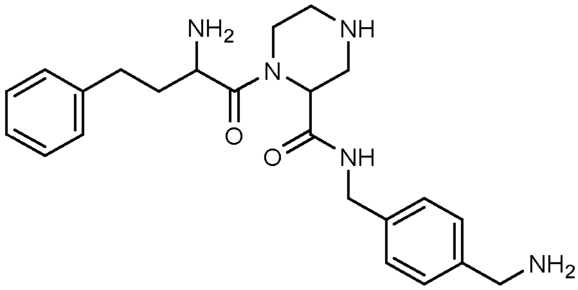

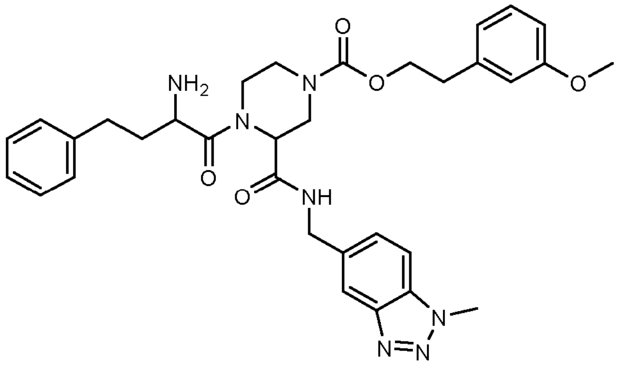

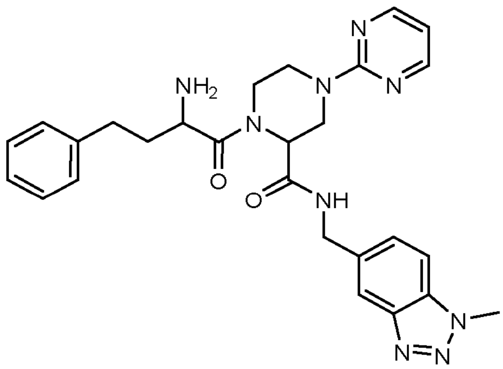

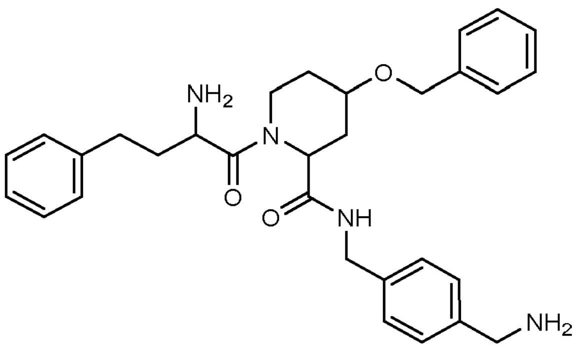

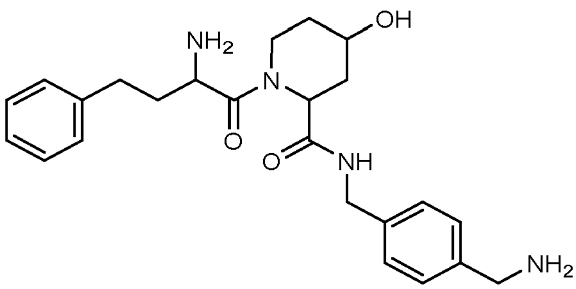

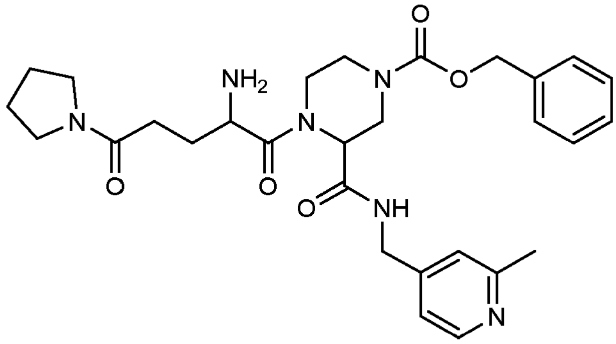

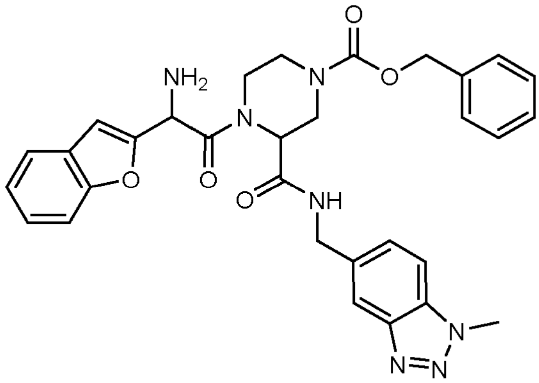

As will be evident to the skilled person the compounds of the present invention contain a number of stereocentres. The present invention encompasses all possible stereoisomers of the present invention whether in a single stereoisomeric form or a mixture thereof. A preferred stereoisomer is the S enantiomer at the 2 position of the piperidine (Y is CH)/piperazine (Y is N) ring. For example:

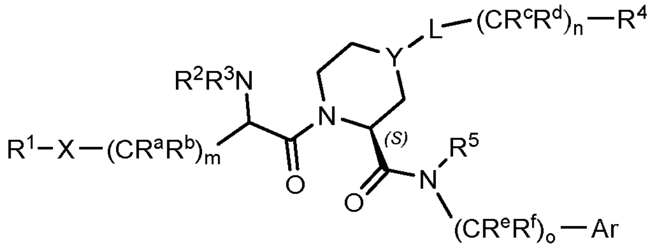

(I)

The preferred stereochemistry of the $-NR^2R^3$ group is R. For example:

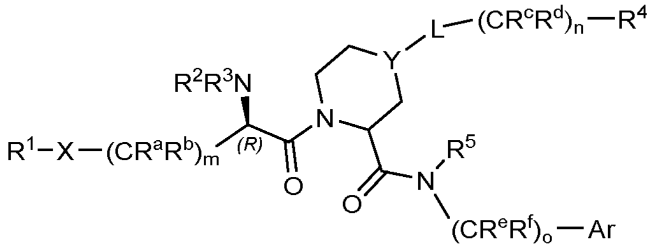

(I)

Accordingly, in an embodiment the compound of the invention is a diastereomer with S configuration at the 2 position of the piperidine/piperazine and a R configuration at the $-NR^2R^3$ group. As such, the compound of formula (I) may be:

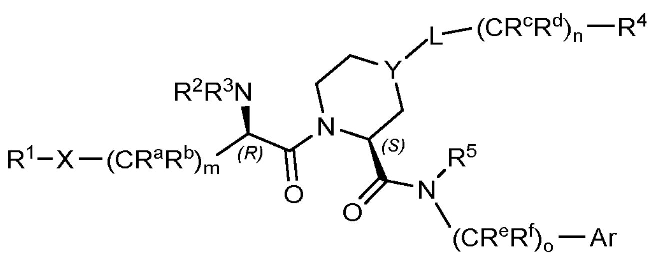

(I)

In an embodiment, the $-(CR^aR^b)-X-R^1$ group substituted onto a pyrrolidine of compounds such as formula (IIa) at the 4 position has a cis relationship with the -C(=O)- group at the 2 position. For example:

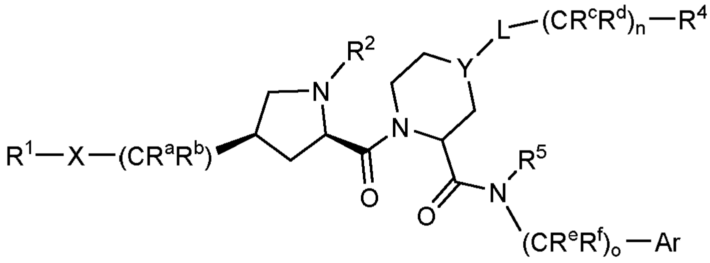

(IIa)

In an embodiment $R^1$ is not indoline. In embodiments $R^1$ is not a 9 membered bicyclic heteroaromatic group.

In an aspect of the invention there is provided the compounds of the present invention for use as a medicament.

In accordance with another aspect, the present invention provides a pharmaceutical formulation comprising a compound of the present invention and a pharmaceutically acceptable excipient.

In an embodiment the pharmaceutical composition may be a combination product comprising an additional pharmaceutically active agent.

In a preferred aspect of the invention, the compounds are selective FXIIa inhibitors. By the term "selective FXIIa inhibitors" is meant compounds that selectively inhibit FXIIa over thrombin and FXa. Generally, a compound of the present invention may have a selectivity for FXIIa over thrombin of at least >10 fold, preferably at least >100 fold.

In accordance with another aspect of the invention, there is provided a compound of the present invention for use in the prevention and/or treatment of a condition which is modulated by Factor XIIa. Conditions preventable and/or treatable by modulation of Factor XIIa would ordinarily be conditions that are preventable and/or treatable by the inhibition of Factor XIIa. Accordingly, the compounds of the present invention may be for use in the prevention and/or treatment of a condition treatable by the inhibition of Factor XIIa.

The compound of the present invention may be for use in the treatment or prevention of a condition selected from the following or as a co-therapy in a treatment or prevention of a condition selected from: thrombosis, deep venous thrombosis, reperfusion injury also know as ischaemia-reperfusion injury, transcatheter aortic valve replacement (TAVR) also known as transcatheter aortic valve implantation (TAVI), complex left-sided ablation (pulmonary vein isolation; VT ablation), spinal or epidural anaesthesia, lumbar diagnostic puncture, thoracic surgery, abdominal surgery, major orthopaedic surgery, liver biopsy, transurethral prostate resection, kidney biopsy, renal insufficiency, liver diseases, patients with atrial fibriliation and chronic kidney disease endoscopy with biopsy, prostate or bladder biopsy, electrophysiological study or radiofrequency catheter ablation for supraventricular tachycardia (including left-sided ablation via single trans-septal puncture), angiography, pacemaker or implantable cardioverter defibrillator (ICD) implantation (unless complex anatomical setting, e.g. congenital heart disease), mechanical valve implantation, prosthetic valve implantation, myocardial infarction, angina pectoris (including unstable angina), reocclusions and restenoses after angioplasty or aortocoronary bypass, stroke, patients with atrial fibrillation to reduce their risk of stroke, transitory ischaemic attacks, peripheral arterial occlusion disorders, deep venous thrombosis, pulmonary embolisms, deep venousmicrovascular disease, patients requiring extra corporeal membrane oxygenation (ECMO), patients requiring extra corporeal circulation such as coronary artery bypass grafting (CABG), disseminated intravascular coagulation (DIC), atherosclerosis, arthritis, thrombosis in patients with cancer, silent brain ischaemia, stroke, neurotraumatic disorder, neurological inflammatory disorders, medical procedures comprising contact with artificial surfaces including renal dialysis, other conditions where inhibition of FXIIa could be beneficial such as Alzheimer's disease, vascular dementia, macular degeneration, diabetic retinopathy, diabetic macular oedema, cerebral oedema in stroke, other causes of oedema, hereditary angioedema or acquired angioedema.

The condition preventable and/or treatable by the inhibition of Factor XIIa may be a condition associated with blood thickening, blood coagulation, or blood clot formulation for example the condition may be thrombosis.

In embodiments of the invention, compounds are provided for use in the prevention and/or treatment of or as a co-therapy for conditions associated with a high risk of bleeding, a low risk of bleeding, or thromboembolic disorders.

In embodiments of the invention, compounds are provided for use in the prevention and/ or treatment of or as a co-therapy for conditions associated with a high risk of bleeding.

In embodiments of the invention, compounds are provided for use in the prevention and/or treatment of or as a co-therapy for conditions associated with a low risk of bleeding.

In embodiments of the invention, compounds are provided for use in the prevention and/or treatment of or as a co-therapy for conditions associated with thromboembolic disorders.

In embodiments of the invention, the compound of the invention is for use as part of a prevention and/or treatment for a condition associated with a high risk of bleeding, wherein the treatment is selected from complex left-sided ablation (pulmonary vein isolation; VT ablation), spinal or epidural anaesthesia, lumbar diagnostic puncture, thoracic surgery,

abdominal surgery, major orthopaedic surgery, liver biopsy, liver diseases, transurethral prostate resection, kidney biopsy, or renal insufficiency.

In embodiments of the invention, the compound of the invention is for use as part of a prevention and/or treatment for a condition associated with a low risk of bleeding, wherein the treatment is selected from endoscopy with biopsy, prostate or bladder biopsy, electrophysiological study or radiofrequency catheter ablation for supraventricular tachycardia (including left-sided ablation via single trans-septal puncture), angiography, pacemaker or implantable cardioverter defibrillator (ICD) implantation (unless complex anatomical setting, e.g. congenital heart disease), mechanical valve implantation, or prosthetic valve implantation.

In an embodiment, compounds of the present invention are for use to avoid or mitigate the contraindications of existing anticoagulant therapies, such as Dabigatran, Rivaroxaban, Apixaban, warfarin, Edoxaban and Betrixaban.

In an aspect of the invention there is provided a use of a compound the invention to avoid or mitigate the contraindications of existing anticoagulant therapies, such as Dabigatran, Rivaroxaban, Apixaban, warfarin, Edoxaban and Betrixaban.

In an embodiment, the compounds of the present invention are for use in mitigating the contraindications of therapies using Rivaroxaban; wherein the contraindication may include: an estimated Glomerular Filtration Rate (eGFR) of less than 15 mL/minute/1.73 $m^2$, active bleeding, a significant risk of major bleeding from: current or recent gastro-intestinal ulcer, oesophageal varices, recent brain or spinal injury, recent brain, spine, or ophthalmic surgery, recent intracranial haemorrhage, malignant neoplasm, vascular aneurysm, prosthetic heart valve, liver disease associated with coagulopathy and clinically relevant bleeding risk, as well as people who have cirrhosis with Child Pugh B and C or people who are taking any other anticoagulants, except when switching to or from warfarin treatment; and people who are taking strong inhibitors of cytochrome P 3A4 enzyme and P-glycoprotein, such as ketoconazole, or HIV protease inhibitors such as ritonavir.

In an embodiment, the compounds of the present invention are for use in mitigating the contraindications of therapies using Apixaban; wherein the contraindication may include: creatinine clearance (CrCl) of less than 15 mL/min, or eGFR <15 mL/minute/1.73 $m^2$, active bleeding, a significant risk of major bleeding such as: current or recent gastro-intestinal ulcer, oesophageal varices, recent brain or spinal injury, recent brain, spine, or ophthalmic surgery, recent intracranial haemorrhage, malignant neoplasm, vascular aneurysm, liver disease associated with coagulopathy and clinically relevant bleeding risk, a prosthetic heart valve, people who are taking any other anticoagulants, except when switching to or from warfarin treatment, or people who are taking strong inhibitors of cytochrome P3A4 enzyme and P-glycoprotein, such as ketoconazole, or HIV protease inhibitors such as ritonavir.

In an embodiment, the compounds of the present invention are for use in mitigating the contraindications of therapies using Edoxaban; wherein the contraindication includes Edoxaban not being used in NVAF patients with CrCl >95 mL/minute because of an increased risk of ischemic stroke compared to warfarin.

In an embodiment, the compounds of the present invention are for use in mitigating the contraindications of therapies using Dabigatran; wherein the contraindication includes stroke prophylaxis with atrial fibrillation (Prevention of stroke and systemic embolism associated with nonvalvular atrial fibrillation), renal impairment CrCl <15 mL/min or dialysis, DVT or PE treatment (Indicated for treatment of deep vein thrombosis (DVT) and pulmonary embolus (PE) in patients who have been treated with a parenteral anticoagulant for 5-10 days) CrCl ≤30 mL/min or on dialysis, DVT or PE prophylaxis (Indicated for the prophylaxis of deep vein thrombosis (DVT) and pulmonary embolism (PE) following hip replacement surgery), Dabigatran is contraindicated with defibrotide, mifepristone and human prothrombin complex concentrate, dabigatran should not be used with the following: antithrombin alfa, antithrombin iii, apixaban, carbamazepine, dalteparin, dexamethasone, doxorubicin, doxorubicin liposomal, dronedarone, edoxaban, enoxaparin, fondaparinux, fosphenytoin, heparin, ketoconazole, lepirudin, nefazodone, phenobarbital, phenytoin, primidone, rifampin, st john's wort, tenofovir df, tipranavir, vinblastine and warfarin.

In an embodiment, the compounds of the present invention are for use in mitigating the contraindications of therapies using Dabigatran; wherein the contraindication includes: renal impairment (CrCl <15 mL/min), hemodialysis, hypersensitivity, active pathologic bleeding, impairment of hemostasis, mechanical or prosthetic heart valves, thromboembolic events (eg, valve thrombosis, stroke, TIAs, MI), excessive major bleeding (predominantly postoperative pericardial effusions requiring intervention for hemodynamic compromise), increased bleeding risk during labour and delivery, anticoagulants for active bleeding, elective surgery, or invasive procedures, patients at an increased risk of stroke, additive risk of bleeding when co-administered with antiplatelet agents, warfarin, heparin, fibrinolytic therapy, and long-term NSAIDs or aspirin, congenital or acquired coagulation disorders, ulcerative GI diseases and other gastritis like symptoms, recent haemorrhage, recent brain, spinal, or ophthalmic surgery, patients undergoing neuraxial anesthesia (spinal/epidural anesthesia), patients undergoing spinal puncture at risk of developing an epidural or spinal hematoma which can result in long-term or permanent paralysis, coadministration with P-gp inducers and inhibitors, P-gp inducers (eg, rifampin) or any combination thereof.

In an embodiment, the compounds of the present invention are for use in mitigating the contraindications of therapies using Betrixaban; wherein the contraindication includes: patients taking P-gp inhibitor, pateints who have severe

renal impairment, patients with hepatic impairment, patients with intrinsic coagulation abnormalities, patients with prosthetic heart valves, coadministration with drugs affecting hemostasis (thereby increasing bleeding risk), coadministration with aspirin, coadministration with other antiplatelet agents, coadministration with other anticoagulants, coadministration with heparin, coadministration with thrombolytic agents, coadministration with selective serotonin reuptake inhibitors (SSRIs), coadministration with serotonin-norepinephrine reuptake inhibitors (SNRIs), and coadministration with non-steroidal anti-inflammatory drugs (NSAIDs).

In an embodiment, compounds of the invention may be used as anticoagulants for the prophylaxis and/or therapy of thromboembolic disorders; wherein the disorder is one of: myocardial infarction, angina pectoris (including unstable angina), reocclusions and restenoses after angioplasty or aortocoronary bypass, stroke, patients with atrial fibrillation to reduce their risk of stroke, patients with atrial fibriliation and chronic kidney disease, transitory ischaemic attacks, peripheral arterial occlusion disorders, reperfusion injury also known as ischaemia-reperfusion injury, transcatheter aortic valve replacement (TAVR) also known as transcatheter aortic valve implantation (TAVI), pulmonary embolisms, deep venousmicrovascular disease or patients requiring extra corporeal membrane oxygenation (ECMO).

In an embodiment, compounds according to the invention may be suitable for preventing and/or treating disseminated intravascular coagulation (DIC).

In an embodiment, the compounds of the invention are also suitable for the prophylaxis and/or treatment of atherosclerosis and arthritis, and additionally also for the prophylaxis and/or treatment of thrombosis in patients with cancer.

In an embodiment the compounds of the present invention is for use in a method of preventing and/or treating thrombosis.

In an aspect of the invention the compound disclosed herein may be for use as an anticoagulant.

In an aspect of the invention there is provided a method for prevention of thrombosis or deep venous thrombosis, prevention and/or treatment of a condition selected from: thrombosis, complex left-sided ablation (pulmonary vein isolation; VT ablation), spinal or epidural anaesthesia, lumbar diagnostic puncture, thoracic surgery, abdominal surgery, major orthopaedic surgery, liver biopsy, transurethral prostate resection, kidney biopsy, renal insufficiency, liver diseases, endoscopy with biopsy, prostate or bladder biopsy, electrophysiological study or radiofrequency catheter ablation for supraventricular tachycardia (including left-sided ablation via single trans-septal puncture), angiography, pacemaker or implantable cardioverter defibrillator (ICD) implantation (unless complex anatomical setting, e.g. congenital heart disease), mechanical valve implantation, prosthetic valve implantation, reperfusion injury also known as ischaemia-reperfusion injury, transcatheter aortic valve replacement (TAVR) also known as transcatheter aortic valve implantation (TAVI), myocardial infarction, angina pectoris (including unstable angina), reocclusions and restenoses after angioplasty or aortocoronary bypass, stroke, patients with atrial fibrillation to reduce their risk of stroke, patients with atrial fibriliation and chronic kidney disease, transitory ischaemic attacks, peripheral arterial occlusion disorders, deep venous thrombosis, pulmonary embolisms, deep venousmicrovascular disease, patients requiring extra corporeal membrane oxygenation (ECMO), patients requiring extra corporeal circulation such as coronary artery bypass grafting (CABG), disseminated intravascular coagulation (DIC), atherosclerosis, arthritis, thrombosis in patients with cancer, silent brain iscahmia, stroke, neurotraumatic disorder, neurological inflammatory disorders, medical procedures comprising contact with artificial surfaces including renal dialysis, other conditions where inhibition of FXIIa could be beneficial such as Alzheimer's disease, vascular dementia, macular degeneration, diabetic retinopathy, diabetic macular oedema, cerebral oedema in stroke, other causes of oedema, hereditary angioedema or acquired angioedema, wherein the method comprises administering a therapeutically effective amount of a compounds of the invention or administering a therapeutically effective amount of a compound of the present invention as a co-therapy.

In an aspect of the invention there is provided a method of preventing coagulation, wherein the method comprises the administration of a therapeutically effective amount of a compound of the invention.

In an aspect of the invention there is provided a method of preventing and/or treating thrombosis, wherein the method comprises the administration of a therapeutically effective amount of a compound of the invention.

In an aspect of the invention there is provided a use of a compound of the invention in the manufacture of a medicament for use in the prevention and/or treatment of conditions treatable by the inhibition of Factor XII (optionally Factor XIIa), for example the condition may be thrombosis.

In another aspect of the invention there is provided a pharmaceutical composition, wherein the composition comprises a compound of the invention and pharmaceutically acceptable excipients.

In an embodiment the pharmaceutical composition may be a combination product comprising an additional pharmaceutically active agent. The additional pharmaceutically active agent may be one disclosed elsewhere herein.

The compounds of the present invention may be used for the prevention and/or treatment of any of the conditions disclosed above. Alternatively, the compounds of the present invention may be used as a co-therapy in the prevention and/or treatment of a condition disclosed above. Alternatively, the compounds of the present invention may be used as a co-therapy in a prevention and/or treatment of a condition disclosed above. Where the compound of the present invention could be used in combination with another art known therapy for the condition. For example a FXII(a) inhibitor may be used

in combination with anti-platelet therapy with the aim of providing enhanced anti-thrombotic efficacy without incurring an increased risk of bleeding compared with the anti-platelet therapy alone. Furthermore, a FXII(a) inhibitor is likely to be used in combination with other treatments.

## BRIEF DESCRIPTION OF THE DRAWINGS

Embodiments of the invention are further described hereinafter with reference to the accompanying drawings, in which:
Figure 1 shows that anticoagulant efficacy of compound M00834 using a standard dose of intravenous administration in a femoral vein ferric chloride induced thrombosis model. The percentage inhibition of clot formation is calculated relative to mice administered vehicle only for the 60 minute time point. A minimum of 4 mice were employed in each group.

## DETAILED DESCRIPTION

Given below are definitions of terms used in this application. Any term not defined herein takes the normal meaning as the skilled person would understand the term.

The term "halo" refers to one of the halogens, group 17 of the periodic table. In particular, the term refers to fluorine, chlorine, bromine and iodine. Preferably, the term refers to bromine or iodine.

The term "alkyl" refers to a linear or branched hydrocarbon chain. For example, the term "$C_{1-6}$ alkyl" refers to a linear or branched hydrocarbon chain containing 1, 2, 3, 4, 5 or 6 carbon atoms, for example methyl, ethyl, *n*-propyl, *iso*-propyl, *n*-butyl, *sec*-butyl, *tert*-butyl, *n*-pentyl and *n*-hexyl. Alkylene groups may likewise be linear or branched and may have two places of attachment to the remainder of the molecule. Furthermore, an alkylene group may, for example, correspond to one of those alkyl groups listed in this paragraph. The alkyl and alkylene groups may be unsubstituted or substituted by one or more substituents. Possible substituents are described below. Substituents for the alkyl group may be halogen, e.g. fluorine, chlorine, bromine and iodine, OH, $C_{1-6}$ alkoxy.

The term "alkoxy" refers to an alkyl group which is attached to a molecule *via* oxygen. For example, the term "$C_{1-6}$ alkoxy" refers to a group where the alkyl part may be linear or branched and may contain 1, 2, 3, 4, 5 or 6 carbon atoms, for example methyl, ethyl, *n*-propyl, *iso*-propyl, *n*-butyl, *sec*-butyl, *tert*-butyl, *n*-pentyl and *n*-hexyl. Therefore, the alkoxy group may be methoxy, ethoxy, *n*-propoxy, *iso*-propoxy, *n*-butoxy, *sec*-butoxy, *tert*-butoxy, *n*-pentoxy and *n*-hexoxy. The alkyl part of the alkoxy group may be unsubstituted or substituted by one or more substituents. Possible substituents are described below. Substituents for the alkyl group may be halogen, e.g. fluorine, chlorine, bromine and iodine, OH, $C_{1-6}$ alkoxy.

The term "haloalkyl" refers to a hydrocarbon chain substituted with at least one halogen atom independently chosen at each occurrence, for example fluorine, chlorine, bromine and iodine. For example, the term "$C_{1-6}$ haloalkyl" refers to a linear or branched hydrocarbon chain containing 1, 2, 3, 4, 5 or 6 carbon atoms substituted with at least one halogen. The halogen atom may be present at any position on the hydrocarbon chain. For example, $C_{1-6}$ haloalkyl may refer to chloromethyl, fluoromethyl, trifluoromethyl, chloroethyl e.g. 1-chloromethyl and 2-chloroethyl, trichloroethyl e.g. 1,2,2-trichloroethyl, 2,2,2-trichloroethyl, fluoroethyl e.g. 1-fluoromethyl and 2-fluoroethyl, trifluoroethyl e.g. 1,2,2-trifluoroethyl and 2,2,2-trifluoroethyl, chloropropyl, trichloropropyl, fluoropropyl, trifluoropropyl.

The term "alkenyl" refers to a branched or linear hydrocarbon chain containing at least one double bond. For example, the term "$C_{2-6}$ alkenyl" refers to a branched or linear hydrocarbon chain containing at least one double bond and having 2, 3, 4, 5 or 6 carbon atoms. The double bond(s) may be present as the *E* or *Z* isomer. The double bond may be at any possible position of the hydrocarbon chain. For example, the "$C_{2-6}$ alkenyl" may be ethenyl, propenyl, butenyl, butadienyl, pentenyl, pentadienyl, hexenyl and hexadienyl.

The term "alkynyl" refers to a branched or linear hydrocarbon chain containing at least one triple bond. For example, the term "$C_{2-6}$ alkynyl" refers to a branched or linear hydrocarbon chain containing at least one triple bond and having 2, 3, 4, 5 or 6 carbon atoms. The triple bond may be at any possible position of the hydrocarbon chain. For example, the "$C_{2-6}$ alkynyl" may be ethynyl, propynyl, butynyl, pentynyl and hexynyl.

The term "heteroalkyl" refers to a branched or linear hydrocarbon chain containing at least one heteroatom selected from N, O and S positioned between any carbon in the chain or at an end of the chain. For example, the term "$C_{1-6}$ heteroalkyl" refers to a branched or linear hydrocarbon chain containing 1, 2, 3, 4, 5, or 6 carbon atoms and at least one heteroatom selected from N, O and S positioned between any carbon in the chain or at an end of the chain. For example, the hydrocarbon chain may contain one or two heteroatoms. The $C_{1-6}$ heteroalkyl may be bonded to the rest of the molecule through a carbon or a heteroatom. For example, the "$C_{1-6}$ heteroalkyl" may be $C_{1-6}$ N-alkyl, $C_{1-6}$ *N,N*-alkyl, or $C_{1-6}$ O-alkyl.

The term "carbocyclic" refers to a saturated or unsaturated carbon containing ring system. A "carbocyclic" system may be monocyclic or a fused polycyclic ring system, for example, bicyclic or tricyclic. A "carbocyclic" moiety may contain from 3 to 14 carbon atoms, for example, 3 to 8 carbon atoms in a monocyclic system and 7 to 14 carbon atoms in a polycyclic system. "Carbocyclic" encompasses cycloalkyl moieties, cycloalkenyl moieties, aryl ring systems and fused

ring systems including an aromatic portion.

The term "heterocyclic" refers to a saturated or unsaturated ring system containing at least one heteroatom selected from N, O or S. A "heterocyclic" system may contain 1, 2, 3 or 4 heteroatoms, for example 1 or 2. A "heterocyclic" system may be monocyclic or a fused polycyclic ring system, for example, bicyclic or tricyclic. A "heterocyclic" moiety may contain from 3 to 14 carbon atoms, for example, 3 to 8 carbon atoms in a monocyclic system and 7 to 14 carbon atoms in a polycyclic system. "Heterocyclic" encompasses heterocycloalkyl moieties, heterocycloalkenyl moieties and heteroaromatic moieties. For example, the heterocyclic group may be: oxirane, aziridine, azetidine, oxetane, tetrahydrofuran, pyrrolidine, imidazolidine, succinimide, pyrazolidine, oxazolidine, isoxazolidine, thiazolidine, isothiazolidine, piperidine, morpholine, thiomorpholine, piperazine, and tetrahydropyran.

The term cycloalkyl refers to a saturated hydrocarbon ring system. For example "$C_{3-8}$ cycloalkyl" refers to a ring system containing 3, 4, 5, 6, 7 or 8 carbon atoms. For example, the "$C_{3-8}$ cycloalkyl" may be cyclopropyl, cyclobutyl, cyclopentyl, cyclohexyl, cycloheptyl and cyclooctyl.

The term "$C_{3-8}$ cycloalkenyl" refers to an unsaturated hydrocarbon ring system containing 3, 4, 5, 6, 7 or 8 carbon atoms that is not aromatic. The ring may contain more than one double bond provided that the ring system is not aromatic. For example, the "$C_{3-8}$ cycloalkyl" may be cyclopropenyl, cyclobutenyl, cyclopentenyl, cyclopentadienyl, cyclohexenyl, cyclohexadienly, cycloheptenyl, cycloheptadiene, cyclooctenyl and cycloatadienyl.

The term "heterocycloalkyl" refers to a saturated hydrocarbon ring system containing carbon atoms and at least one heteroatom within the ring selected from N, O and S. For example there may be 1, 2 or 3 heteroatoms, optionally 1 or 2. The "heterocycloalkyl" may be bonded to the rest of the molecule through any carbon atom or heteroatom. The "heterocycloalkyl" may have one or more, e.g. one or two, bonds to the rest of the molecule: these bonds may be through any of the atoms in the ring. For example, the "heterocycloalkyl" may be a "$C_{3-8}$ heterocycloalkyl". The term "$C_{3-8}$ heterocycloalkyl" refers to a saturated hydrocarbon ring system containing 3, 4, 5, 6, 7 or 8 atoms at least one of the atoms being a heteroatom within the ring selected from N, O and S. The "heterocycloalkyl" may be oxirane, aziridine, azetidine, oxetane, tetrahydrofuran, pyrrolidine, imidazolidine, succinimide, pyrazolidine, oxazolidine, isoxazolidine, thiazolidine, isothiazolidine, piperidine, morpholine, thiomorpholine, piperazine, and tetrahydropyran.

The term "heterocycloalkenyl" refers to an unsaturated hydrocarbon ring system that is not aromatic, containing carbon atoms and at least one heteroatom within the ring selected from N, O and S. For example there may be 1, 2 or 3 heteroatoms, optionally 1 or 2. The "heterocycloalkenyl" may be bonded to the rest of the molecule through any carbon atom or heteroatom. The "heterocycloalkenyl" may have one or more, e.g. one or two, bonds to the rest of the molecule: these bonds may be through any of the atoms in the ring. For example, the "heterocycloalkenyl" may be a "$C_{3-8}$ heterocycloalkenyl". The term "$C_{3-8}$ heterocycloalkenyl" refers to a saturated hydrocarbon ring system containing 3, 4, 5, 6, 7 or 8 atoms at least one of the atoms being a heteroatom within the ring selected from N, O and S. The "heterocycloalkenyl" may be tetrahydropyridine, dihydropyran, dihydrofuran, pyrroline.

The term "aromatic" when applied to a substituent as a whole means a single ring or polycyclic ring system with 4n + 2 electrons in a conjugated $\pi$ system within the ring or ring system where all atoms contributing to the conjugated $\pi$ system are in the same plane.

The term "aryl" refers to an aromatic hydrocarbon ring system. The ring system has 4n +2 electrons in a conjugated $\pi$ system within a ring where all atoms contributing to the conjugated $\pi$ system are in the same plane. For example, the "aryl" may be phenyl and naphthyl. The aryl system itself may be substituted with other groups. The term "aryl" also includes bicyclic or tricyclic ring systems that are not completely aromatic but contain an aromatic ring within the ring system, for example, indane or tetralin.

The term "heteroaryl" refers to an aromatic hydrocarbon ring system with at least one heteroatom within a single ring or within a fused ring system, selected from O, N and S. The ring or ring system has 4n +2 electrons in a conjugated $\pi$ system where all atoms contributing to the conjugated $\pi$ system are in the same plane. For example, the "heteroaryl" may be imidazole, thiene, furane, thianthrene, pyrrol, benzimidazole, pyrazole, pyrazine, pyridine, pyrimidine and indole. The term "heteroaryl" also includes bicyclic or tricyclic ring systems that are not completely aromatic but contain an aromatic ring. The heteroatoms may be present within the ring system in the aromatic ring or in a non-aromatic ring. For example heteroaryl also encompasses chromene, chromane, indoline, tetrahydroquinoline,

The term "halogen" herein includes reference to F, Cl, Br and I. Halogen may be Br. Halogen may be I.

A bond terminating in a

“ 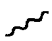 ”

represents that the bond is connected to another atom that is not shown in the structure. A bond terminating inside a cyclic structure and not terminating at an atom of the ring structure represents that the bond may be connected to any of the atoms in the ring structure where allowed by valency.

A bond drawn as a solid line and a dotted line represents a bond which can be either a single bond or a double bond, where chemically possible. For example, the bond drawn below could be a single bond or a double bond.

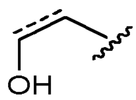

Where a moiety is substituted, it may be substituted at any point on the moiety where chemically possible and consistent with atomic valency requirements. The moiety may be substituted by one or more substituents, e.g. 1, 2, 3 or 4 substituents; optionally there are 1 or 2 substituents on a group. Where there are two or more substituents, the substituents may be the same or different. The substituent(s) may be selected from: OH, NHR, amidino, guanidino, hydroxyguanidino, formamidino, isothioureido, ureido, mercapto, C(O)H, acyl, acyloxy, carboxy, sulfo, sulfamoyl, carbamoyl, cyano, azo, nitro, halo, $C_{1-6}$ alkyl, $C_{1-6}$ alkoxy, $C_{1-6}$ haloalkyl, $C_{3-8}$ cycloalkyl, $C_{2-6}$ alkenyl, $C_{2-6}$ alkynyl, aryl, heteroaryl or alkaryl. Where the group to be substituted is an alkyl group the substituent may be =O. R may be selected from H, $C_{1-6}$ alkyl, $C_{3-8}$ cycloalkyl, phenyl, benzyl or phenethyl group, e.g. R is H or $C_{1-3}$ alkyl. Where the moiety is substituted with two or more substituents and two of the substituents are adjacent the adjacent substituents may form a $C_{4-8}$ ring along with the atoms of the moiety on which the substituents are substituted, wherein the $C_{4-8}$ ring is a saturated or unsaturated hydrocarbon ring with 4, 5, 6, 7, or 8 carbon atoms or a saturated or unsaturated hydrocarbon ring with 4, 5, 6, 7, or 8 carbon atoms and 1, 2 or 3 heteroatoms.

Substituents are only present at positions where they are chemically possible, the person skilled in the art being able to decide (either experimentally or theoretically) without inappropriate effort which substitutions are chemically possible and which are not.

Ortho, meta and para substitution are well understood terms in the art. For the absence of doubt, "ortho" substitution is a substitution pattern where adjacent carbons possess a substituent, whether a simple group, for example the fluoro group in the example below, or other portions of the molecule, as indicated by the bond ending in

“ 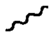 ”.

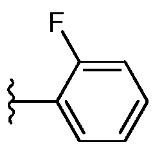 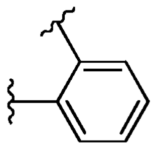 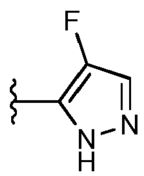 .

"Meta" substitution is a substitution pattern where two substituents are on carbons one carbon removed from each other, i.e with a single carbon atom between the substituted carbons. In other words there is a substituent on the second atom away from the atom with another substituent. For example the groups below are meta substituted.

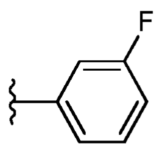 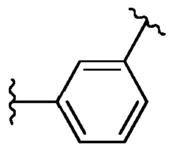 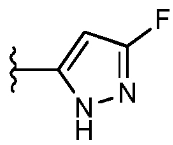 .

"Para" substitution is a substitution pattern where two substituents are on carbons two carbons removed from each other, i.e with two carbon atoms between the substituted carbons. In other words there is a substituent on the third atom away from the atom with another substituent. For example the groups below are para substituted.

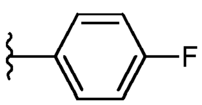 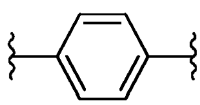 .

By "acyl" is meant an organic radical derived from, for example, an organic acid by the removal of the hydroxyl group, e.g. a radical having the formula R-C(O)-, where R may be selected from H, $C_{1-6}$ alkyl, $C_{3-8}$ cycloalkyl, phenyl, benzyl or phenethyl group, eg R is H or $C_{1-3}$ alkyl. In one embodiment acyl is alkyl-carbonyl. Examples of acyl groups include, but are not limited to, formyl, acetyl, propionyl and butyryl. A particular acyl group is acetyl.

Throughout the description the disclosure of a compound also encompasses pharmaceutically acceptable salts, solvates and stereoisomers thereof. Where a compound has a stereocentre, both (R) and (S) stereoisomers are

contemplated by the invention, equally mixtures of stereoisomers or a racemic mixture are completed by the present application. Where a compound of the invention has two or more stereocentres any combination of (R) and (S) stereoisomers is contemplated. The combination of (R) and (S) stereoisomers may result in a diastereomeric mixture or a single diastereoisomer. The compounds of the invention may be present as a single stereoisomer or may be mixtures of stereoisomers, for example racemic mixtures and other enantiomeric mixtures, and diasteroemeric mixtures. Where the mixture is a mixture of enantiomers the enantiomeric excess may be any of those disclosed above. Where the compound is a single stereoisomer the compounds may still contain other diasteroisomers or enantiomers as impurities. Hence a single stereoisomer does not necessarily have an enantiomeric excess (e.e.) or diastereomeric excess (d.e.) of 100% but could have an e.e. or d.e. of about at least 85%, at least 60% or less. For example, the e.e. or d.e. may be 90% or more, 90% or more, 80% or more, 70% or more, 60% or more, 50% or more, 40% or more, 30% or more, 20% or more, or 10% or more.

The invention contemplates pharmaceutically acceptable salts of the compounds of the invention. These may include the acid addition and base salts of the compounds. These may be acid addition and base salts of the compounds. In addition the invention contemplates solvates of the compounds. These may be hydrates or other solvated forms of the compound.

Suitable acid addition salts are formed from acids which form non-toxic salts. Examples include the acetate, aspartate, benzoate, besylate, bicarbonate/carbonate, bisulfate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 1,5-naphthalenedisulfonate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulfate and hemicalcium salts. For a review on suitable salts, see "Handbook of Pharmaceutical Salts: Properties, Selection, and Use" by Stahl and Wermuth (Wiley-VCH, Weinheim, Germany, 2002).

Pharmaceutically acceptable salts of compounds of formula (I) may be prepared by one or more of three methods:

(i) by reacting the compound of the invention with the desired acid or base;
(ii) by removing an acid- or base-labile protecting group from a suitable precursor of the compound of the invention or by ring-opening a suitable cyclic precursor, for example, a lactone or lactam, using the desired acid or base; or
(iii) by converting one salt of the compound of the invention to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of the invention may exist in both unsolvated and solvated forms. The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionised, partially ionised, or non-ionised. For a review of such complexes, see J Pharm Sci, 64 (8), 1269-1288 by Haleblian (August 1975).

Hereinafter all references to compounds of any formula include references to salts, solvates and complexes thereof and to solvates and complexes of salts thereof.

The compounds of the invention include compounds of a number of formula as herein defined, including all polymorphs and crystal habits thereof, prodrugs and isomers thereof (including optical, geometric and tautomeric isomers) as hereinafter defined and isotopically-labelled compounds of the invention.

The present invention also includes all pharmaceutically acceptable isotopically-labelled compounds of the invention wherein one or more atoms are replaced by atoms having the same atomic number, but an atomic mass or mass number different from the atomic mass or mass number most commonly found in nature.

Examples of isotopes suitable for inclusion in the compounds of the invention include isotopes of hydrogen, such as $^{2}H$ and $^{3}H$, carbon, such as $^{11}C$, $^{13}C$ and $^{14}C$, chlorine, such as $^{36}Cl$, fluorine, such as $^{18}F$, iodine, such as $^{123}I$ and $^{125}I$, nitrogen, such as $^{13}N$ and $^{15}N$, oxygen, such as $^{15}O$, $^{17}O$ and $^{18}O$, phosphorus, such as $^{32}P$, and sulphur, such as $^{35}S$.

Certain isotopically-labelled compounds, for example, those incorporating a radioactive isotope, are useful in

drug and/or substrate tissue distribution studies. The radioactive isotopes tritium, i.e. $^3H$, and carbon-14, i.e. $^{14}C$, are particularly useful for this purpose in view of their ease of incorporation and ready means of detection.

Substitution with heavier isotopes such as deuterium, i.e. $^2H$, may afford certain therapeutic advantages resulting from greater metabolic stability, for example, increased in vivo half-life or reduced dosage requirements, and hence may be preferred in some circumstances.

Before purification, the compounds of the present invention may exist as a mixture of enantiomers depending on the synthetic procedure used. The enantiomers can be separated by conventional techniques known in the art. Thus the invention covers individual enantiomers as well as mixtures thereof.

For some of the steps of the process of preparation of the compounds of the invention, it may be necessary to protect potential reactive functions that are not wished to react, and to cleave said protecting groups in consequence. In such a case, any compatible protecting radical can be used. In particular methods of protection and deprotection such as those described by T.W. GREENE (Protective Groups in Organic Synthesis, A. Wiley- Interscience Publication, 1981) or by P. J. Kocienski (Protecting groups, Georg Thieme Verlag, 1994), can be used. All of the above reactions and the preparations of novel starting materials used in the preceding methods are conventional and appropriate reagents and reaction conditions for their performance or preparation as well as procedures for isolating the desired products will be well-known to those skilled in the art with reference to literature precedents and the examples and preparations hereto.

Also, the compounds of the present invention as well as intermediates for the preparation thereof can be purified according to various well-known methods, such as for example crystallization or chromatography.

One or more compounds of the invention may be combined with one or more pharmaceutical agents, for example anti-inflammatory agents, anti-fibrotic agents, chemotherapeutics, anti cancer agents, immunosuppressants, anti-tumour vaccines, , cytokine therapy, or tyrosine kinase inhibitors, for the treatment of conditions modulated by the inhibition of ROCK, for example fibrotic diseases, auto-immune, inflammatory-fibrotic conditions, inflammatory conditions, central nervous system disorders, or cancer.

Such combination treatment may be achieved by way of the simultaneous, sequential or separate dosing of the individual components of the treatment. Such combination products employ the compounds of this invention within a therapeutically effective dosage range described hereinbefore and the other pharmaceutically-active agent within its approved dosage range.

The compounds of the invention can be administered in vivo either alone or in combination with other pharmaceutically active agents, e.g. agents effective in particular for the treatment and/or prophylaxis of the aforementioned diseases. A suitable combination consists of a compound of the present invention with one or more active substances which may be mentioned by way of example and preferably are: lipid-lowering agents, in particular HMG-CoA-(3-hydroxy-3-methylglutaryl-coenzyme A)-reductase inhibitors; coronary therapeutics/vasodilators, in particular ACE (angiotensin converting enzyme) inhibitors; All (angiotensin II) receptor antagonists; β-adrenoceptor antagonists; alpha-1-adrenoceptor antagonists; diuretics; calcium channel blockers; substances which bring about an increase in cyclic guanosine monophosphate (cOMP), such as, for example, stimulators of soluble guanylate cyclase; plasminogen activators (thrombolytics/fibrinolytics) and thrombolysis/fibrinolysis-increasing compounds such as inhibitors of the plasminogen activator inhibitor (PAI inhibitors) or inhibitors of the thrombin-activated fibrinolysis inhibitor (TAFI); substances having anticoagulatory activity (anticoagulants); substances inhibiting platelet aggregation (platelet aggregation inhibitors, thrombocyte aggregation inhibitors); and fibrinogen receptor antagonists (glycoprotein IIb/IIIa antagonists).

The compounds of the invention may be advantageous in the treatment of cancer since cancer patients have a pro-thrombotic state and may need anticoagulants. This normally has to be balanced with risk of bleeding, therefore, the compounds described herein offer a safer anticoagulant in cancer patients because of the reduced risk of bleeding. For the treatment of cancer the compounds of the invention may be administered in combination with known cancer treating therapies.

Compounds of the invention may exist in a single crystal form or in a mixture of crystal forms or they may be amorphous. Thus, compounds of the invention intended for pharmaceutical use may be administered as crystalline or amorphous products. They may be obtained, for example, as solid plugs, powders, or films by methods such as precipitation, crystallization, freeze drying, or spray drying, or evaporative drying. Microwave or radio frequency drying may be used for this purpose.

For the above-mentioned compounds of the invention the dosage administered will, of course, vary with the compound employed, the mode of administration, the treatment desired and the disorder indicated. For example, if the compound of the invention is administered orally, then the daily dosage of the compound of the invention may be in the range from 0.01 micrograms per kilogram body weight (μg/kg) to 100 milligrams per kilogram body weight (mg/kg).

A compound of the invention, or pharmaceutically acceptable salt thereof, may be used on their own but will generally be administered in the form of a pharmaceutical composition in which the compounds of the invention, or pharmaceutically acceptable salt thereof, is in association with a pharmaceutically acceptable adjuvant, diluent or carrier. Conventional procedures for the selection and preparation of suitable pharmaceutical formulations are described in, for example, "Pharmaceuticals - The Science of Dosage Form Designs", M. E. Aulton, Churchill Livingstone, 1988.

Depending on the mode of administration of the compounds of the invention, the pharmaceutical composition which is used to administer the compounds of the invention will preferably comprise from 0.05 to 99 %w (per cent by weight) compounds of the invention, more preferably from 0.05 to 80 %w compounds of the invention, still more preferably from 0.10 to 70 %w compounds of the invention, and even more preferably from 0.10 to 50 %w compounds of the invention, all percentages by weight being based on total composition.

The pharmaceutical compositions may be administered topically (e.g. to the skin) in the form, e.g., of creams, gels, lotions, solutions, suspensions, or systemically, e.g. by oral administration in the form of tablets, capsules, syrups, powders or granules; or by parenteral administration in the form of a sterile solution, suspension or emulsion for injection (including intravenous, subcutaneous, intramuscular, intravascular or infusion); by rectal administration in the form of suppositories; or by inhalation in the form of an aerosol.

For oral administration the compounds of the invention may be admixed with an adjuvant or a carrier, for example, lactose, saccharose, sorbitol, mannitol; a starch, for example, potato starch, corn starch or amylopectin; a cellulose derivative; a binder, for example, gelatine or polyvinylpyrrolidone; and/or a lubricant, for example, magnesium stearate, calcium stearate, polyethylene glycol, a wax, paraffin, and the like, and then compressed into tablets. If coated tablets are required, the cores, prepared as described above, may be coated with a concentrated sugar solution which may contain, for example, gum arabic, gelatine, talcum and titanium dioxide. Alternatively, the tablet may be coated with a suitable polymer dissolved in a readily volatile organic solvent.

For the preparation of soft gelatine capsules, the compounds of the invention may be admixed with, for example, a vegetable oil or polyethylene glycol. Hard gelatine capsules may contain granules of the compound using either the above-mentioned excipients for tablets. Also liquid or semisolid formulations of the compound of the invention may be filled into hard gelatine capsules. Liquid preparations for oral application may be in the form of syrups or suspensions, for example, solutions containing the compound of the invention, the balance being sugar and a mixture of ethanol, water, glycerol and propylene glycol. Optionally such liquid preparations may contain colouring agents, flavouring agents, sweetening agents (such as saccharine), preservative agents and/or carboxymethylcellulose as a thickening agent or other excipients known to those skilled in art.

For intravenous (parenteral) administration the compounds of the invention may be administered as a sterile aqueous or oily solution.

The size of the dose for therapeutic purposes of compounds of the invention will naturally vary according to the nature and severity of the conditions, the age and sex of the animal or patient and the route of administration, according to well-known principles of medicine.

Dosage levels, dose frequency, and treatment durations of compounds of the invention are expected to differ depending on the formulation and clinical indication, age, and co-morbid medical conditions of the patient. The standard duration of treatment with compounds of the invention may be any length of time. For example, the treatment duration may be days, weeks, months or years. The treatment may be indefinite. It may be that the treatment may be for between one and seven months for most clinical indications. It may be necessary to extend the duration of treatment beyond seven months in instances of recurrent infections or infections associated with tissues or implanted materials to which there is poor blood supply including bones/joints, respiratory tract, endocardium, and dental tissues.

## EXAMPLES AND SYNTHESIS

1H-NMR: Spectra are obtained on a Bruker DRX 400MHz or Jeol ECS 400MHz spectrometer. Spectra are measured at 294K (unless otherwise stated) and chemical shifts ($\delta$-values) are reported in parts per million (ppm), referenced to either TMS (0.0 ppm), DMSO-d6 (2.50 ppm), CDCl3 (7.26 ppm). Coupling constants (J) are reported in Hertz (Hz), spectra splitting pattern are designated as singlet (s), doublet (d), triplet (t), quadruplet (q), multiplet or more overlapping signals (m), broad signal (br); solvent is given in parentheses.

Abbreviations

The following abbreviations are used in the Examples and other parts of the description.

ABCN : azobis cyclohexanecarbonitrile; Boc: *tert*-butyloxycarbonyl; Cbz: Carbobenzyloxy; DavePhos: 2-dicyclohexylphosphino-2'-(*N,N*-dimethylamino)biphenyl; dba: tris(dibenzylideneacetone); DBU: 1,8-diazabicyclo[5.4.0]undec-7-ene; DCE: 1,2-dichloroethane; DCM: dichloromethane; DIAD: diisopropyl azodicarboxylate; dioxane: 1,4-dioxane; DIPEA: Diisopropyl ethylamine; DMA: dimethyl acetamide; DMAP: 4-(dimethylamino)pyridine; DMF: *N,N*-dimethylformamide; DMS: Dimethylsulfide; DMSO: dimethylsulfoxide; Dppf: 1,1'-bis(diphenylphosphino)ferrocene; dtbpf: ([1,1'-bis(di-tert-butylphosphino)ferrocene]; EtOAc: ethyl acetate; Fmoc: 9-Fluorenylmethoxycarbonyl; h: hour(s); HATU: 2-(7-aza-1H-benzotriazole-1-yl)-1,1,3,3-tetramethyluronium. Hexafluorophosphate; HBTU: (2-(1H-benzotriazol-1-yl)-1,1,3,3-tetramethyluronium hexafluorophosphate; HPLC: High-performance liquid chromatography; MIDA: N-methyliminodiacetic acid; min: minute(s); LCMS: Liquid chromatography - mass spectrometry; MS: mass spectroscopy; Ms:

Mesyl; Pet-ether: petroleum ether (b.p. 60-80°C); quant.: quantitative (conversion); Rt: retention time; RT: room temperature; SCX: strong cation exchange; TEA: triethylamine; TFA: trifluoroacetic acid; THF: tetrahydrofuran; TsCl: p-toluenesulfonyl chloride; XPhos: 2-dicyclohexylphosphino-2',4',6'-triisopropylbiphenyl; XantPhos: 4,5-bis(diphenylphosphino)-9,9-dimethylxanthene;

Analytical methods

Analysis of products and intermediates has been carried out using reverse phase analytical HPLC-MS using the parameters set out below.

HPLC Analytical Methods:

**AnalpH2_MeOH_4min:** Phenomenex Luna C18 (2) 3 μm, 50 x 4.6 mm; A = water + 0.1% formic acid; B = MeOH + 0.1% formic acid; 45 °C; %B: 0.0 min 5%, 1.0 min 37.5%, 3.0 min 95%, 3.5 min 95%,3.51 min 5%, 4.0 min 5%; 2.25 mL/min.

**AnalpH2_50-95MeOH_4min:** Phenomenex Luna C18 (2) 3 μm, 50 x 4.6 mm; A = water + 0.1% formic acid; B = MeOH + 0.1% formic acid; 45 °C; %B: 0.0 min 50%, 1.5 min 95%, 3.5 min 95%,3.51 min 5%, 4.0 min 5%; 2.25 mL/min.

**AnalpH9_MeOH_4min:** Phenomenex Luna C18 (2) 3 μm, 50 x 4.6 mm; A = water pH 9 (Ammonium Bicarbonate 10 mM); B = MeOH + 0.1% formic acid; 45 °C; %B: 0.0 min 5%, 1.0 min 37.5%, 3.0 min 95%, 3.5 min 95%, 3.51 5%, 4.0 min 5%; 2.25 mL/min.

**AnalpH2_MeOH_QC_V1:** Phenomenex Gemini NX C18 5 μm, 150 x 4.6 mm; A = water + 0.1% formic acid; B = MeOH + 0.1% formic acid; 40 °C; %B: 0.0 min 5%, 0.5 min, 5%, 7.5 min 95%, 10.0 min 95%, 10.1 min 5%, 13.0 min 5%; 1.5 mL/min.

**AnalpH9_MeOH_QC_V1:** Phenomenex Gemini NX C18 5 μm, 150 x 4.6 mm; A = water + pH 9 (Ammonium Bicarbonate 10 mM); B = MeOH; 40 °C; %B: 0.0 min 5%, 0.50 min 5%, 7.5 min 95%, 10.0 min 95%, 10.1 min 5%, 13.0 min 5%; 1.5 mL/min.

**Agilent_MeCN_HPLC_3min:** Phenomenex Luna C18, 50x2mm: A = water + 0.1% formic acid; B = MeCN + 0.1% formic acid; 5-95% B 0-3 min; 1 mL/min

UPLC Analytical Methods

**AnalpH2_MeCN_UPLC_3.8min:** Acquity UPLC BEH C-18 1.7um, 2.1 x 50 mm, A = water + 0.05% formic acid; B: acetonitrile + 0.05% formic acid; 35°C; %B: 0.0 min 10%, 0.5 min 10%, 1 min 35%, 1.5min 45%, 2.3min 90%, 3.2min 90%, 3.8min 10%; 0.55 mL/min

**AnalpH2_MeCN_UPLC_4.0min:** Acquity UPLC BEH C-18 1.7um, 2.1 x 50 mm, A = water + 0.05% formic acid; B: acetonitrile + 0.05% formic acid; 35°C; %B: 0.0 min 10%, 0.5 min 10%, 1 min 35%, 1.5min 45%, 2.3min 90%, 3.2min 90%, 3.6min 10%, 4.0min 10%; 0.55 mL/min

**AnalpH2_MeCN_UPLC_4.2min:** Acquity UPLC BEH C-18 1.7um, 2.1 x 50 mm, A = water + 0.05% formic acid; B: acetonitrile + 0.05% formic acid; 40°C; %A: 0.0 min 95%, 0.3 min 95%, 2 min 5%, 3.5min5%, 3.6min 95%, 4.2min 95%; 0.6 mL/min

**AnalpH2_MeCN_UPLC_5.0min:** Acquity UPLC BEH C-18 1.7um, 2.1 x 50 mm, A = water + 0.05% formic acid; B: acetonitrile + 0.05% formic acid; 40°C; %A: 0.0 min 50%, 3.0 min 90%, 5.0min 90%, 5.1 min 50%; 0.4 mL/min

**AnalpH2_MeCN_UPLC_6.1min:** Acquity UPLC BEH C-18 1.7um, 2.1 x 100 mm, A = water + 0.05% formic acid; B: acetonitrile + 0.05% formic acid; 40°C; %A: 0.0 min 60%, 2.0 min 90%, 6.0min 90%, 6.1 min 60%; 0.3 mL/min

**AnalpH9_MeCN_UPLC_10min:** Acquity UPLC BEH C-18 1.7um, 2.1 x 50 mm, A = 5 mM ammonium acetate in water; B: acetonitrile; 40°C; %B: 0.0 min 3%, 1.0 min 3%, 7.0 min 100%, 7.5 min 100%, 9.0 min 3%, 10 min 3%; 0.5 mL/min

**Thermo_MeOH_UHPLC_1.2 min:** Phenomenex Kinetex, 2.6 uM, 50 x 2.1mm, A = water + 0.1% formic acid; B = MeOH + 0.1% formic acid; 2-95% B 0-1.0 min; 1.3 mL/min

**General Methods**

**General Method 1 (GM1): Amide coupling**

A mixture of carboxylic acid (1.0 eq), amine (1.0-1.5 eq), N,N-diisopropylethylamine or triethylamine (1.5-5.0 eq) and a coupling agent such as HBTU (2-(1H-benzotriazol-1-yl)-1,1,3,3-tetramethyluronium hexafluorophosphate), HATU (1-[Bis(dimethylamino)methylene]-1H-1,2,3-triazolo[4,5-b]pyridinium 3-oxidhexafluorophosphate), HCTU (O-(1H-6-Chlorobenzotriazole-1-yl)-1,1,3,3-tetramethyluronium hexafluorophosphate (1.0-1.5 eq) or TBTU 2-(1H-Benzotriazole-1-yl)-1,1,3,3-tetramethylaminium tetrafluoroborate in anhydrous solvents such as DMF, DCM or MeCN was stirred

at room temperature for 1-72 h. The product was isolated and purified using one of the following methods:

a) The reaction mixture was diluted with a mixture of water and aqueous sat. NaCl solution and extracted with EtOAc. The organic phase was dried over $Na_2SO_4$ or $MgSO_4$, filtered and concentrated *in vacuo* to yield the crude material which was either used without further purification, or purified by column chromatography.
b) The solvent was removed *in vacuo* and the residue dissolved in EtOAc, and the organic phase washed with $NaHCO_3$(aq) solution, $H_2O$ then brine. The organic phase was dried over $Na_2SO_4$ or $MgSO_4$, filtered and the filtrate concentrated *in vacuo* to yield the crude material which was either used without further purification, or purified by column chromatography.
c) The reaction was diluted with water or aqueous sat. NaCl solution and extracted with DCM. The organic phase was dried over $MgSO_4$ and filtered, or passed through a hydrophobic frit and concentrated *in vacuo.* Crude material was either used without further purification, or purified by column chromatography.
d) The reaction was cooled in an ice bath and diluted with water and extracted with EtOAc. The organic phase was washed sequentially with $NaHCO3_3$ (aq) solution, $NH_4Cl$ (aq) and brine, then dried over $Na_2SO_4$, filtered and the filtrate concentrated *in vacuo* to yield the crude material which was purified by column chromatography.

**General Method 2 (GM2): Boc deprotection**

**Method Boc deprotection 2A:** Boc-protected amine was stirred in a mixture of DCM:TFA (in a ratio from 10:1 to 1:1) for 1-18h.
**Method Boc deprotection 2B:** Boc-protected amine was dissolved in EtOAc or DCM and either 4M HCl in dioxane or 1 M HCl in $Et_2O$ added. The reaction mixture was stirred at room temperature for 0.25-18h.
**Method Boc deprotection 2C:** The crude Boc-protected amine in DCM was passed through a MP-TsOH cartridge, washed with MeOH (up to 5 column volumes) and eluted with 2M $NH_3$-MeOH.
The reaction mixture (or product-containing fractions : Method 2C) were concentrated *in vacuo* to yield the crude material which was either used crude, or purified by one of the following methods:

**a)** SCX-2 followed by prep HPLC
**b)** Basified by addition of 1M $NH_3$ in MeOH, concentrated *in vacuo* and purified by prep HPLC
**c)** Diluted with 0.5N HCl $_{(aq)}$ and EtOAc and the layers separated. The aqueous phase may be washed with EtOAc then basified (pH ≈ 10) and extracted with EtOAc. The combined organic extracts dried ($MgSO_4$), filtered and the solvent evaporated *in vacuo.*
**d)** Reverse-phase chromatography
**e)** Water and sat. aq. $NaHCO_3$ added. The product was extracted into EtOAc, and the organic extract dried ($MgSO_4$) and the solvent removed *in vacuo.*
**f)** Prep HPLC optionally followed by SCX-2
**g)** SCX-2 optionally followed by addition of 4 M HCl in dioxane and the solvent removed to give the HCl salt

**General Method 3 (GM3): Hydrogenation**

**General Method 3A (hydrogenation with $H_2$ balloon):** The alkene or Cbz protected species (1 eq) was dissolved in EtOH or MeOH, placed under $N_2$ atmosphere, and Pd/C or $Pd(OH)_2$ (10wt%) added. A $H_2$ atmosphere was introduced and the reaction mixture stirred at room temperature for 1-72h. The mixture was filtered through celite and the filtrate concentrated to give the crude product which was used with no further purification.
Optionally additional aliquots of Pd/C may be added during the course of the reaction.
**General Method 3B (hydrogenation with ammonium formate):** The alkene (1 eq) was dissolved in EtOH and Pd/C (0.5 eq), and $NH_4HCO_2$ (10 eq) added. The mixture was stirred at reflux for 1-72h. The solution was cooled to room temperature and filtered through celite, washing with MeOH or EtOAc. The solvent were evaporated *in vacuo* and the residue partitioned between EtOAc and sat. aq. $NaHCO_3$. The organic phase was dried ($MgSO_4$), filtered and the solvent removed to yield the crude product which was used without further purification.
Optionally additional aliquots of Pd/C and/or $NH_4HCO_2$ may be added during the course of the reaction.
**General Method 3C (hydrogenation with H-Cube):** The alkene was dissolved in a protic solvent and passed through a H-cube reactor (Pd/C cartridge), typical conditions: 30 °C, 20 bar, 1 mL/min. The solvent was evaporated *in vacuo* and the material used without further purification.

**General Method 4 (GM4)** : **Ester Hydrolysis**

**Method Ester Hydrolysis 4A:** The ester (1.0 eq) was dissolved in MeOH or 1,4-dioxane, and 1M LiOH (1-2 eq)

added and stirred at room temperature for 1-64h.

Optionally additional equivalents of 1M LiOH (aq) may be added during the reaction.

**Method Ester Hydrolysis 4B:** The ester (1.0 eq) was dissolved in a 1:1:1 solution of 1M NaOH/MeOH/THF and stirred at room temperature for 1-18h.

**Method Ester Hydrolysis 4C:** The ester (1.0 eq) was dissolved in 10M NaOH (5 equiv) in MeOH, and stirred at 60 °C for 1-18h.

The solvent was removed *in vacuo* and the product isolated using one of the following methods:

a) Crude product used without further purification.
**b)** Diluted with water and acidified to pH4, then extracted with EtOAc or DCM. The organic extracts were dried over $MgSO_4$, filtered and the solvent removed to yield the product which was used without further purification.
**c)** The crude product was dissolved in water and the aqueous layer washed with EtOAc or DCM. The aqueous layer was acidified with 1M HCl and the product extracted into EtOAc or DCM. The combined organic extracts were dried ($MgSO_4$), filtered and the solvent removed to yield the product which was used without further purification.
**d)** 2N HCl added, adjusting to pH 7. The solvent was evaporated *in vacuo* and the residue dissolved in DCM. The solution was filtered and the filtrate concentrated under reduced pressure, and the resulting solid residue dried *in vacuo.*

**General Method 5 (GM5): Fmoc Deprotection**

Fmoc-protected amine was stirred in a 10:1 mixture of piperidine and either DCM or DMF at room temperature for 1-18h. The solvent was removed under reduced pressure, and the residue either used without further purification, or was purified by column chromatography or prep HPLC.

**General Method 6 (GM6): Nitrile reduction with $NaBH_4/NiCl_2.6H_2O$**

**Method $NaBH_4/NiCl_2.6H_2O$ *in situ* Boc protection 6:** To the benzonitrile (1eq) in MeOH at 0°C was added $NiCl_2.6H_20$ (0.1eq) and $Boc_2O$ (2eq), followed by portionwise addition of $NaBH_4$ (10 eq). Reaction temperature was maintained < 5 °C. Stirred for 1-2h. Further addition of $NiCl_2.6H_20$ and $NaBH_4$ added if required. The reaction mixture was concentrated *in vacuo,* then suspended in sat. $NaHCO_{3(aq)}$ sol. and water and extracted with EtOAc. Organic phases were dried over $Na_2SO_4$, filtered and concentrated *in vacuo.* Crude product purified using column chromatography.

**General Method 7 (GM7):** Carbamate formation using chloroformates

A mixture of amine (1.0 eq) and $Et_3N$ (2.0 eq) in anhydrous DCM under $N_2$ was cooled to 0°C and chloroformate added (1.5 eq). After 5 min, mixture warmed to RT and stirred for 1-3 h. Further $Et_3N$ and chloroformate added if required. Reaction mixture was diluted with DCM and washed with sat. $NaHCO_{3(aq)}$ solution and brine, dried over $Na_2SO_4$, filtered and concentrated *in vacuo.* Purified using column chromatography.

**General Method 8 (GM8)- Amide coupling with T3P**

To a stirred suspension of acid (1.0-1.1 eq dependant on whether acid or amine is limiting reagent), amine (1.0-1.1eq dependant on whether acid or amine is limiting reagent) and $Et_3N$ (4.0-5.0 eq) was added dropwise T3P (50% in EtOAc)(2.0 eq) at 0°C. Suspension was warmed to RT and stirred for until starting material fully consumed, then cooled to 0°C, diluted with water and stirred for a further 30 min. The aqueous solution was extracted with EtOAc (x3) and the combined organic phases were washed with water (x1) and brine (x1), dried over $Na_2SO_4$ and concentrated *in vacuo.* Crude product purified using either:

a) Column chromatography

b) SCX-2 followed by addition of 4 M HCl in dioxane and the solvent removed to give the HCl salt

**General Method 9 (GM9)- Carbamate formation using triphosgene**

To a solution of amine (1.0 eq), alcohol (2.0 eq), pyridine (3.0 eq) in anhydrous DCM under $N_2$ was added triphosgene (0.5 eq) and the RT stirred for (17-20 h). The reaction was the quenched with water and extracted with DCM. Organic phase dried over $Na_2SO_4$ and concentrated *in vacuo.* Crude mixture purified by column chromatography.

**General Method 10 (GM10): Sulfonamide formation**

To a solution of amine (1.0 eq) in anhydrous DCM under $N_2$ was added DIPEA (1.5 eq), then sulfonyl chloride (1.0 eq) and the reaction stirred at RT for 3-24h. Further DIPEA and sulfonyl chloride added if required. Reaction mixture was diluted with DCM and washed sequentially with sat. $NaHCO_3$ solution and brine and dried ($Na_2SO_4$). Crude products were purified using column chromatography.

**General Method 11 (GM11): Cross coupling reaction**

To a solution of amine (1.0 eq) in DCM and DMF (4:1 solvent ratio) was added boronic acid (5.0 eq), $Et_3N$ (5.0 eq) and $Cu(OAc)_2$ monohydrate (2.5 eq) and the reaction stirred at RT for up to 7 days. Volatile solvent was removed *in vacuo* and the crude mixture diluted with water. The resulting precipitate was collected by filtration and the aqueous solution extracted with EtOAc. The combined organic phases were washed with brine and dried ($Na_2SO_4$), before combining with the material collected by filtration. Crude product purified using a combination of column chromatography and SCX cartridges.

**General schemes**

Synthesis of RgA: **C-(1-Methyl-1H-benzotriazol-5-yl)-methylamine**

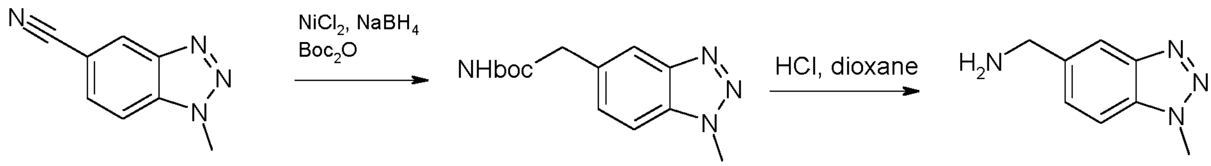


Step-1: *tert*-butyl (1-methyl-1H-benzo[d][1,2,3]triazol-5-yl)methylcarbamate

To a stirred solution of 1-methyl-1H-benzotriazole-5-carbonitrile (12 g, 38 mmol) in dry methanol (300 mL) were added $Boc_2O$ (33g, 0.075 mmol) and $NiCl_2 \cdot 6H_2O$ (1g, 3.8 mmol) at 0 °C. $NaBH_4$ (20 g, 26.6 mmol) was then added portionwise over 30 minutes and the reaction mixture allowed to warm to room temperature and stirring continued for an additional 1 h. After 1h, diethylenetriamine (4.0 mL, 38.0 mmol) was added and stirring continued for further 30 minutes. After completion of the reaction, the reaction mixture was concentrated under reduced pressure to afford a purple residue which was dissolved in EtOAc (100 mL) and washed with saturated aqueous $NaHCO_3$ (2x50 mL). The organic extract was dried over anhydrous $Na_2SO_4$, filtered and concentrated under reduced pressure to afford (1-methyl-1H-benzotriazol-5-ylmethyl)-carbamic acid tert-butyl ester (10 g, 80%) as a white solid.

Step-2: (1-methyl-1H-benzo[d][1,2,3]triazol-5-yl)methanamine hydrochloride

To a solution of HCl in 1,4-dioxane (12 mL, 4M) was added (1-methyl-1H-benzotriazol-5-ylmethyl)-carbamic acid tert-butyl ester (10 g, 74.1 mmol) at 0 °C and stirred for 4 h. The solid was collected by filtration, washed with diethyl ether and dried to afford (1-methyl-1H-benzo[d][1,2,3]triazol-5-yl)methanamine hydrochloride (6.07 g, 82%) as a white solid.
AnalpH2_MeOH_4MIN: Rt: 1.35 min, m/z 163 $[M+H]^+$

**Synthesis of 6-Aminomethyl-benzo[d]isoxazol-3-ylamine:**

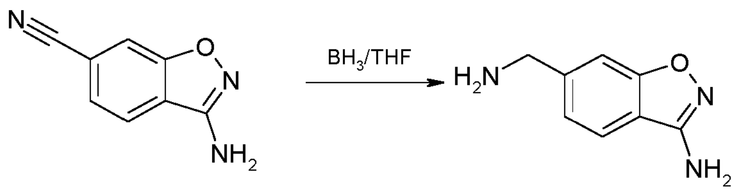

3-Amino-1,2-benzoxazole-6-carbonitrile (225 mg, 1.41 mmol) was dissolved in THF (10 mL) and $BH_3$ (1 M in THF, 4.2 mL, 4.2 mmol) was added dropwise. The reaction mixture was heated at 60 °C for 1 hour, then cooled to room temperature and the reaction quenched with water. The mixture was purified by SCX-2, washing with MeOH and eluting with $NH_3$/MeOH. The product-containing fractions were combined and concentrated *in vacuo* to give 6-aminomethyl-benzo[d]isoxazol-3-ylamine (190 mg, 83%) as a yellow solid.

AnalpH2_MeOH_4MIN: Rt: 1.20min, m/z 164 $[M+H]^+$

**Synthesis of (4-Aminomethyl-benzyl)-carbamic acid 9H-fluoren-9-ylmethyl ester:**

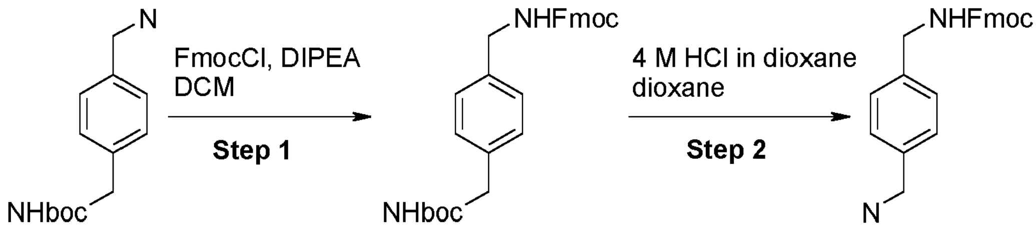


**Step 1:** 1-(N-boc-aminomethyl)-4-(aminomethyl) benzene (3.80g, 16.1 mmol) was dissolved in DCM (100 mL) and DIPEA (5.0 mL, 29 mmol), followed by FmocCl (5.0g, 19 mmol) were added. The reaction mixture was stirred at room temperature for 1 hour, after which a precipitate appeared. Water (100 mL) was added and the precipitate filtered and dried to give [4-(tert-butoxycarbonylaminomethyl)-benzyl]-carbamic acid 9H-fluoren-9-ylmethyl ester (6.32g, 86%) as a white solid.

AnalpH2_MeOH_4MIN: Rt: 3.52 min, m/z 481.3 $[M+H]^+$

**Step** 2: [4-(*tert*-Butoxycarbonylamino-methyl)-benzyl]-carbamic acid 9H-fluoren-9-ylmethyl ester (6.32g, 13.2 mmol) was suspended in dioxane (50 mL) and HCl solution (4 M in dioxane, 20 mL) was added slowly. The reaction mixture was stirred overnight at room temperature, then a further aliquot of 4 M HCl in dioxane (10 mL) was added and the mixture stirred for a further 2 hours at room temperature. The reaction mixture was diluted with ihexane (150 mL), the product collected by filtration and dried *in vacuo* to give (4-aminomethyl-benzyl)-carbamic acid 9H-fluoren-9-ylmethyl ester (4.0g, 73%) as an off-white solid.

AnalpH2_MeOH_4MIN: Rt: 2.32 min, m/z 359.3 $[M+H]^+$

**Synthesis of 4-(aminomethyl)pyridin-2-amine**

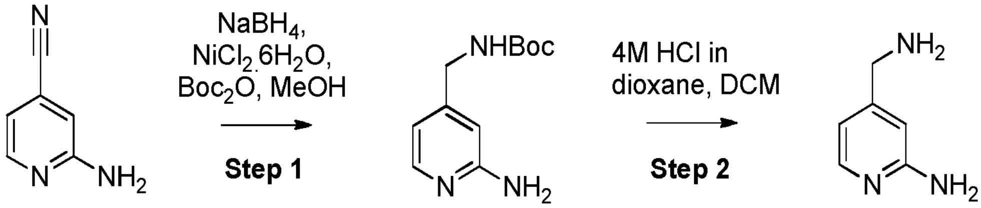


**Step 1:** To a solution of 2-Amino-4-cyanopyridine (0.50 g, 4.2 mmol), $NiCl_2.6H_2O$ (20 mg, 0.08 mmol) and $Boc_2O$ (2.00 g, 9.2 mmol) in methanol (4.5 mL) and THF (6 mL) at -5°C was added $NaBH_4$ (1.52 g, 40 mmol) portionwise over 20 min. The mixture stirred for 1h and the temperature warmed to 10°C. Reaction mixture was then diluted with $NaHCO_{3(aq)}$ (50 mL) and stirred for 15 min. Aqueous solution was then extraction with EtOAc (50 mL). Organic phase was then washed with water (3 x 30 mL) and brine (2 x 30 mL), dried ($Na_2SO_4$) and solvent removed *in vacuo.* The residue was purified by column chromatography hexane/EtOAc 3:2 to 1:2 to 0:100 to give tert-butyl N-[(2-aminopyridin-4-yl)methyl]carbamate as a white solid (540 mg, 58%).

Agilent_MeCN_HPLC_3min **LCMS:** $R_t$ = 1.27 min m/z = 223.8 $[M+H]^+$

**Step 2:** A solution of tert-butyl N-[(2-aminopyridin-4-yl)methyl]carbamate (400 mg, 1.79 mmol) in boiling methanol (2 mL) was cooled to 30°C. 4M HCl in 1,4-dioxane (4.5 mL) was added and the reaction stood for 3h. The 1-4-dioxane was decanted and the resulting cream solid triturated with diethyl ether (4 x 0.75 mL). The resulting pale yellow solid was dried under high vacuum to give 4-(aminomethyl)pyridin-2-amine dihydrochloride in a 96% yield (370 mg).

Agilent_MeCN_HPLC_3min **LCMS:** $R_t$ = 0.14 min m/z = 124.4 $[M+H]^+$

**4-(aminomethyl)-6-methylpyridin-2-amine:**

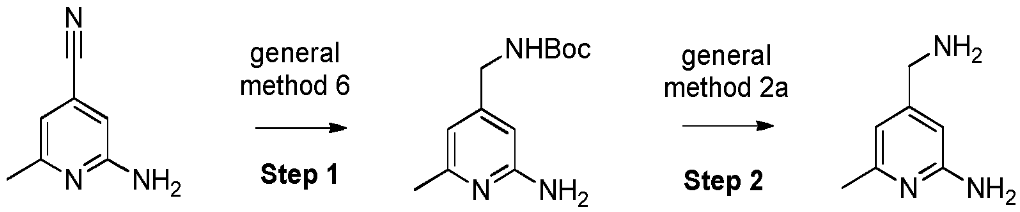


**Step 1:** tert-Butyl N-[(2-amino-6-methylpyridin-4-yl)methyl]carbamate was synthesised following general method 6A from 2-amino-6-methylpyridine-4-carbonitrile (90 mg) in a 33% yield (52mg).

Agilent_MeCN_HPLC_3min LCMS: $R_t$ = 1.42 min m/z = 238.4 $[M+H]^+$

**Step 2:** 4-(aminomethyl)-6-methylpyridin-2-amine synthesised following general method 2B using 4M HCl in 1,4-dioxane from tert-butyl N-[(2-amino-6-methylpyridin-4-yl)methyl]carbamate (160 mg) to give 72 mg, 44%.

**Synthesis of RgD**

**Synthesis of (R)-2-*tert*-Butoxycarbonylamino-5-oxo-5-pyrrolidin-1-yl-pentanoic** acid:

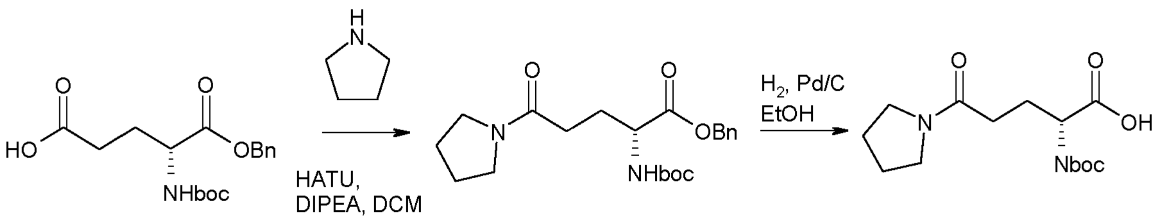


**Step 1:** Boc-D-Glu-OBzl (1.00 mg, 2.96 mmol) was dissolved in DCM (20 mL) and HBTU (1.12 g, 2.96 mmol) and DIPEA (1.53 mL, 8.88 mmol) were added. Pyrrolidine (0.25 mL, 2.96 mmol) was added and the mixture stirred at room temperature for 1h. The reaction mixture was diluted with water (20 mL) and extracted with DCM (2 x 40 mL). The combined organic extracts were dried ($MgSO_4$) and solvent removed *in vacuo*. The residue was purified by column chromatography (Biotage, 50g SNAP, 0-100% EtOAc/ihexane) to give (R)-2-tert-butoxycarbonylamino-5-oxo-5-pyrrolidin-1-yl-pentanoic acid benzyl ester (1.25g, quant) as a colourless oil.

AnalpH2_MeOH_4MIN: Rt: 3.14 min, m/z 391 [M+H]+

**Step 2:** (R)-2-*tert*-Butoxycarbonylamino-5-oxo-5-pyrrolidin-1-yl-pentanoic acid benzyl ester (1.25g, 2.96 mmol) was dissolved in EtOH (20 mL), placed under $N_2$ atmosphere, and Pd/C (100 mg) added. A $H_2$ atmosphere was introduced and the reaction mixture stirred at room temperature for 8h. The reaction mixture was filtered through celite and solvent removed *in vacuo* to give (R)-2-*tert*-butoxycarbonylamino-5-oxo-5-pyrrolidin-1-yl-pentanoic acid (780 mg, 88%) as a white solid.

AnalpH2_MeOH_4MIN: Rt: 2.56 min, m/z 323 [M+Na]+

**Synthesis of (R)-2-Dimethylamino-5-oxo-5-pyrrolidin-1-yl-pentanoic acid**

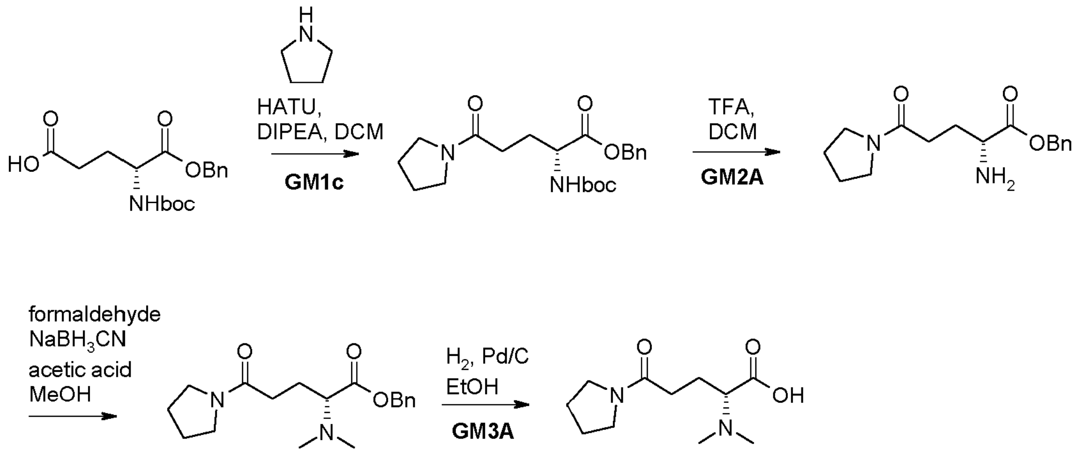


**Step 1:** Amide coupling of Boc-D-Glu-OBzl (2.0g, 5.9 mmol) with pyrrolidine (0.60 mL, 7.1 mmol) using HATU and DIPEA in DCM following General Method 1c. The product was purified using column chromatography (Biotage, 25g SNAP, 20-80% EtOAc/ihexane) to give (R)-2-*tert*-butoxycarbonylamino-5-oxo-5-pyrrolidin-1-yl-pentanoic acid benzyl ester as a colourless oil, which was used directly in subsequent reaction.

ANALPH2_MEOH_4min, Rt: 3.04 min, m/z 391.5 [M+H]+

**Step 2:** Boc deprotection of (R)-2-*tert*-butoxycarbonylamino-5-oxo-5-pyrrolidin-1-yl-pentanoic acid benzyl ester using General Method **2A** for 1.5h followed by purification by SCX-2 followed by drying under vacuum to give (R)-2-amino-5-oxo-5-pyrrolidin-1-yl-pentanoic acid benzyl ester (1.4g, 82% over 2 steps).

ANALPH2_MEOH_4min, Rt: 1.68 min, m/z 291.3 [M+H]+

**Step** 3: Formaldehyde (37% in water, 1 mL), acetic acid (0.5 mL) and $NaBH_3CN$ (0.6 g, 9.4 mmol) were added to a solution of (R)-2-amino-5-oxo-5-pyrrolidin-1-yl-pentanoic acid benzyl ester (1.37g, 4.7 mmol) in methanol (30 mL). The reaction mixture was stirred at room temperature for 2 hours, then the solvent removed *in vacuo.* The residue was partitioned between DCM and 10% $K_2CO_3$ (aq), the aqueous was extracted with DCM and the combined organic extracts dried ($MgSO_4$), and the solvent removed *in vacuo* to give (R)-2-dimethylamino-5-oxo-5-pyrrolidin-1-yl-pentanoic acid benzyl ester (1.7g, quant.) as an opaque oil.

ANALPH2_MEOH_4min, Rt: 2.99 min, m/z 319.4 [M+H]+

**Step 4:** Hydrogenation of (R)-2-dimethylamino-5-oxo-5-pyrrolidin-1-yl-pentanoic acid benzyl ester was carried-out using General Method **3A** for 36 hours. The product was dried *in vacuo* to give (R)-2-dimethylamino-5-oxo-5-pyrrolidin-1-yl-pentanoic acid (1.1g, 100%) as a white solid.

ANALPH2_MEOH_4min, Rt: 0.75min, m/z 229.3 [M+H]+

**Synthesis of (2R)-2-(dimethylamino)-4-phenylbutanoic acid**

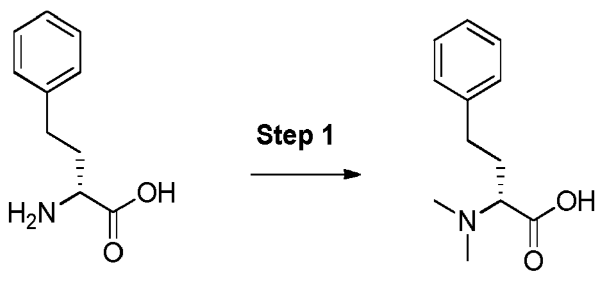


Step 1: To a solution of D-homophenylalanine (448 mg, 2.50 mmol) and paraformaldehyde (675mg, 22.5 mmol) in 2,2,2-trifluoroethanol (12.5 mL) was heated to 65°C and $NaBH_4$ (380 mg, 10.0 mmol) was added portionwise over 5 min. The resulting suspension was heated at 65°C for 20h, then cooled to room temperature and the suspension was filtered. The filtrate was concentrated *in vacuo* to give a pale yellow gum which was triturated with diethyl ether and EtOAc and ultrasonicated to give a cream solid. Recrystallisation from EtOH gave cream crystals (55 mg, 11%).

Agilent_MeCN_HPLC_3min **LCMS:** $R_t$ = 1.05 min m/z = 208.2 $[M+H]^+$

***General Scheme 1***

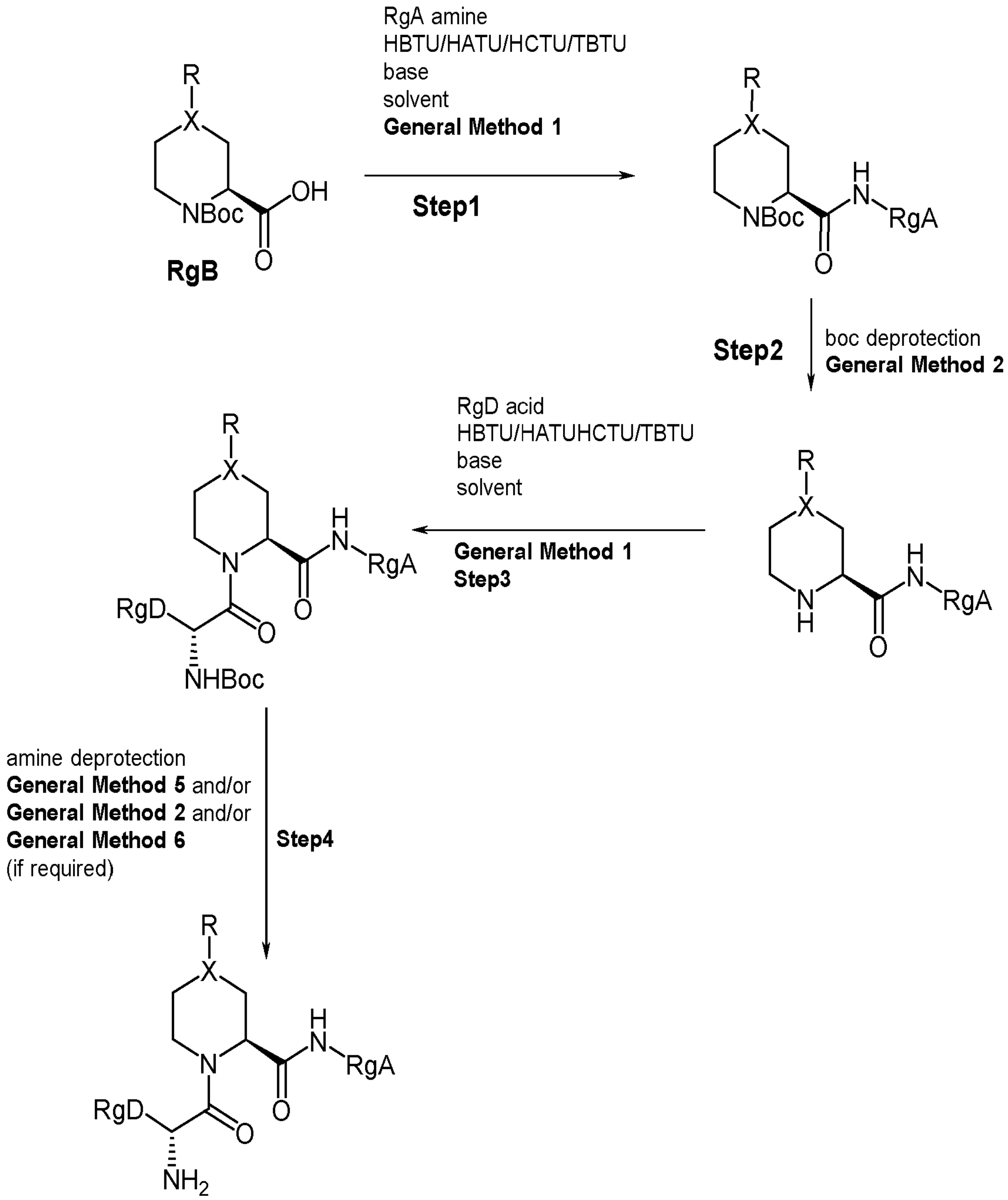


X = N or C

Compounds synthesised following the route of ***general scheme 1:***

: (S)-4-((R)-2-Amino-4-phenyl-butyryl)-3-[(1-methyl-1H-benzotriazol-5-ylmethyl)-carbamoyl]-piperazine-1-carboxylic acid benzyl ester M05098

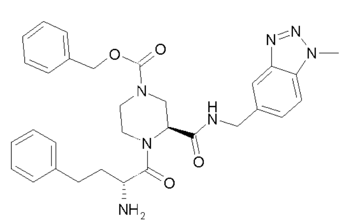

**Step 1:** (S)-N-1-Boc-4-Cbz-2-Piperazine carboxylic acid (150 mg, 0.41 mmol), (1-methyl-1H-benzo[d][1,2,3]triazol-5-yl)methanamine hydrochloride (82 mg, 0.41 mmol) and HATU (234 mg, 0.61 mmol) were dissolved in DMF (2 mL). $Et_3N$ (144 μL, 1.03 mmol) was added and the reaction mixture stirred at room temperature overnight. The sample was

diluted with DMSO, filtered and purified by prep HPLC. The product-containing fractions were concentrated *in vacuo* to give (S)-2-[(1-methyl-1H-benzotriazol-5-ylmethyl)-carbamoyl]-piperazine-1,4-dicarboxylic acid 4-benzyl ester 1-tert-butyl ester as a clear oil (111 mg, 53%).

AnalpH2_MeOH_4MIN: Rt: 3.00 min, m/z 509.3 $[M+H]^+$

**Step 2:** (S)-2-[(1-Methyl-1H-benzotriazol-5-ylmethyl)-carbamoyl]-piperazine-1,4-dicarboxylic acid 4-benzyl ester 1-tert-butyl ester (111 mg, 0.22 mmol) was dissolved in DCM (2 mL) and TFA (2 mL) added. The reaction mixture was stirred at room temperature for 1 hour, then concentrated *in vacuo.* The crude material was purified by SCX-2 cartridge (10g), eluting with 0.5 M $NH_3$/MeOH and the product-containing fractions concentrated *in vacuo* to give (S)-3-[(1-methyl-1H-benzotriazol-5-ylmethyl)-carbamoyl]-piperazine-1-carboxylic acid benzyl ester (75 mg, 84%) as a clear oil.

AnalpH2_MeOH_4MIN: Rt: 1.80 min, m/z 409.3 $[M+H]^+$

**Step 3:** (S)-3-[(1-Methyl-1H-benzotriazol-5-ylmethyl)-carbamoyl]-piperazine-1-carboxylic acid benzyl ester (75 mg, 0.18 mmol), Boc-D-homophenyl alanine (51 mg, 0.18 mmol) and HATU (464 mg, 1.22 mg) were dissolved in DMF. $Et_3N$ (171 μL, 1.22 mmol) was added and the reaction mixture stirred at room temperature overnight. The crude reaction was purified by prep HPLC and the product-containing fractions concentrated *in vacuo* to give (S)-4-((R)-2-tert-butoxycarbonylamino-4-phenyl-butyryl)-3-[(1-methyl-1H-benzotriazol-5-ylmethyl)-carbamoyl]-piperazine-1-carboxylic acid benzyl ester (48.3 mg, 40 %) as a brown oil.

AnalpH2_MeOH_4MIN: Rt: 3.36 min, m/z 670.47 $[M+H]^+$

**Step 4:** (S)-4-((R)-2-*tert*-Butoxycarbonylamino-4-phenyl-butyryl)-3-[(1-methyl-1H-benzotriazol-5-ylmethyl)-carbamoyl]-piperazine-1-carboxylic acid benzyl ester (48.3 mmol, 0.072 mmol) was dissolved in DCM (2 mL) and TFA (2 mL) added. The reaction mixture was stirred at room temperature for 1 hour, then concentrated *in vacuo* and purified by prep HPLC to give (S)-4-((R)-2-amino-4-phenyl-butyryl)-3-[(1-methyl-1H-benzotriazol-5-ylmethyl)-carbamoyl]-piperazine-1-carboxylic acid benzyl ester (21.3 mg, 49%) as a white solid.

ANALPH9_MEOH_QC_v1, Rt: 7.45 min, m/z 570.3 [M+H]+

ANALPH2_MEOH_QC_v1, Rt: 5.59 min, m/z 570.3 [M+H]+

$^1H$ NMR (400 MHz, $CDCl_3$) δ 7.87 (m, 1H), 7.46 - 7.27 (m, 8H), 7.28 - 7.09 (m, 5H), 5.31 - 4.93 (m, 4H), 4.62 (d, *J* = 71.4 Hz, 3H), 4.24 (s, 2.7H), 4.12 (s, 0.3H), 4.00 - 3.69 (m, 1H), 3.27 (s, 2H), 3.20 - 3.04 (m, 1H), 3.03 - 2.84 (m, 1H), 2.86 - 2.56 (m, 2H), 2.05 - 1.82 (m, 2H).

The following compounds were made by analogous methods:

| Example No. | Structure & Conditions | Analytical Data | Mass, % yield, state |
|---|---|---|---|
| M00832 (not of the invention) | 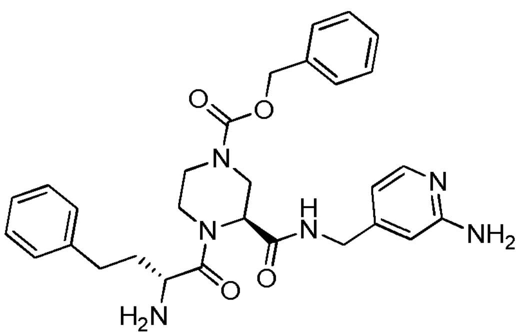<br>RgA: 4-(aminomethyl)pyridin-2-amine dihydrochloride<br>RgB: (S)-N-1-Boc-N-4-Cbz-2-Piperazine carboxylic acid<br>RgD: N-Boc-D-homophenylalanine<br>Step 1 & 3: **GM8** purification a<br>Step 2 & 4: **GM2B** | Agilent_MeCN_HPLC_3 min **LCMS:** $R_t$ = 1.52 min m/z = 530.7 $[M+H]^+$ | 23 mg, fawn solid |

(continued)

| Example No. | Structure & Conditions | Analytical Data | Mass, % yield, state |
|---|---|---|---|
| M00833 (not of the invention) | 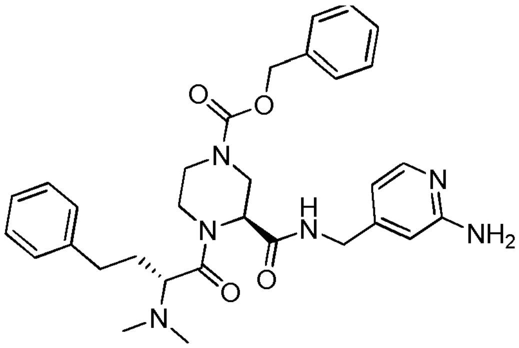 RgA: 4-(aminomethyl)pyridin-2-amine dihydrochloride<br>RgB: (S)-N-1-Boc-N-4-Cbz-2-Piperazine carboxylic acid<br>RgD: (2R)-2-(dimethylamino)-4-phenylbutanoic acid<br>Step 1 **GM8** purification a<br>Step 2: **GM2B**<br>Step 3: **GM8** purification b | Agilent_MeCN_HPLC_3 min<br>**LCMS:** $R_t$ = 1.59 min m/z = 559.6 $[M+H]^+$ | 49 mg, cream powder |
| M00834 (not of the invention) | 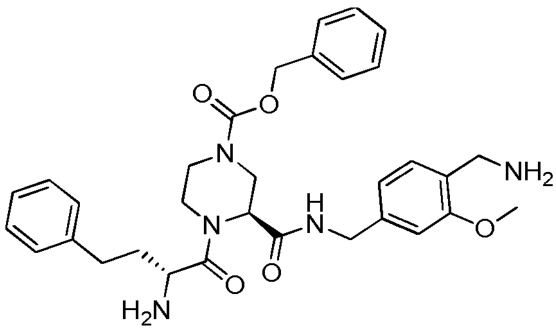 RgA: 4-(aminomethyl)-2-methoxybenzonitrile<br>RgB: (S)-N-1-Boc-N-4-Cbz-2-Piperazine carboxylic acid<br>RgD: N-Boc-D-homophenylalanine<br>Step 1 & 3: **GM1** HCTU & $Et_3N$ in DMF<br>Step 2: **GM2B**<br>Step 4: **GM6 & GM2B** with purification F | Agilent_MeCN_HPLC_3 min<br>**LCMS:** $R_t$ = 1.58 min m/z = 574.5 $[M+H]^+$ | 62 mg, white solid |

(continued)

| Example No. | Structure & Conditions | Analytical Data | Mass, % yield, state |
|---|---|---|---|
| M00951 | 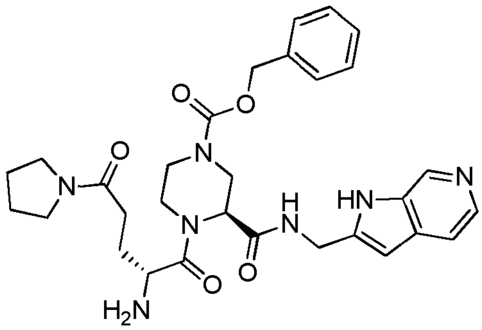<br>RgA: {1H-pyrrolo[2,3-c]pyridine-2-yl}methanamine<br>RgB: (S)-N-1-Boc-N-4-Cbz-2-Piperazine carboxylic acid<br>RgD: (R)-2-*tert*-Butoxycarbonylamino-5-oxo-5-pyrrolidin-1-yl-pentanoic acid<br>Step 1 & 3: **GM1** with HCTU and $Et_3N$ in DMF<br>Step 2: & 4 **GM2B** | Thermo_MeOH_UHPLC _1.2 min<br>**LCMS:** Rt= 0.5 min m/z = 576.6 $[M+H]^+$ | 17 mg, colourless gum |
| M00950 | 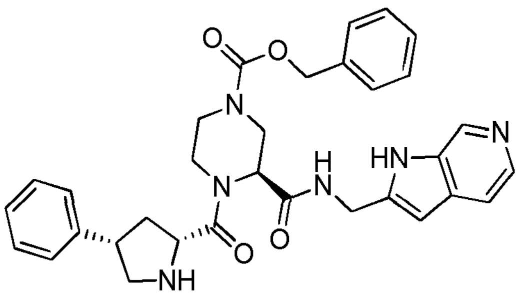<br>RgA: {1H-pyrrolo[2,3-c]pyridine-2-yl}methanamine<br>RgB: (S)-N-1-Boc-N-4-Cbz-2-Piperazine carboxylic acid<br>RgD: (2R,4S)-Boc-4-phenyl-pyrrolidine-2-carboxylic acid<br>Step 1 & 3: **GM1** with HCTU and $Et_3N$ in DMF<br>Step 2: & 4 **GM2B** | Thermo_MeOH_UHPLC _1.2 min<br>**LCMS:** $R_t$ = 0.5 min m/z = 567.60 $[M+H]^+$ | 8 mg, colourless gum |

(continued)

| Example No. | Structure & Conditions | Analytical Data | Mass, % yield, state |
|---|---|---|---|
| M00971 | 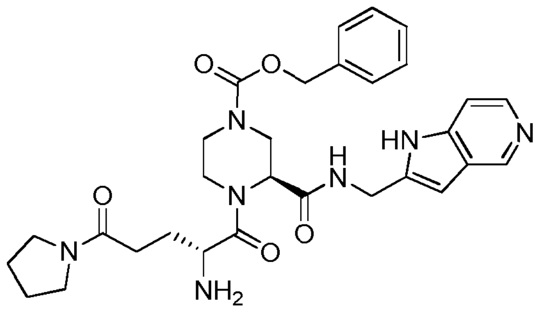<br>RgA: (1H-pyrrolo[3,2-c]pyridin-2-yl)methanamine<br>RgB: (S)-N-1-Boc-N-4-Cbz-2-Piperazine carboxylic acid<br>RgD: (R)-2-*tert*-Butoxycarbonylamino-5-oxo-5-pyrrolidin-1-yl-pentanoic acid<br>Step 1 & 3: **GM1** with HCTU and $Et_3N$ in DMF<br>Step 2: **GM2B**<br>Step 4: **GM2B** with purification g | Thermo_MeOH_UHPLC _1.2 min<br>**LCMS:** $R_t$ = 0.5 min m/z = 576.49 $[M+H]^+$ | 17 mg, pale yellow oil |
| M05086 (not of the invention) | 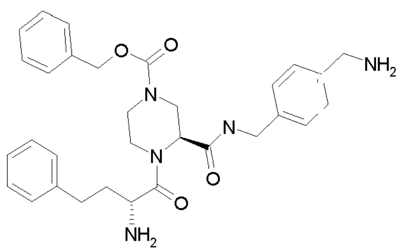<br>RgA: (4-Aminomethyl-benzyl)-carbamic acid 9H-fluoren-9-ylmethyl ester<br>RgB: (S)-N-1-Boc-4-Cbz-2-piperazine carboxylic acid<br>RgD: Boc-D-homophenylalanine<br>Step 1 & 3: **GM1** with HBTU & DIPEA in DMF **purification a;** Step 2: **GM2A;** Step 4: **GM5** followed by **GM2A** purification **f** | ANALPH9_MEOH_QC_ v1, Rt: 7.45 min, m/z 544.4 [M+H]+ ANALPH2_MEOH_QC_ v1, Rt: 4.31 min, m/z 544.4 [M+H]+ | 23.8 mg, white solid |
| M05124 | 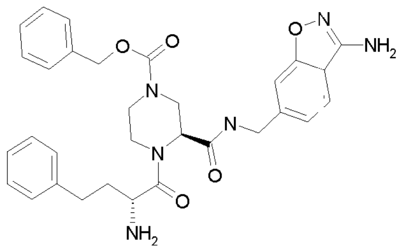<br>RgA: 6-Aminomethyl-benzo[d]isoxazol-3-ylamine<br>RgB: (S)-N-1-Boc-4-Cbz-2-piperazine carboxylic acid<br>RgD: Boc-D-homophenylalanine<br>Step 1 & 3: **GM1** with HATU & $Et_3N$ in DMF **purification a;** Step 2 & 4: **GM2A** final purification **f** | ANALPH9_MEOH_QC_ v1, Rt: 7.34 min, m/z 571.38 [M+H] + ANALPH2_MEOH_QC_ v1, Rt: 5.54 min, m/z 571.32 [M+H] + | 30 mg, white solid |

(continued)

| Example No. | Structure & Conditions | Analytical Data | Mass, % yield, state |
|---|---|---|---|
| M05209 (not of the invention) | 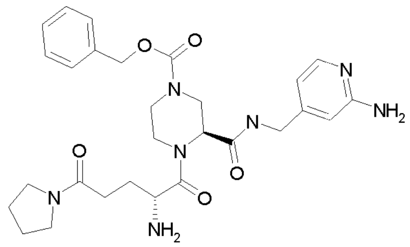 RgA: 4-aminomethylpyridin-2-ylamine RgB: (S)-N-1-Boc-4-Cbz-2-piperazine carboxylic acid RgD:(R)-2-*tert*-Butoxycarbonylamino-5-oxo-5-pyrrolidin-1-yl-pentanoic acid Step 1 & 3: **GM1** with HBTU & DIPEA in DCM **purification c;** Step 2 & 4: **GM2A;** final purification **f** | ANALPH2_MEOH_QC_ v1, Rt: 3.55 min, m/z 552.2 [M+H]+ ANALPH9_MEOH_QC_ v1, Rt: 6.67 min, m/z 552.4 [M+H]+ | 19 mg, white solid |
| M05212 | 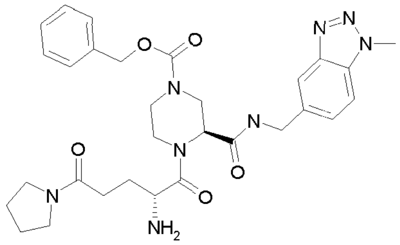 RgA: C-(1-Methyl-1H-benzotriazol-5-yl)-methylamine RgB: (S)-N-1-Boc-4-Cbz-2-piperazine carboxylic acid RgD: (R)-2-*tert*-Butoxycarbonylamino-5-oxo-5-pyrrolidin-1-yl-pentanoic acid Step 1 & 3: **GM1** with HBTU & DIPEA in DCM **purification c;** Step 2 & 4: **GM2A;** final purification **f** | ANALPH9_MEOH_QC_ v1, Rt: 6.81 min, m/z 591.4 [M+H]+ ANALPH2_MEOH_QC_ v1, Rt: 5.02 min, m/z 591.4 [M+H]+ | 30 mg, white solid |
| M05213 | 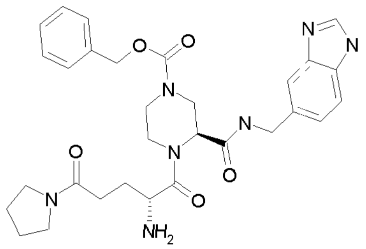 RgA: (1 H)-Benzimidazole-5-ylmethylamine hydrochloride RgB: (S)-N-1-Boc-4-Cbz-2-piperazine carboxylic acid RgD: (R)-2-*tert*-Butoxycarbonylamino-5-oxo-5-pyrrolidin-1-yl-pentanoic acid Step 1 & 3: **GM1** with HBTU & DIPEA in DCM **purification** c; Step 2 & 4: **GM2A;** final purification **f** | ANALPH2_MEOH_QC_ v1, Rt: 3.93 min, m/z 576.3 [M+H]+ ANALPH9_MEOH_QC_ v1, Rt: 6.77 min, m/z 576.4 [M+H]+ | 47 mg, white solid |

(continued)

| Example No. | Structure & Conditions | Analytical Data | Mass, % yield, state |
|---|---|---|---|
| M05217 | 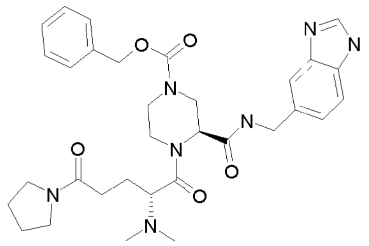 RgA: (1H)-Benzimidazole-5-ylmethylamine hydrochloride RgB: (S)-N-1-Boc-4-Cbz-2-piperazine carboxylic acid RgD: (R)-2-Dimethylamino-5-oxo-5-pyrrolidin-1-yl-pentanoic acid Step 1 & 3: **GM1** with HBTU & DIPEA in DMF **purification c;** Step 2: **GM2A;** Step 4 omitted | ANALPH2_MEOH_QC_ v1, Rt: 3.79 min, m/z 604.3 [M+H]+ ANALPH9_MEOH_QC_ v1, Rt: 7.09 min, m/z 604.5 [M+H]+ | 17 mg, white solid |
| M05276 | 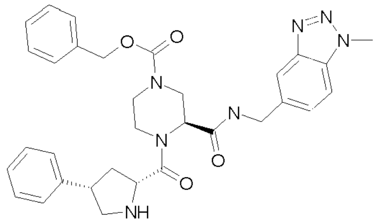 RgA: C-(1-Methyl-1H-benzotriazol-5-yl)-methylamine RgB: (S)-N-1-Boc-4-Cbz-2-piperazine carboxylic acid RgD: (2R, 4S)-Boc-4-phenylpyrrolidine-2-carboxylic acid Step 1 & 3: **GM1** with HATU & DIPEA in DMF; Step 2 & 4: **GM2A;** final purification **a** | ANALPH9_MEOH_QC_ v1, Rt: 7.37 min, m/z 582.3 [M+H]+ ANALPH2_MEOH_QC_ v1, Rt: 5.44 min, m/z 582.3 [M+H]+ | 21 mg, white solid |
| M05252 | 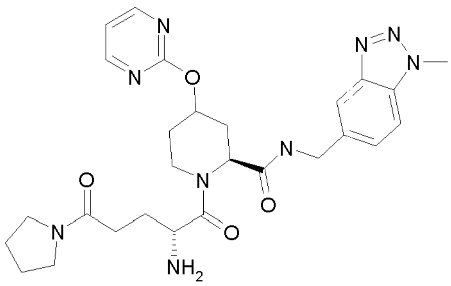 RgA: C-(1-Methyl-1H-benzotriazol-5-yl)-methylamine RgB: (S)-4-(Pyrimidin-2-yloxy)-piperidine-1,2-dicarboxylic acid 1-tert-butyl ester RgD: (R)-2-*tert*-Butoxycarbonylamino-5-oxo-5-pyrrolidin-1-yl-pentanoic acid Step 1 & 3: **GM1** with HBTU & DIPEA in DCM; Step 2 & 4: **GM2B;** final purification **f** | ANALPH2_MEOH_QC_ v1, Rt: 4.51/4.62 min, m/z 550.4 [M+H]+ ANALPH9_MEOH_QC_ v1, Rt: 6.08 min, m/z 550.4 [M+H]+ | 13 mg, white solid |

(continued)

| Example No. | Structure & Conditions | Analytical Data | Mass, % yield, state |
|---|---|---|---|
| M05375 | 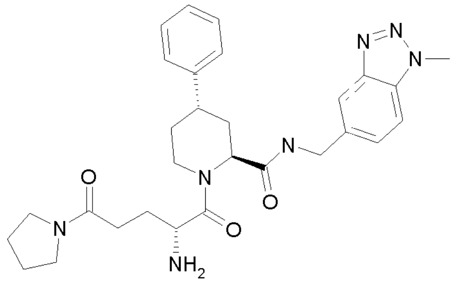 RgA: C-(1-Methyl-1H-benzotriazol-5-yl)-methylamine RgB: (2R,4S)-1-(*tert*-butoxycarbonyl)-4-phenylpiperidine-2-carboxylic acid RgD: (R)-2-*tert*-Butoxycarbonylamino-5-oxo-5-pyrrolidin-1-yl-pentanoic acid Step 1 & 3: **GM1** with HBTU & DIPEA in DCM; Step 2 & 4: **GM2B;** final purification **f** | ANALPH9_MEOH_QC_ v1, Rt: 7.23 min, m/z 532.5 [M+H]+ ANALPH2_MEOH_QC_ v1, Rt: 5.55 min, m/z 532.5 [M+H]+ | 65 mg, white solid |
| M05385 | 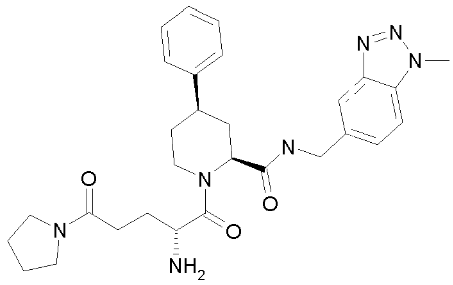 RgA: C-(1-Methyl-1 H-benzotriazol-5-yl)-methylamine RgB: (2S,4R)-1-(*tert*-butoxycarbonyl)-4-phenylpiperidine-2-carboxylic acid RgD: (R)-2-*tert*-Butoxycarbonylamino-5-oxo-5-pyrrolidin-1-yl-pentanoic acid Step 1 & 3: GM1 with HBTU & DIPEA in DCM; Step 2 & 4: GM2B; final purification f | ANALPH9_MEOH_QC_ v1, Rt: 7.23 min, m/z 532.5 [M+H]+ ANALPH2_MEOH_QC_ v1, Rt: 5.56 min, m/z 532.5 [M+H]+ | 33.5 mg, white solid |

***General Scheme 2***

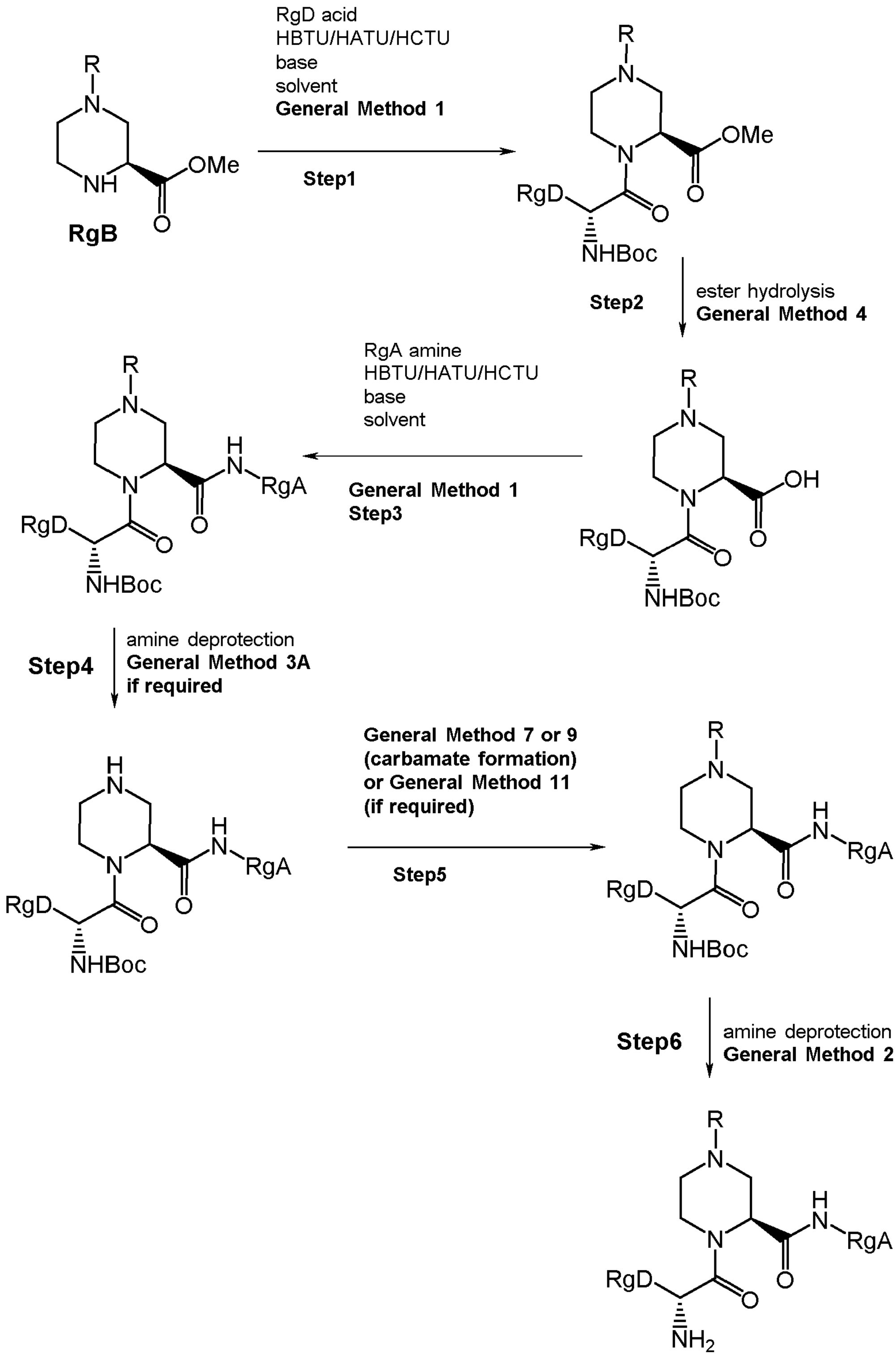


Compounds synthesised following the route of ***general scheme 2:***

| Example No. | Structure and conditions | Analytical Data | Mass, % yield, state |
|---|---|---|---|
| M00841 (not of the invention) | 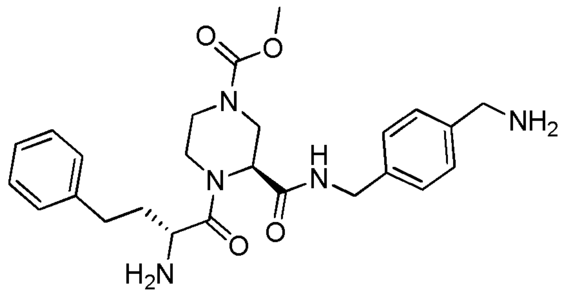<br>RgA: 1-(N-Boc-aminomethyl)-4-(aminomethyl)benzene<br>RgB: (S)-4-N-Cbz-Piperazine-2-carboxylic acid methyl ester and methyl chloroformate<br>RgD: N-Boc-D-homophenylalanine<br>Step 1 & 3: **GM1** HCTU, $Et_3N$, DMF, Step 2: **GM4C,** Step 4: **GM3A,** Step 5: **GM7,** Step 6: **GM2B** purification F | Agilent_MeCN_HPLC_3 min<br>**LCMS:** $R_t$ = 1.42 min m/z = 469.0 $[M+H]^+$ | 24 mg, colourless gum |
| M00858 (not of the invention) | 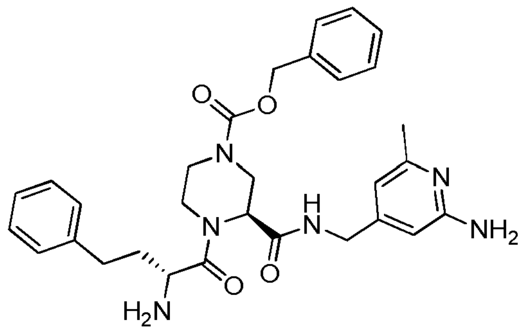<br>RgA: 4-(aminomethyl)-6-methylpyridin-2-amine<br>RgB: (S)-4-N-Cbz-Piperazine-2-carboxylic acid methyl ester<br>RgD: N-Boc-D-homophenylalanine<br>Step 1: **GM1** HCTU, $Et_3N$, DMF, Step 2: **GM4C,** Step 3: **GM1** TBTU, DIPEA, MeCN Step 4: **GM2B** | No data | 9 mg white solid |
| M00871 | 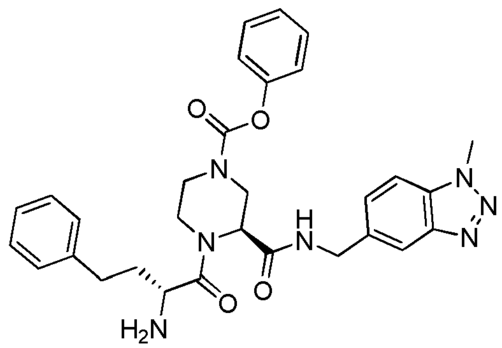<br>RgA: C-(1-Methyl-1 H-benzotriazol-5-yl)-methylamine<br>RgB: (S)-4-N-Cbz-Piperazine-2-carboxylic acid methyl ester and phenyl chloroformate RgD: N-Boc-D-homophenylalanine<br>Step 1 & 3: **GM1** HCTU & $Et_3N$ in DMF, Step 2: **GM4A,** Step 4: **GM3A,** Step 5: **GM7,** Step 6: **GM2B** | Agilent_MeCN_HPLC_3 min<br>**LCMS:** $R_t$ = 1.64 min m/z = 556.5 $[M+H]^+$ | 24 mg, yellow solid |

(continued)

| Example No. | Structure and conditions | Analytical Data | Mass, % yield, state |
|---|---|---|---|
| M00932 | 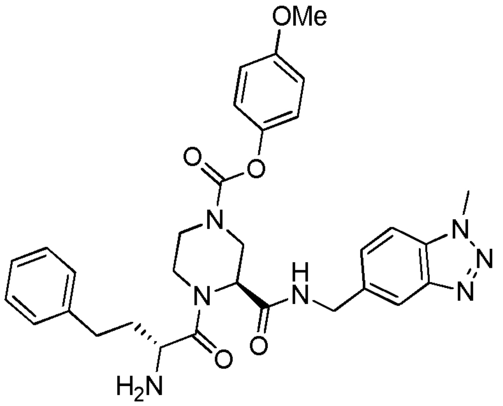<br>RgA: C-(1-Methyl-1 H-benzotriazol-5-yl)-methylamine<br>RgB: (S)-4-N-Cbz-Piperazine-2-carboxylic acid methyl ester and 4-methoxyphenylchloroformate<br>RgD: N-Boc-D-homophenylalanine<br>Step 1 & 3: **GM1** HCTU & $Et_3N$ in DMF, Step 2: **GM4A,** Step 4: **GM3A,** Step 5: **GM7,** Step 6: **GM2B** | Thermo_MeOH_UHPLC _1.2 min<br>**LCMS:** $R_t$ = 28.8 sec m/z = 586.22 $[M+H]^+$ | 30 mg, pale yellow solid |
| M00933 | 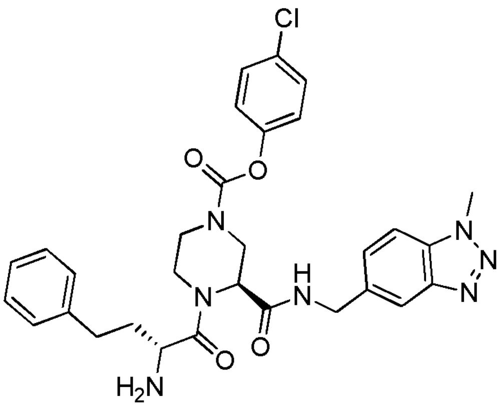<br>RgA: C-(1-Methyl-1 H-benzotriazol-5-yl)-methylamine<br>RgB: (S)-4-N-Cbz-Piperazine-2-carboxylic acid methyl ester and 4-chlorophenylchloroformate<br>RgD: N-Boc-D-homphenylalanine<br>Step 1 & 3: **GM1** HCTU & $Et_3N$ in DMF, Step 2: **GM4A,** Step 4: **GM3A,** Step 5: **GM7,** Step 6: **GM2B** | Thermo_MeOH_UHPLC _1.2 min<br>**LCMS:** $R_t$ = 30.4 sec m/z = 590.15 $[M+H]^+$ | 48 mg, pale yellow solid |
| M00934 | 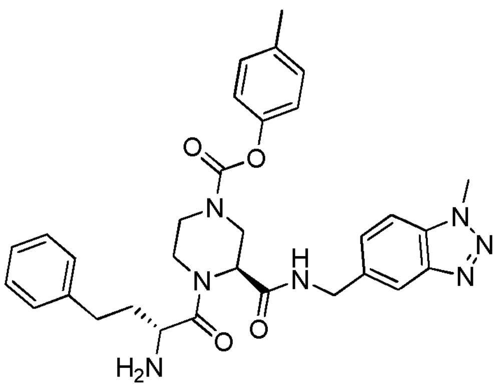<br>RgA: C-(1-Methyl-1 H-benzotriazol-5-yl)-methylamine | Thermo_MeOH_UHPLC _1.2 min<br>**LCMS:** $R_t$ = 30.3 sec m/z = 570.30 $[M+H]^+$ | 15 mg, pale yellow solid |

(continued)

| Example No. | Structure and conditions | Analytical Data | Mass, % yield, state |
|---|---|---|---|
| | RgB: (S)-4-N-Cbz-Piperazine-2-carboxylic acid methyl ester and 4-tolylchloroformate RgD: N-Boc-D-homophenylalanine<br>Step 1 & 3: **GM1** HCTU & $Et_3N$ in DMF, Step 2: **GM4A,** Step 4: **GM3A,** Step 5: **GM7,** Step 6: **GM2B** | | |
| M00890 (not of the invention) | 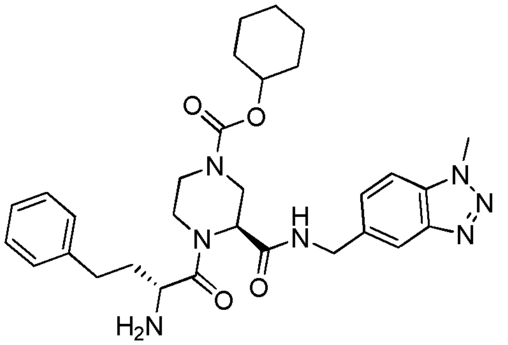<br>RgA: C-(1-Methyl-1H-benzotriazol-5-yl)-methylamine<br>RgB: (S)-N-1-Boc-N-4-Cbz-2-Piperazine carboxylic acid and cyclohexanol<br>RgD: N-Boc-D-homophenylalanine<br>Step 1 & 3: GM1 TBTU & DIPEA in MeCN<br>Step 2 & 6: GM2B<br>Step 4: GM3A<br>Step 5: GM9<br>Step 6: GM2B | Thermo_MeOH_UHPLC _1.2 min<br>LCMS: Rt = 31 sec m/z = 563.36 [M+H]+ | 0.9 mg |
| M00940 | 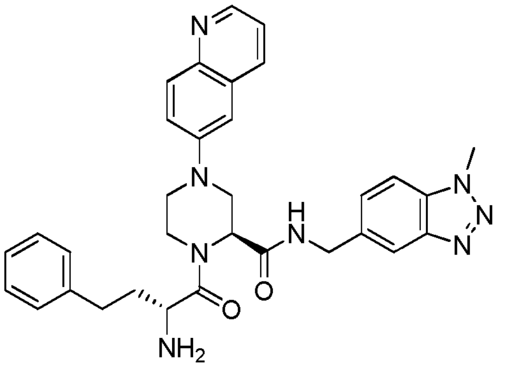<br>(2S)-1-[(2R)-2-amino-4-phenylbutanoyl]-N-[(1-methyl-1H-1,2,3-benzotriazol-5-yl)methyl]-4-(quinolin-6-yl) piperazine-2-carboxamide<br>RgA: C-(1-Methyl-1H-benzotriazol-5-yl)-methylamine<br>RgB: (S)-4-N-Cbz-Piperazine-2-carboxylic acid methyl ester and (quinolin-5-yl)boronic acid<br>RgD: N-Boc-D-homophenylalanine<br>Step 1 & 3: **GM1** TBTU & DIPEA in MeCN<br>Step 2: **GM2B**<br>Step 4: **GM3A**<br>Step 5: **GM11** with (quinolin-5-yl)boronic acid<br>Step 6: **GM2B** | Thermo_MeOH_UHPLC _1.2 min<br><br>**LCMS:** Rt= 0.5 min m/z = 563.66 $[M+H]^+$ | 13 mg brown solid |

Scheme: Late stage deprotection of piperazine amine

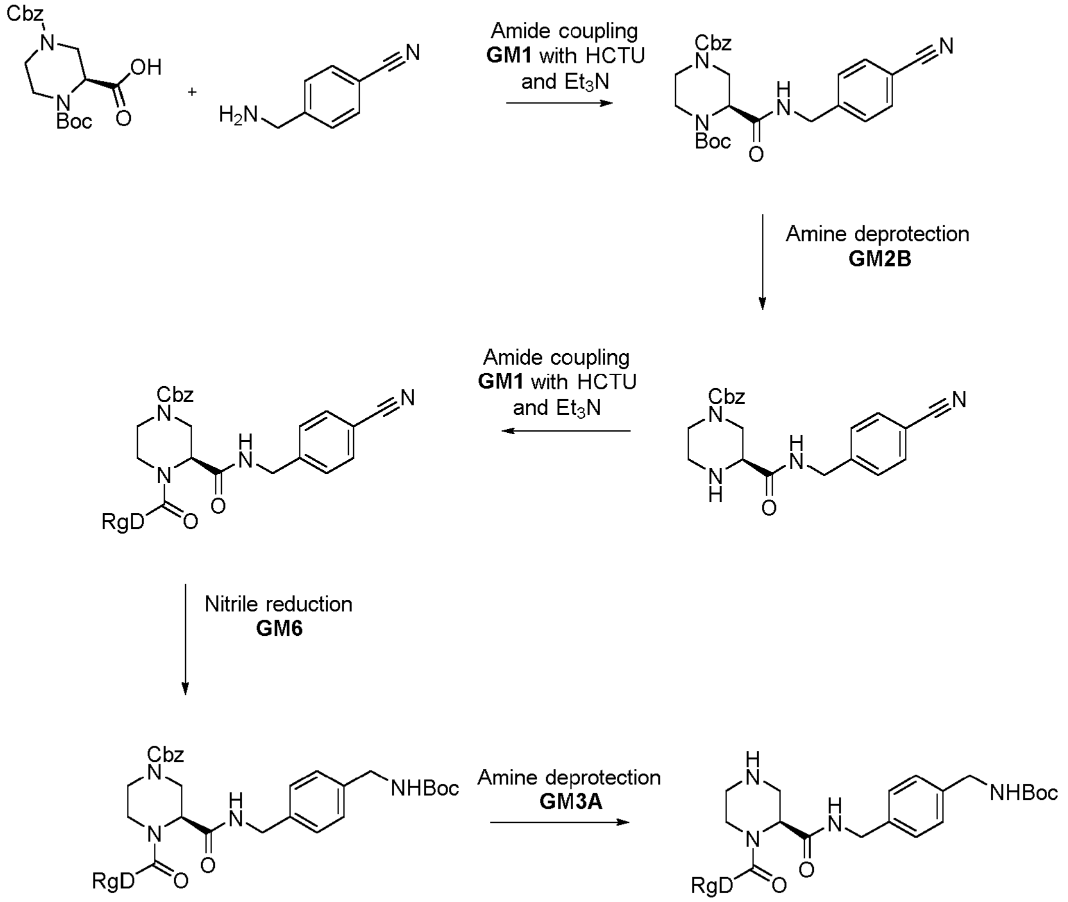


Scheme: Late stage sulfonamide formation

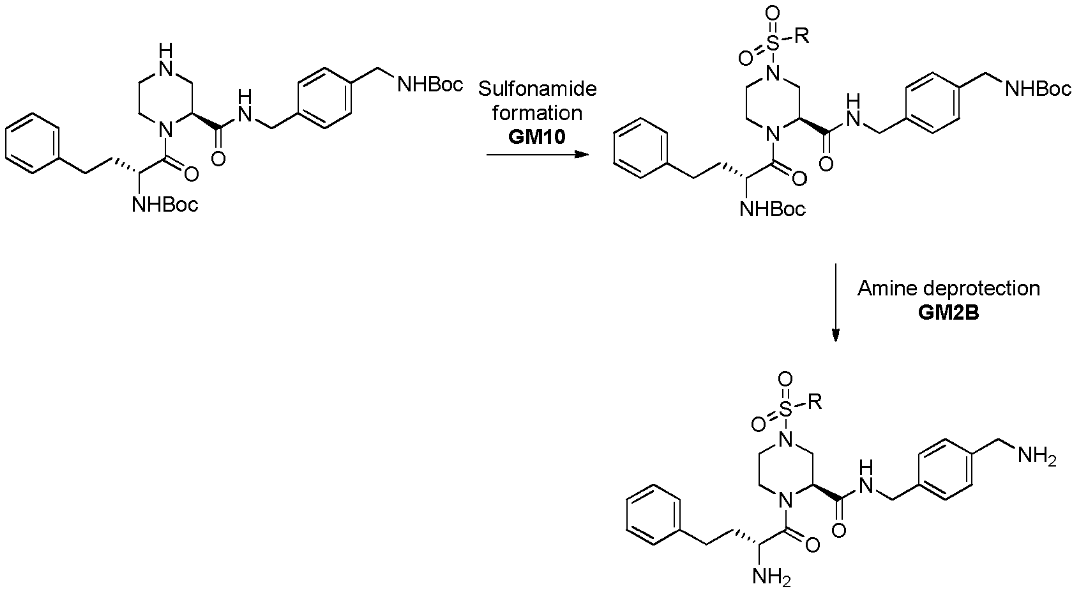


The following compound was synthesised using the above general method

| | | | |
|---|---|---|---|
| M00838 (not of the in- ventio n) | 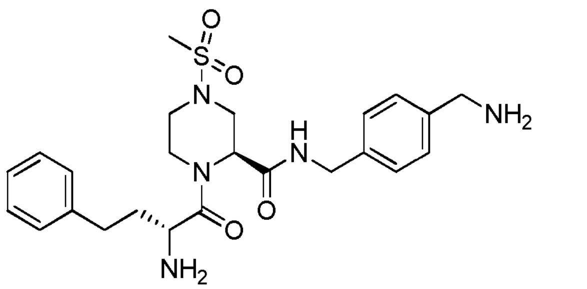<br>(2S)-1-[(2R)-2-amino-4-phenylbutanoyl]-N-{[4-(aminomethyl) phenyl]methyl}-4-methanesulfonylpiperazine-2-carboxamide [synthesised via Scheme: Late stage deprotection of piperazine amine using N-Boc-D-homophenylalanine as RgD] ] Step 1: **GM10** with tert-butyl N-[(2R)-1-[(2S)-2-({[4-({[(tertbutoxy)carbonyl]amino} methyl)phenyl]methyl}carbamoyl)piperazin-1-yl]-1-oxo-4-phenylbutan-2-yl]carbamate and methanesulfonyl chloride Step 2: **GM2B** | Agilent_MeCN_HPLC_3mi n<br>**LCMS:** $R_t$ = 1.29 min m/z = 510.6 $[M+Na]^+$ | 26 mg, gummy light brown solid |

Other compounds contemplated by the present invention are shown in the table below.

| | | | |
|---|---|---|---|
| M00556 (not of the inventio n) | 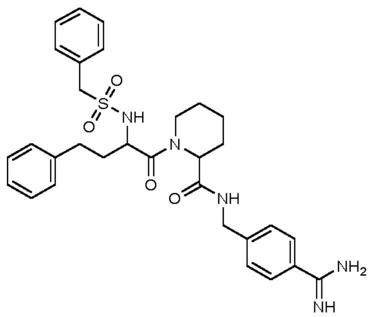 | M00882 | 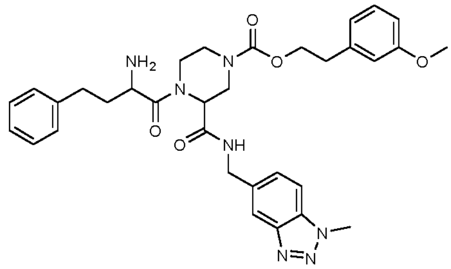 |
| M00836 (not of the inventio n) | 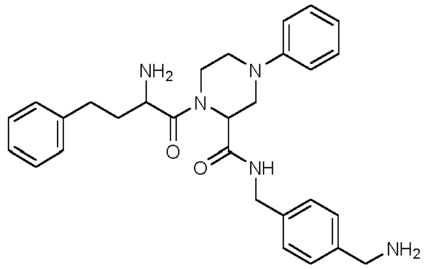 | M00883 | 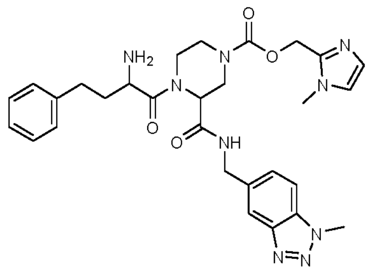 |
| M00838 (not of the inventio n) | 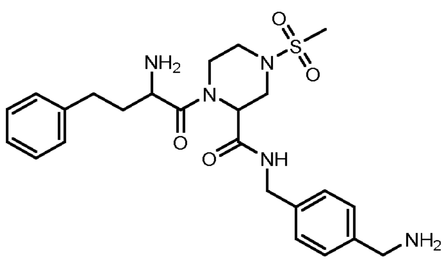 | M00891 | 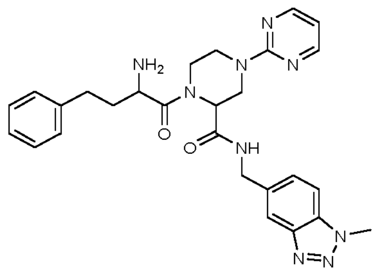 |

(continued)

| | | | |
|---|---|---|---|
| M00840 (not of the invention) | 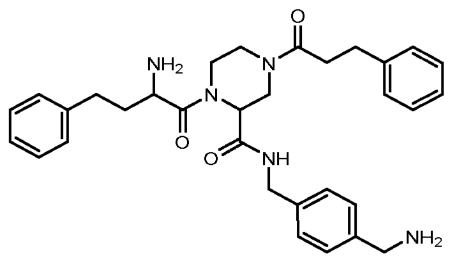 | M00940 | 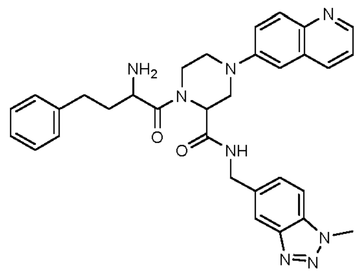 |
| M00842 (not of the invention) | 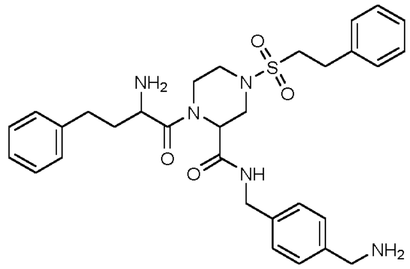 | M00942 | 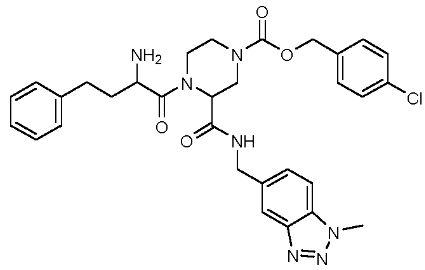 |
| M00843 (not of the invention) | 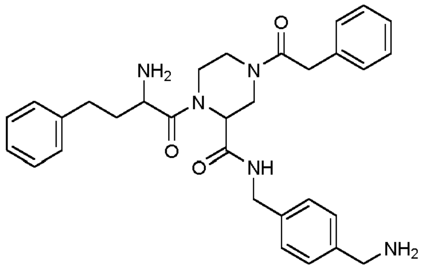 | M05062 (not of the invention) | 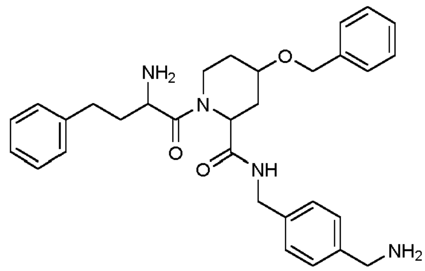 |
| M00844 (not of the invention) | 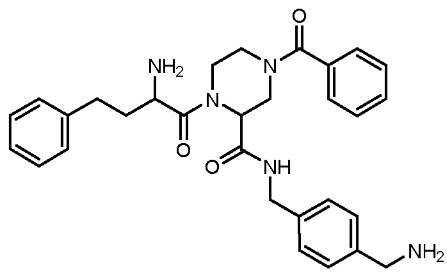 | M05071 (not of the invention) | 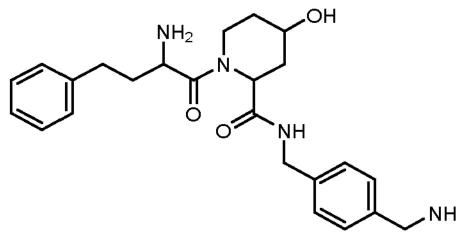 |
| M00847 (not of the invention) | 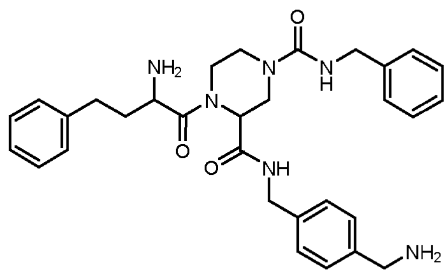 | M05260 (not of the invention) | 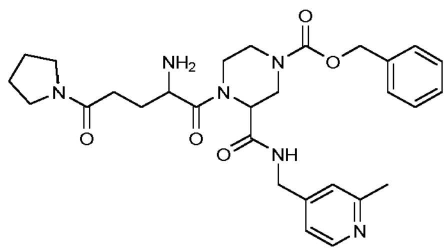 |
| M00848 (not of the invention) | 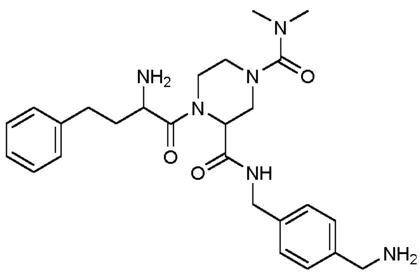 | M05263 (not of the invention) | 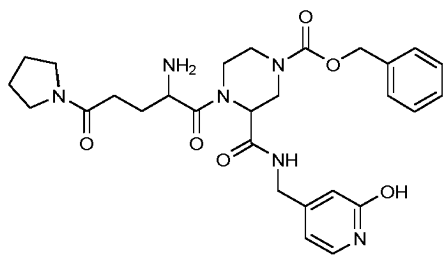 |

(continued)

| | | | |
|---|---|---|---|
| M00850 (not of the inventio n) | 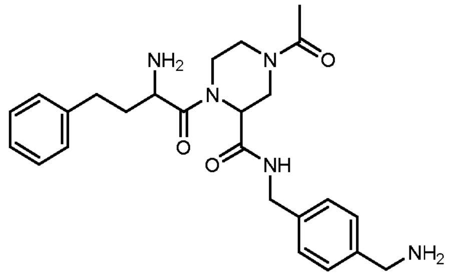 | M05273 | 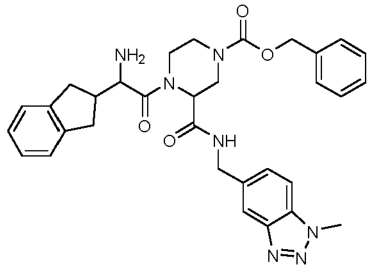 |
| M00859 (not of the inventio n) | 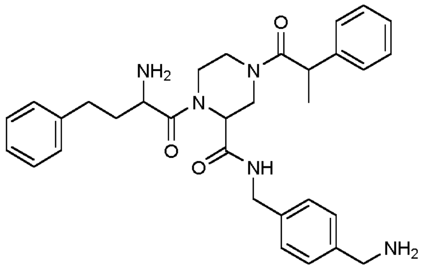 | M05275 | 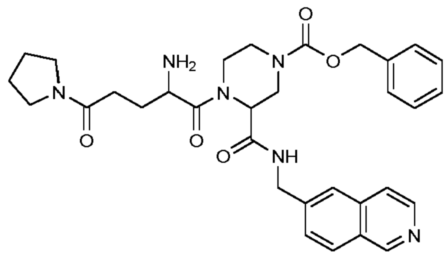 |
| M00860 (not of the inventio n) | 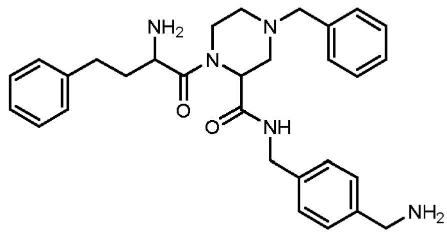 | M05277 | 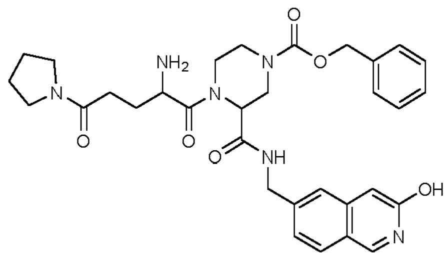 |
| M00862 ] (not of the inventio n) | 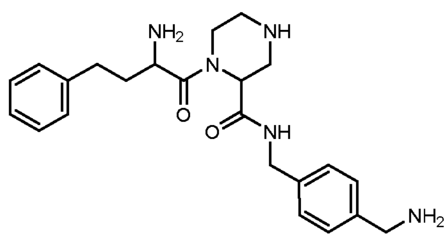 | M05286 | 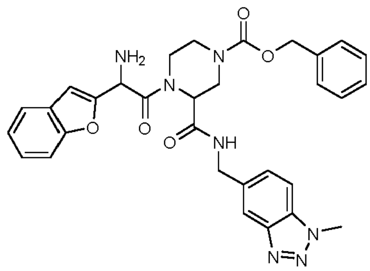 |
| M00880 (not of the inventio n) | 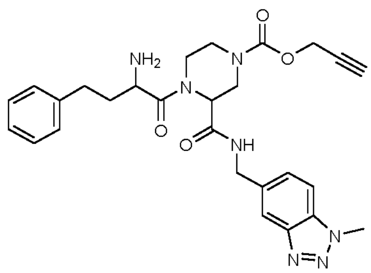 | M05292 | 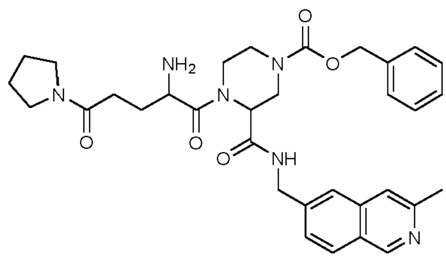 |

General Test Methods

The activities of the compounds of the invention have been determined *in vitro* using the following assays protocols for the screening of activity of FXIIa and other proteases. Each of these assays were performed in a purified system employing the use of chromogenic assays in microplate plate wells. Chromogenic peptide substrates mimicking natural protein substrates are attached via an amide bond to a chromogenic group. Paranitroaniline (pNA) is released from the peptide following catalyses by the proteolytic enzyme; the absorbance increases and can be monitored at 405 nm.

All compounds were dissolved in 100% (v/v) DMSO to a stock concentration of 10mM, the highest concentration of compound used in each assay is 500μM. The final concentrations of DMSO were 5% (v/v) in 50mM Tris 137mM NaCl pH

7.4. Where no test compound was added a final concentration of 5% DMSO was employed.

Determination of Factor XIIa Inhibition

Factor XIIa activity was measured using a chromogenic substrate S-2302 (Chromogenix). Various concentrations of compound were incubated with 10nM of FXIIa and incubated at 37°C for 10 minutes in 50mM Tris, 137mM NaCl, pH 7.4, prior to the addition of a final concentration of 450μM S-2302 chromogenic substrate. Kinetic readings at 405nm were monitored every 12 secs for a total duration of 3 hours at 37°C. Gradients of initial rates were determined and employed to calculate $IC_{50}$ values. Values of $IC_{50}$ were converted to Ki values based on the formula:

$$K_i=IC_{50}/(1+[Substrate]/Km)$$

The $K_i$ data obtained in the above manner is shown in Table 1 below. The activity of the compounds of the invention has been categorised based on the $K_i$ values, the categories being "*", "**" and "***". The category "*" refers to compounds with a $K_i$ value of greater than 2 μM. The category "**" refers to compounds with a $K_i$ value of 0.2 μM to 2 μM. The category "***" refers to compounds with a $K_i$ value of less than 0.2 μM. A "-" indicates that no test was conducted. The category "NA" indicates a compound that did not show any activity within the tested concentrations and within the limits of the assay.

Determination of Selectivity

To determine selectivity of test compounds, these test compounds were assayed for inhibitory activity against other serine proteases including FXa and thrombin. Essentially compounds at increasing concentrations were incubated with each enzyme: FXa (5nM) and thrombin (5nM), for 10 mins at 37°C followed the appropriate chromogenic substrate, S2765 (350μM), and GPR (250μM) respectively in 50mM Tris, 137mM NaCl, pH 7.4. Chromogenic substrates S2765 was from Chromogenix, and GPR from Bachem. Kinetic readings at 405nm were monitored every 12 secs for a total duration of 3 hours at 37 °C. Gradients of initial rates were determined and employed to calculate $IC_{50}$ values. Values of $IC_{50}$ were converted to Ki values based on the formula:

$$K_i=IC_{50}/(1+[Substrate]/Km)$$

Where [Substrate] denotes the concentration of substrate used in the assay and Km is the determined value of each enzyme with its own substrate. Compounds of this chemical series demonstrate competitive inhibition.

The fold selectivity for thrombin and FXa are also shown in Table 1 below. The fold selectivity demonstrates a preferential inhibition of FXIIa over FXa and thrombin. The fold selectiviety for FXIIa over thrombin for the compounds of the invention has been categorised based on the fold selectivity values, the categories being "+", "++" and "+++". The category "+" refers to fold selectivity values less than 10. The category "++" refers to a fold selectivity value of 10 to 100. The category "+++" refers to fold selectivity values greater than 100. A "-" indicates that no test was conducted. The category "NA" indicates a compound that did not show any activity within the tested concentrations and within the limits of the assay.

The fold selectiviety for FXIIa over FXa for the compounds of the invention has been categorised based on the fold selectivity values, the categories being "o", "oo" and "ooo". The category "o" refers to fold selectivity values less than 10. The category "oo" refers to a fold selectivity value of 10 to 100. The category "ooo" refers to fold selectivity values greater than 100. A "-" indicates that no test was conducted. The category "NA" indicates a compound that did not show any activity within the tested concentrations and within the limits of the assay.

Determination of *in vivo* anticoagulant efficacy

Reagents

AlexaFluor488 conjugate fibrinogen was purchased from Invitrogen (Paisley, UK).

Animals

C57BL/6 male mice weighing between 20 and 30 g were used for all experiments. All procedures were approved by the University of Sheffield ethics committee and performed in accordance with the Home Office Animals (Scientific Procedures) Act 1985 of the United Kingdom.

Intravital microscopy for real time assessment of fibrin formation in vivo

Microscopic observation of thrombus formation following ferric chloride ($FeCl_3$)-induced injury in vivo were made using an upright microscope (Nikon eclipse E600-FN, Nikon UK, Kingston upon Thames, United Kingdom) equipped for bright field and fluorescence microscopy and with a water immersion objective (40/0.80 W).

Mice were anaesthetised with an i.p. injection of 125 mg/kg ketamine hydrochloride (Ketaset; Willows Francis Veterinary, Crawley, UK), 12.5 mg/kg xylazine hydrochloride (Bayer Suffolk, UK) and 0.025 mg/kg Atropine sulphate (phoenix Pharmaceuticals Ltd, UK). Cannulation of the trachea (to aid breathing) and carotid artery (for maintenance of anaesthesia and substance administration) were performed and the femoral vein was exposed. 100 μl of AlexaFluor488 conjugate fibrinogen (2mg/ml) and 100 μl of compound (diluted in 10% DMSO and 90% saline in the100 μl) or vehicle (10% DMSO in saline in 100 μl) were administered via the carotid artery 5 min prior to application of a 3mmx2mm filter paper saturated with 10%(v/v) $FeCl_3$ being placed directly on the femoral vein for 3 minutes.

Real-time, Alexa488nm (green channel) images using Slidebook imaging software (Version 5.0; Intelligent Imaging Innovations, 3i, Denver, USA) were taken to monitor thrombus formation in vivo at regular intervals for 1h. The area was flushed with warm PBS following $FeCl_3$ exposure and throughout the experiment.
Data analyses employed Slidebook to determine fibrin clot formation in real time.

Real time images of thrombus formation were analysed using Slidebook image analysis software by setting a background region outside the thrombus area and measuring Alexa680nm signal intensities above background over entire area of injury. Setting individual background intensities for the green channel in this way allows selection of pixels that only show signal above background for both probes at each time frame. The resulting selection of pixels or "masked" region (defined as region used for data analyses) is then determined for the pixel's signal intensity for FITC 488nm (encompassing intensity and area of signal). The Slidebook software allows for the calculation of background for each image file representing different time points in an automated manner, therefore allowing for background subtraction at each time point. Thrombus area is determined by quantifying pixel intensities above background (at each time point) in the FITC 488nm channel and expressing the masked pixels as total pixel area. When establishing the background region, all time frames within the background are run as a movie to ensure that the region selected as background does not develop any clot growth over the duration of experiment. Background signal prior to ferric chloride injury is determined and subtracted from readings post ferric chloride injury. This is important for analyses with Slidebook because the same region of background is employed for signal determination at each time frame. Data generated is reflective of area intensity of each pixel and as background subtraction takes place with the same image/time frame this data provides an accurate assessment of FITC area with intensity. Data is plotted as relative fluorescence units (RFU) over time.

The percentage inhibition of clot formation is calculated relative to mice administered vehicle only for the 60 minute time point. The resuts are shown in Figure 1.

Table 1

| Compound code | FXIIa alpha (Human) Ki | Thrombin (human) / FXIIa alpha (human) | FXa (human) / FXIIa alpha (human) |
|---|---|---|---|
| **M00556** | ** | + | o |
| **M00832** | * | ++ | oo |
| **M00833** | * | ++ | oo |
| **M00834** | *** | +++ | ooo |
| **M00836** | * | - | - |
| **M00838** | * | + | o |
| **M00840** | * | - | - |
| **M00841** | * | ++ | oo |
| **M00842** | * | - | - |
| **M00843** | * | +++ | oo |
| **M00844** | * | - | - |
| **M00847** | * | - | - |
| **M00848** | * | - | - |
| **M00850** | * | - | - |
| **M00858** | * | ++ | oo |
| **M00859** | * | - | - |
| **M00860** | NA | - | - |
| **M00862** | * | - | - |

(continued)

| Compound code | FXIIa alpha (Human) Ki | Thrombin (human) / FXIIa alpha (human) | FXa (human) / FXIIa alpha (human) |
|---|---|---|---|
| M00871 | * | ++ | oo |
| M00880 | * | - | - |
| M00882 | NA | - | - |
| M00883 | * | - | - |
| M00890 | * | ++ | oo |
| M00891 | * | - | - |
| M00932 | * | ++ | oo |
| M00933 | * | ++ | oo |
| M00934 | * | ++ | oo |
| M00940 | * | - | - |
| M00942 | * | - | - |
| M00950 | ** | ++ | oo |
| M00951 | ** | ++ | oo |
| M00971 | *** | +++ | ooo |
| M05062 | NA | - | - |
| M05071 | * | - | - |
| M05086 | * | ++ | oo |
| M05098 | * | ++ | oo |
| M05124 | * | + | oo |
| M05209 | ** | +++ | oo |
| M05212 | * | +++ | ooo |
| M05213 | * | ++ | oo |
| M05217 | * | ++ | oo |
| M05252 | * | ++ | oo |
| M05260 | NA | - | - |
| M05263 | * | - | - |
| M05273 | * | - | - |
| M05275 | * | - | - |
| M05276 | * | ++ | oo |
| M05277 | * | ++ | - |
| M05286 | * | - | - |
| M05292 | * | - | - |
| M05375 | * | ++ | oo |
| M05385 | * | ++ | oo |

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

## Claims

1. A compound according to formula (I) and pharmaceutically acceptable salts thereof:

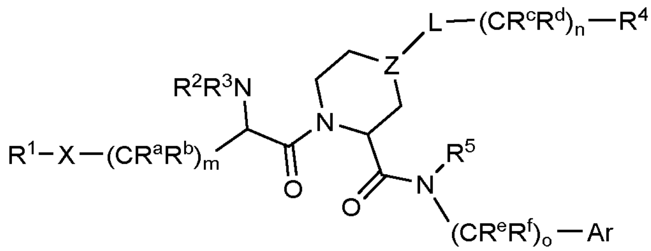

(I)

wherein

Z is either N or $CR^{4a}$;
X is either a bond, -C(O)NH-, -C(O)O- or -C(O)-;
L is selected from: bond, -O-, -C(O)O-, $-NR^6-$, $-C(O)NR^7-$, and $-SO_2-$;
Ar is a substituted or unsubstituted 9 to 10 membered bicyclic heteroaromatic ring system, wherein the bicyclic heteroaromatic ring system is an aromatic hydrocarbon ring system with at least one heteroatom within the ring system selected from O, N and S or a bicyclic ring system which is not completely aromatic but contains an aromatic ring and wherein the at least one heteroatom is present within the aromatic ring or the non-aromatic ring, wherein, when substituted, Ar is substituted with 1, 2, or 3 substituents selected from: halo, $C_{1-6}$ alkyl, $-OR^g$, $-NR^gR^h$ or $C_{1-4}$ alkyl substituted by $-NR^gR^h$;
m is selected from 0, 1, 2, or 3;
n is selected from 0, 1, 2, 3, or 4;
o is selected from 1 or 2;
$R^1$ is selected from substituted or unsubstituted: $-NR^8R^9$, 5 to 10 membered carbocyclic ring system or a 5 to 10 membered heterocyclic ring system;
wherein when substituted $R^1$ is substituted with 1, 2, or 3 groups selected from: =O, CN, -OH, or $-O-C_{1-6}$ alkyl, halo, $C_{1-6}$ alkyl and $C_{1-6}$ haloalkyl;
$R^2$ is selected from: H, $C_{1-6}$ alkyl, $C_{1-6}$ haloalkyl, phenyl, benzyl, $-C(O)R^{2a}$, and $-S(O_2)R^{2a}$;
wherein $R^{2a}$ is selected from: $C_{1-6}$ alkyl, phenyl, and benzyl;
$R^3$ is:

(a) H or $C_{1-6}$ alkyl; or
(b) $R^3$ together with one of $R^a$ or $R^b$ forms a bond, $-CH_2-$ or $-CH_2CH_2-$ group resulting in a 4, 5 or 6 membered heterocycloalkyl ring comprising the $-CH_2-$ or $-CH_2CH_2-$ group, the N atom to which $R^3$ is attached, the C atom to which $R^a$ or $R^b$ are attached, and any intervening atoms; or
(c) $R^3$ forms a bond, $-CH_2-$ or $-CH_2CH_2-$ group with an atom of $R^1$ when $R^1$ is a carbocyclic ring system or a heterocyclic ring system;

$R^4$ is selected from: H, $=CH_2$, -CN, halo, $C_{1-4}$ alkyl, $C_{1-4}$ haloalkyl, $-OR^{10}$, $-NR^{10}R^{11}$, 6 to 10 membered aryl, 5 to 10 membered heteroaryl, wherein the 6 to 10 membered aryl or 5 to 10 membered heteroaryl group is unsubstituted or substituted with 1, 2 or 3 $R^{12}$;
$R^{4a}$ is selected from: H, -OH, halo or $C_{1-4}$ alkyl;
$R^5$ is H or $C_{1-6}$ alkyl;
$R^6$ is H, $C_{1-6}$ alkyl or $-C(O)C_{1-6}$ alkyl;
$R^7$ is H or $C_{1-6}$ alkyl;
$R^8$ and $R^9$ are independently at each occurrence selected from: H, $C_{1-6}$ alkyl, $C_{3-6}$ cycloalkyl, phenyl, $C_{1-4}$ alkyl substituted with $-OR^i$, or $C_{1-4}$ alkyl substituted with phenyl, or $R^8$ and $R^9$ taken together with the atom to which they are attached form 3 to 8 membered heterocycloalkyl ring, which is unsubstituted or substituted with: CN, halo, $C_{1-6}$ alkyl or $-OR^i$;
$R^{12}$ is independently at each occurrence selected from: halo, $C_{1-4}$ alkyl, $C_{1-4}$ haloalkyl, $-OR^{13}$, -CN, $-C(O)R^{10}$, =O, $SO_2R^{10}$, benzyl, phenyl, unsubstituted 5 or 6 membered heteroaryl, or methyl substituted 5 or 6 membered heteroaryl;
$R^{10}$ and $R^{11}$ are independently at each occurrence selected from: H and $C_{1-4}$ alkyl;
$R^{13}$ is selected from: H, $C_{1-4}$ alkyl, $C_{1-4}$ haloalkyl, phenyl or benzyl;
$R^a$ and $R^b$ are independently at each occurrence selected from: H, $C_{1-4}$ alkyl, $-OR^j$ or one of $R^a$ or $R^b$ together with $R^3$ forms a bond, $-CH_2-$ or $-CH_2CH_2-$ group resulting in a 4, 5 or 6 membered heterocycloalkyl ring comprising the $-CH_2-$ or $-CH_2CH_2-$ group, the N atom to which $R^3$ is attached, the C atom to which $R^a$ or $R^b$ are attached, and any

intervening atoms; and
$R^c$, $R^d$, $R^e$, $R^f$, $R^g$, $R^h$, $R^i$ and $R^j$ are independently at each occurrence selected from: H and $C_{1-4}$ alkyl.

**2.** The compound of claim 1 wherein the compound is a compound of formula (Ia) and pharmaceutically acceptable salts thereof:

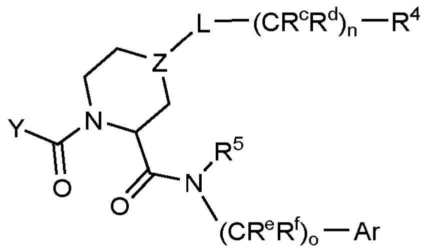

(Ia)

wherein

Y is selected from:

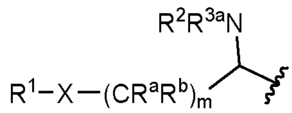 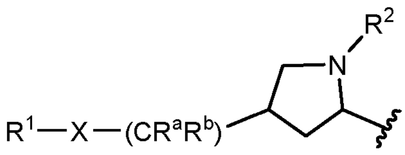 and 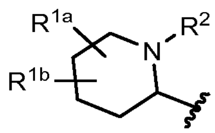

$R^{1a}$ and $R^{1b}$ taken together form a substituted or unsubstituted: 5 or 6 membered heteroaromatic ring or a phenyl ring;
wherein when the ring formed from $R^{1a}$ and $R^{1b}$ is substituted it is substituted with 1, 2, or 3 $R^z$ groups wherein $R^z$ is independently selected at each occurrence from: =O, CN, -OH, or -O-$C_{1-6}$ alkyl, halo and $C_{1-6}$ alkyl;
$R^{3a}$ is H or $C_{1-6}$ alkyl; and
m is selected from 1, 2, or 3.

**3.** The compound of any preceding claim wherein L is selected from bond, -O-, or -C(O)O-.

**4.** The compound of any preceding claim, wherein $R^2$ is H and/or $R^3$ is H and/or $R^5$ is H.

**5.** The compound of any preceding claim wherein $R^1$ is selected from substituted or unsubstituted: phenyl or a 5 or 6 membered heterocycloalkyl ring system.

**6.** The compound of any preceding claim wherein Ar is unsubstituted or substituted with methyl, chloro, -OMe, $-NH_2$ or $-CH_2NH_2$.

**7.** The compound of claim 6 wherein Ar is selected from:

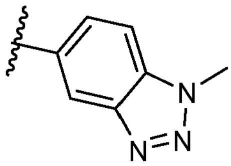 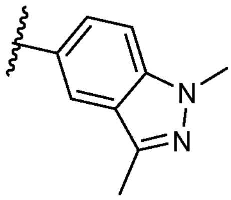 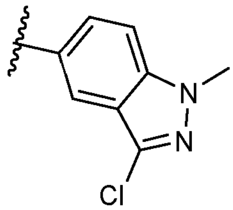 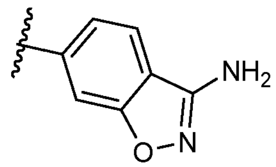

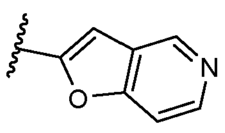 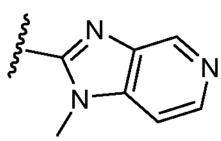 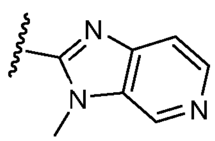 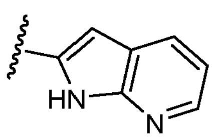

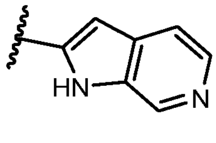 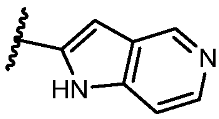 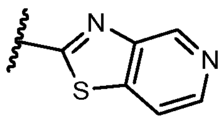 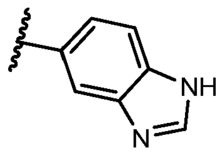 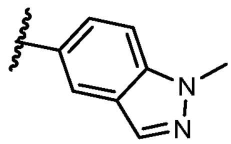

**8.** The compound of claim 6 wherein Ar is azaindole, benzotriazole or N-methyl benzotriazole.

**9.** The compound of any preceding claim wherein $R^6$ is H, Me or -C(O)Me.

**10.** The compound of any preceding claim wherein $-L-(CR^cR^d)_n-$ is selected from: a bond, $CH_2$, - NH-, $-NHCH_2-$, $-NH(CH_2)_2-$, $-NH(CH_2)_3-$, -N(Me)-, $-N(C(O)Me)CH_2-$, -NHC(O)-, $-NHC(O)CH_2-$, - $NHC(O)(CH_2)_2-$, or $NHC(O)(CH_2)_3-$.

**11.** The compound of any preceding claim wherein $R^4$ is selected from: $=CH_2$, -CN, halo, $C_{1-4}$ alkyl, $C_{1-4}$ haloalkyl, $-OR^{10}$, $-NR^{10}R^{11}$, phenyl or napthalenyl, pyridinyl, pyrazinyl, pyrazolyl, imidazolyl, dihydrobenzofuran, benzodioxolanyl or isoindolinyl; wherein any group that is cyclic is unsubstituted or substituted with 1, 2, or 3 $R^{12}$.

**12.** The compound of any preceding claim wherein $R^{12}$ is independently at each occurrence selected from: halo, $C_{1-4}$ alkyl, or $-OR^{13}$, optionally $R^{12}$ is independently selected from: Cl, Br, F, $CF_3$, OMe, OEt, OPh, CN, $SO_2Me$, methyl, pyridinyl, or methylpyrazole.

**13.** The compound of any preceding claim wherein $R^{4a}$ is H, OH or F and $-L-(CR^cR^d)_n-R^4$ is selected from: $-CF_3$, -OH, $-NH_2$, $=CH_2$, -CN, -NHC(O)Me, phenyl, isoindoline, benzyl, $-CH_2$phenyl, - $CH_2$pyridinyl, $-CH_2$pyrazolyl, $-CH_2$dihydrobenzofuran, $-CH_2$imidazolyl, $-CH_2$benzodioxolanyl, - NHpyrazinyl, $-NHCH_2Ph$, $-NHCH_2CH_2Ph$, and $-NHCH_2CH_2CH_2Ph$; wherein any of the above cyclic groups is unsubstituted or substituted with 1, 2 or 3 groups selected from: Cl, Br, F, $CF_3$, OMe, OEt, - O-phenyl, -O-benzyl, CN, $SO_2Me$, methyl, pyridinyl, or methylpyrazole.

**14.** The compound of any preceding claim wherein $R^1$ is selected from substituted or unsubstituted: phenyl, or 5, 6 membered heteroaryl; wherein when substituted $R^1$ is substituted with 1, 2, or 3 groups selected from: =O, CN, -OH, or $-O-C_{1-6}$ alkyl, halo and $C_{1-6}$ alkyl.

**15.** The compound of claim 14 wherein $R^1$ is selected from: $-NMe_2$, -N(Me)i-Pr, -NH-cyclopropyl, cyclopropyl, phenyl, pyridinyl, pyridinonyl, pyrimidinyl, imidazolyl, pyrazolyl, oxazolyl, pyrollidinyl, fluoropyrollidinyl, azetidinyl, piperidinyl, piperazinyl, azepanyl, indoline, tetrahydronapthalenyl, or

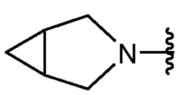 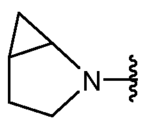 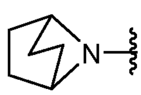 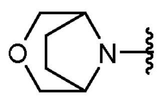 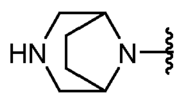

**16.** The compound of claim 14 or claim 15 wherein $R^1$ is selected from: phenyl, pyridinyl, or pyrollidinyl, wherein $R^1$ is unsubstituted or substituted with a group selected from: F, CN, -OH, $-OCF_3$, -OMe, Me, i-Pr, or $-CF_3$.

**17.** The compound of claim 1, wherein the compound is selected from:

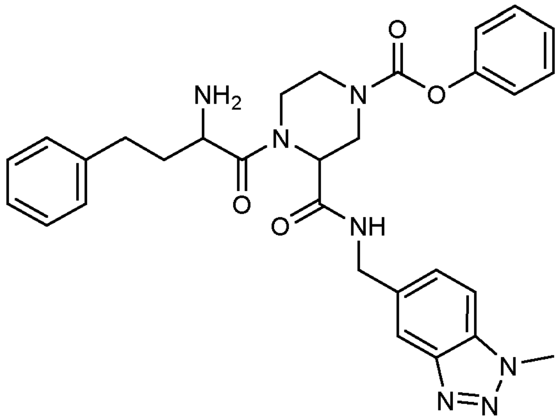

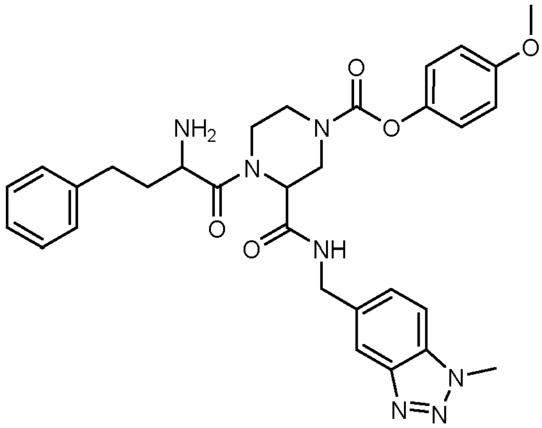

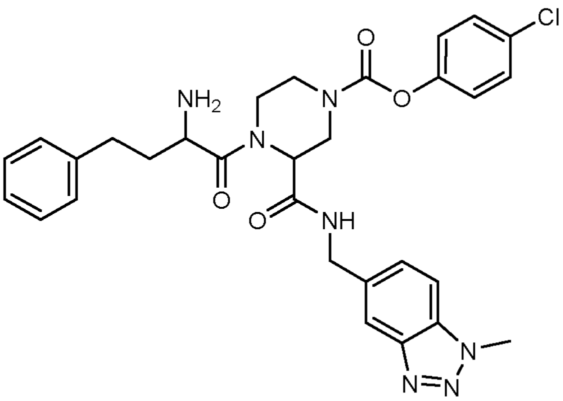

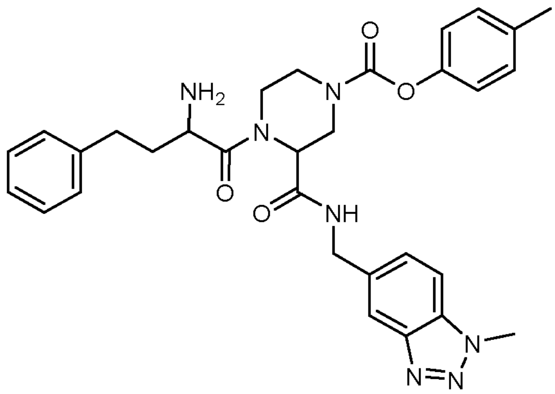

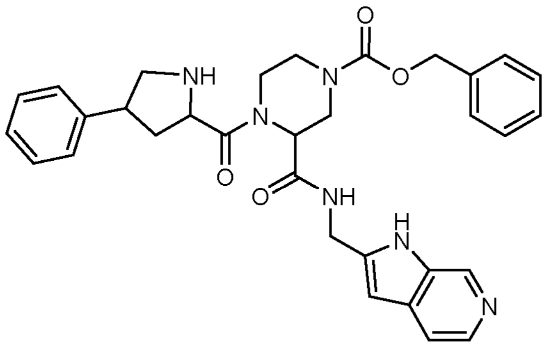

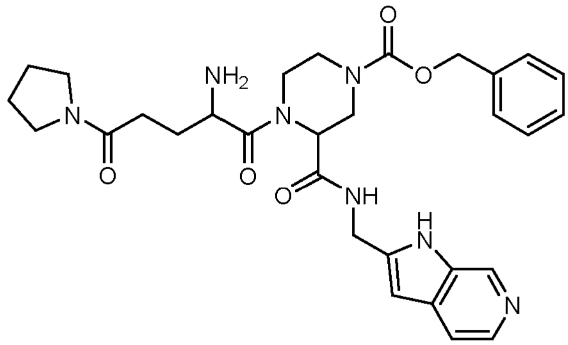

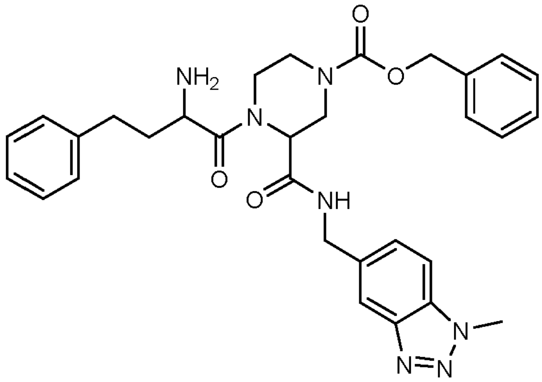

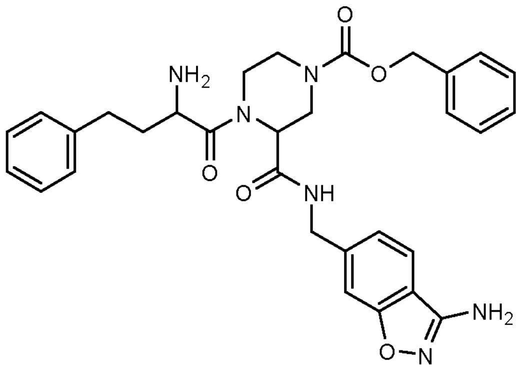

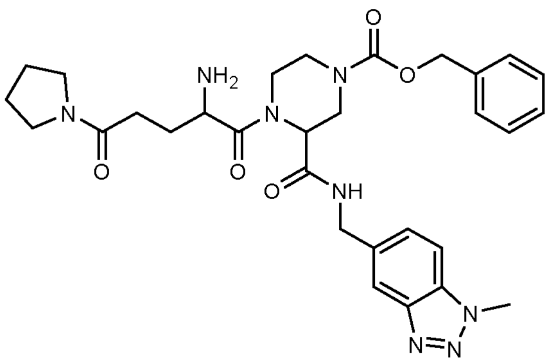

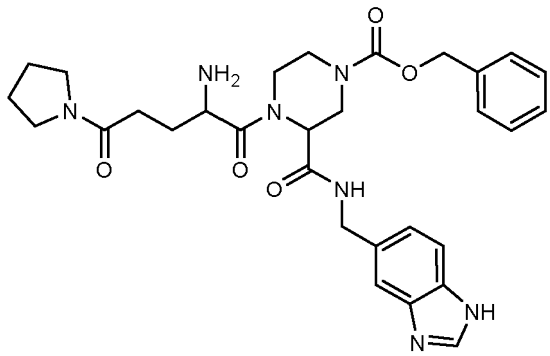

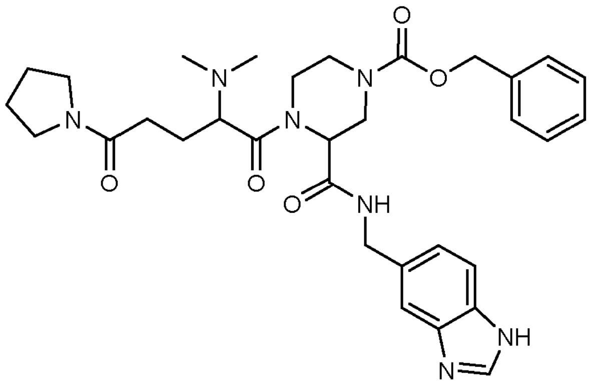

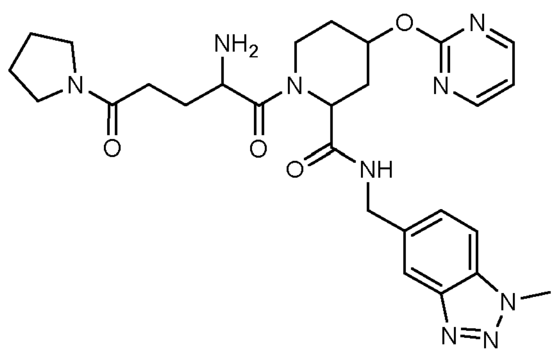

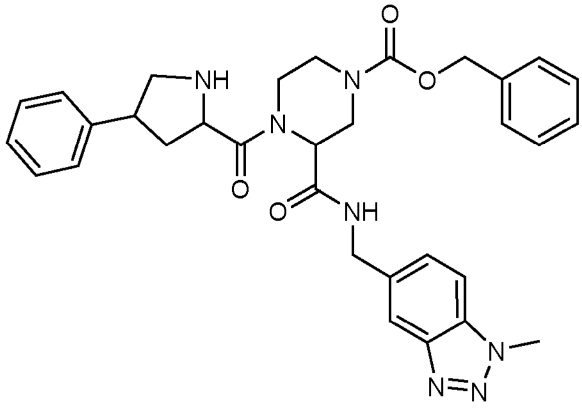

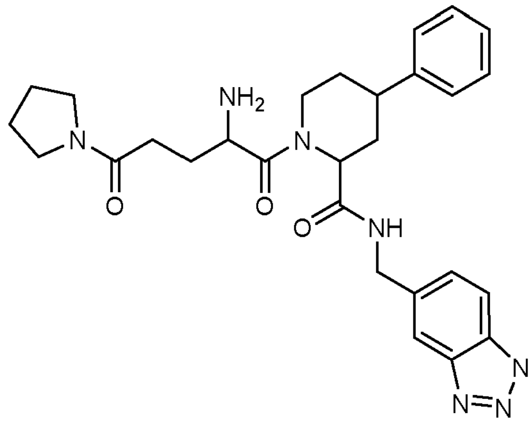

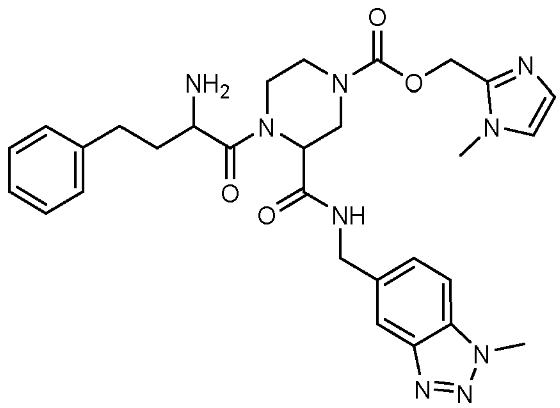

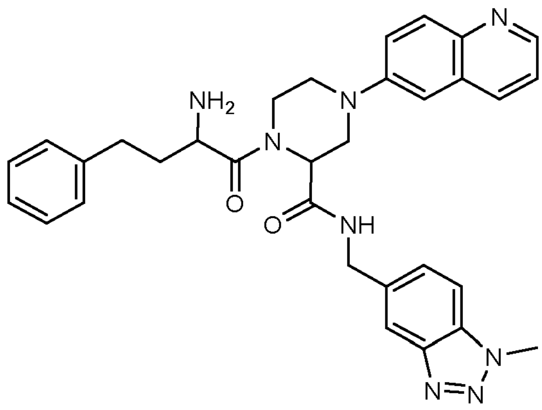

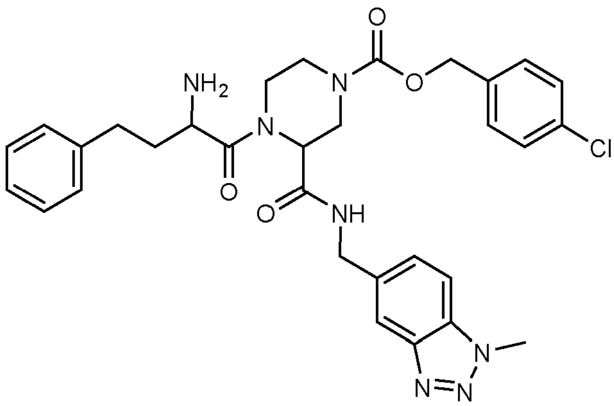

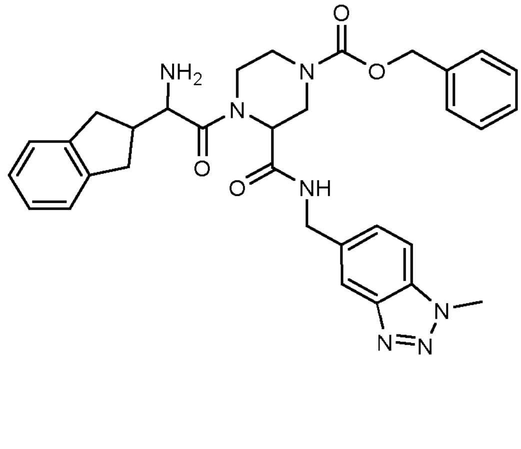

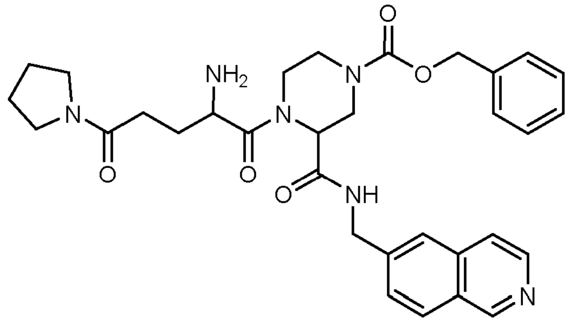

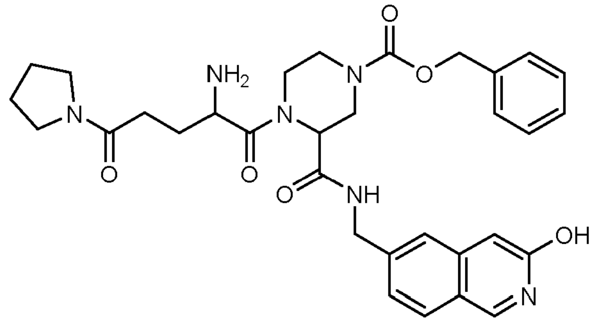

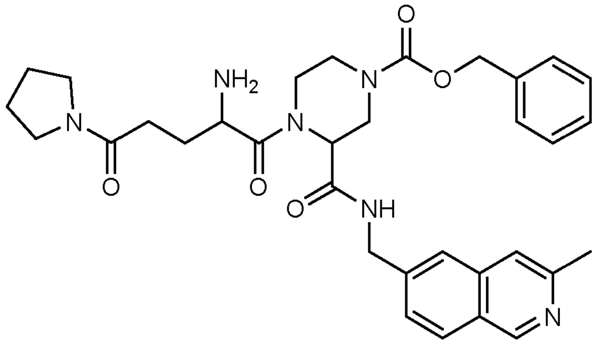

**18.** The compounds of any previous claim for use as a medicament.

**19.** The compound of any one of claims 1 to 17 for use in the treatment and/or prevention of a condition selected from the following or as a co-therapy in a treatment and/or prevention of a condition selected from: thrombosis, deep venous thrombosis, complex left-sided ablation (pulmonary vein isolation; VT ablation), reperfusion injury also know as ischaemia-reperfusion injury, transcatheter aortic valve replacement (TAVR) also known as transcatheter aortic valve implantation (TAVI), spinal or epidural anaesthesia, lumbar diagnostic puncture, thoracic surgery, abdominal surgery, major orthopaedic surgery, liver biopsy, transurethral prostate resection, kidney biopsy, renal insufficiency, liver diseases, endoscopy with biopsy, prostate or bladder biopsy, electrophysiological study or radiofrequency catheter ablation for supraventricular tachycardia (including left-sided ablation via single trans-septal puncture), angiography, pacemaker or implantable cardioverter defibrillator (ICD) implantation (unless complex anatomical setting, e.g. congenital heart disease), mechanical valve implantation, prosthetic valve implantation, myocardial infarction, angina pectoris (including unstable angina), reocclusions and restenoses after angioplasty or aortocoronary bypass, stroke, patients with atrial fibrillation to reduce their risk of stroke, patients with atrial fibriliation and chronic kidney disease, transitory ischaemic attacks, peripheral arterial occlusion disorders, deep venous thrombosis, pulmonary embolisms, deep venousmicrovascular disease, patients requiring extra corporeal membrane oxygenation (ECMO), patients requiring extra corporeal circulation such as coronary artery bypass grafting (CABG), disseminated intravascular coagulation (DIC), atherosclerosis, arthritis, thrombosis in patients with cancer, silent brain ischaemia, stroke, neurotraumatic disorder, neurological inflammatory disorders, medical procedures comprising contact with artificial surfaces including renal dialysis, other conditions where inhibition of FXIIa could be beneficial such as Alzheimer's disease, vascular dementia, macular degeneration, diabetic retinopathy, diabetic macular oedema, cerebral oedema in stroke, other causes of oedema, hereditary angioedema or acquired angioedema.

**20.** The compound for use of claim 19, wherein the condition is a condition associated with blood thickening, blood coagulation, or blood clot formulation, for example the condition may be thrombosis.

**21.** The compound of any one of claims 1 to 17 for use as an anticoagulant.

**22.** A pharmaceutical composition, wherein the composition comprises a compound of any one of claims 1 to 17 and pharmaceutically acceptable excipients.

**23.** The compound of any one of claims 1 to 17 for use to avoid or mitigate the contraindications of existing anticoagulant therapies, optionally selected from Dabigatran, Rivaroxaban, Apixaban, warfarin, Edoxaban and Betrixaban.

**Patentansprüche**

**1.** Verbindung nach Formel (I) und pharmazeutisch verträgliche Salze davon:

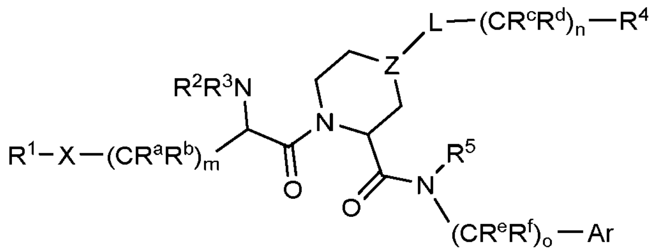

(I)

wobei

Z entweder N oder $CR^{4a}$ ist;
X entweder eine Bindung, -C(O)NH-, -C(O)O- oder -C(O)- ist;
L ausgewählt ist aus: Bindung, -O-, -C(O)O-, $-NR^6-$, $-C(O)NR^7-$ und $-SO_2-$;
Ar ein substituiertes oder unsubstituiertes 9- bis 10-gliedriges bicyclisches heteroaromatisches Ringsystem ist, wobei das bicyclische heteroaromatische Ringsystem ein aromatisches Kohlenwasserstoffringsystem mit zumindest einem Heteroatom innerhalb des Ringsystems ausgewählt aus O, N und S oder ein bicyclisches Ringsystem ist, das nicht vollständig aromatisch ist, sondern einen aromatischen Ring enthält, und wobei das zumindest eine Heteroatom innerhalb des aromatischen Rings oder des nichtaromatischen Rings vorhanden ist, wobei, wenn substituiert, Ar substituiert ist mit 1, 2 oder 3 Substituenten ausgewählt aus: Halo, $C_{1-6}$-Alkyl, $-OR^g$, $-NR^gR^h$ oder $C_{1-4}$-Alkyl substituiert durch $-NR^gR^h$;
m ausgewählt ist aus 0, 1, 2 oder 3;
n ausgewählt ist aus 0, 1, 2, 3 oder 4;
o ausgewählt ist aus 1 oder 2;
$R^1$ ausgewählt ist aus substituiertem oder unsubstituiertem: $-NR^8R^9$, 5- bis 10-gliedrigem carbocyclischem Ringsystem oder einem 5- bis 10-gliedrigen heterocyclischen Ringsystem; wobei, wenn substituiert, $R^1$ substituiert ist mit 1, 2 oder 3 Gruppen ausgewählt aus: =O, CN, -OH oder $-O-C_{1-6}$-Alkyl, Halo, $C_{1-6}$-Alkyl und $C_{1-6}$-Haloalkyl;
$R^2$ ausgewählt ist aus: H, $C_{1-6}$-Alkyl, $C_{1-6}$-Haloalkyl, Phenyl, Benzyl, $-C(O)R^{2a}$ und $-S(O_2)R^{2a}$; wobei $R^{2a}$ ausgewählt ist aus: $C_{1-6}$-Alkyl, Phenyl und Benzyl;
$R^3$ wie folgt ist:

(a) H oder $C_{1-6}$-Alkyl; oder
(b) $R^3$ zusammen mit einem von $R^a$ oder $R^b$ eine Bindung, eine $-CH_2$-- oder $-CH_2CH_2$--Gruppe bildet, die in einem 4-, 5- oder 6-gliedrigen Heterocycloalkylring resultiert, der die $-CH_2$-- oder - $CH_2CH_2$--Gruppe, das N-Atom, an das $R^3$ gebunden ist, das C-Atom, an das $R^a$ oder $R^b$ gebunden ist, und beliebige dazwischenliegende Atome umfasst; oder
(c) $R^3$ eine Bindung, eine $-CH_2$-- oder $-CH_2CH_2$--Gruppe mit einem Atom von $R^1$ bildet, wenn $R^1$ ein carbocyclisches Ringsystem oder ein heterocyclisches Ringsystem ist;

$R^4$ ausgewählt ist aus: H, $=CH_2$, -CN, Halo, $C_{1-4}$-Alkyl, $C_{1-4}$-Haloalkyl, $-OR^{10}$, $-NR^{10}R^{11}$, 6- bis 10-gliedrigem Aryl, 5- bis 10-gliedrigem Heteroaryl, wobei die 6- bis 10-gliedrige Aryl- oder 5-bis 10-gliedrige Heteroarylgruppe unsubstituiert oder substituiert ist mit 1, 2 oder 3 $R^{12}$;
$R^{4a}$ ausgewählt ist aus: H, -OH, Halo oder $C_{1-4}$-Alkyl;
$R^5$ H oder $C_{1-6}$-Alkyl ist;
$R^6$ H, $C_{1-6}$-Alkyl oder $-C(O)C_{1-6}$-Alkyl ist;
$R^7$ H oder $C_{1-6}$-Alkyl ist;
$R^8$ und $R^9$ unabhängig bei jedem Vorkommen ausgewählt sind aus: H, $C_{1-6}$-Alkyl, $C_{3-6}$-Cycloalkyl, Phenyl, $C_{1-4}$-Alkyl substituiert mit $-OR^i$ oder $C_{1-4}$-Alkyl substituiert mit Phenyl, oder wobei $R^8$ und $R^9$ zusammengenommen mit dem Atom, an das sie gebunden sind, einen 3- bis 8-gliedrigen Heterocycloalkylring bilden, der unsubstituiert oder substituiert ist mit: CN, Halo, $C_{1-6}$-Alkyl oder $-OR^i$
$R^{12}$ unabhängig bei jedem Vorkommen ausgewählt ist aus: Halo, $C_{1-4}$-Alkyl, $C_{1-4}$-Haloalkyl, - $OR^{13}$, -CN, -C(O)

$R^{10}$, =O, $SO_2R^{10}$, Benzyl, Phenyl, unsubstituiertem 5- oder 6-gliedrigem Heteroaryl oder methylsubstituiertem 5- oder 6-gliedrigem Heteroaryl;
$R^{10}$ und $R^{11}$ unabhängig bei jedem Vorkommen ausgewählt sind aus: H und $C_{1-4}$-Alkyl;
$R^{13}$ ausgewählt ist aus: H, $C_{1-4}$-Alkyl, $C_{1-4}$-Haloalkyl, Phenyl oder Benzyl;
$R^a$ und $R^b$ unabhängig bei jedem Vorkommen ausgewählt sind aus: H, $C_{1-4}$-Alkyl, $-OR^j$, oder wobei eines von $R^a$ oder $R^b$ zusammen mit $R^3$ eine Bindung, eine $-CH_2$-- oder-$CH_2CH_2$--Gruppe bildet, die in einem 4-, 5- oder 6-gliedrigen Heterocycloalkylring resultiert, der die $-CH_2$-- oder - $CH_2CH_2$--Gruppe, das N-Atom, an das $R^3$ gebunden ist, das C-Atom, an das $R^a$ oder $R^b$ gebunden sind, und beliebige dazwischenliegende Atome umfasst; und
$R^c$, $R^d$, $R^e$, $R^f$, $R^g$, $R^h$, $R^i$ und $R^j$ unabhängig bei jedem Vorkommen ausgewählt sind aus: H und $C_{1-4}$-Alkyl.

2. Verbindung nach Anspruch 1, wobei die Verbindung eine Verbindung der Formel (Ia) und pharmazeutisch verträgliche Salze davon ist:

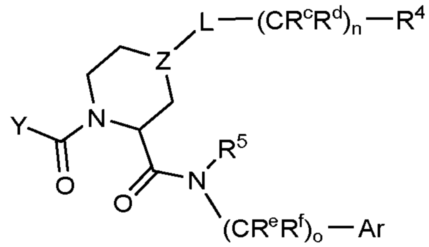

(Ia)

,

wobei

Y ausgewählt ist aus:

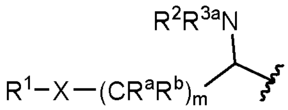

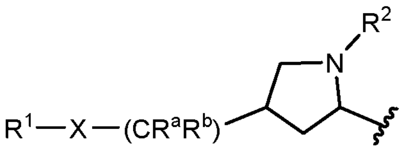

und

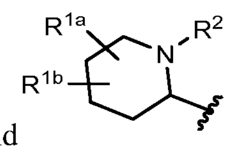

,

$R^{1a}$ und $R^{1b}$ zusammengenommen sind, um einen substituierten oder unsubstituierten Folgenden zu bilden: 5- oder 6-gliedrigen heteroaromatischen Ring oder einen Phenylring;
wobei, wenn der Ring, der aus $R^{1a}$ und $R^{1b}$ gebildet ist, substituiert ist, er mit 1, 2 oder 3 $R^z$-Gruppen substituiert ist, wobei $R^z$ unabhängig bei jedem Vorkommen ausgewählt ist aus: =O, CN, -OH oder $-O-C_{1-6}$-Alkyl, Halo und $C_{1-6}$-Alkyl;
$R^{3a}$ H oder $C_{1-6}$-Alkyl ist; und
m ausgewählt ist aus 1, 2 oder 3.

3. Verbindung nach einem vorhergehenden Anspruch, wobei L ausgewählt ist aus Bindung, -O- oder -C(O)O-.

4. Verbindung nach einem vorhergehenden Anspruch, wobei $R^2$ H ist und/oder $R^3$ H ist und/oder $R^5$ H ist.

5. Verbindung nach einem vorhergehenden Anspruch, wobei $R^1$ ausgewählt ist aus substituiertem oder unsubstituiertem: Phenyl oder einem 5- oder 6-gliedrigen Heterocycloalkylringsystem.

6. Verbindung nach einem vorhergehenden Anspruch, wobei Ar unsubstituiert oder substituiert ist mit Methyl, Chlor, -OMe, $-NH_2$ oder $-CH_2NH_2$.

7. Verbindung nach Anspruch 6, wobei Ar ausgewählt ist aus:

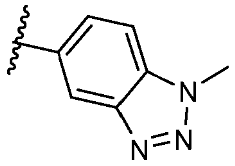 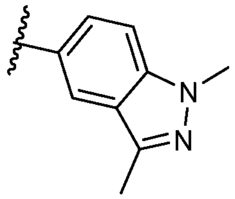 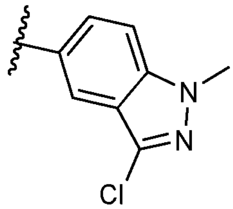 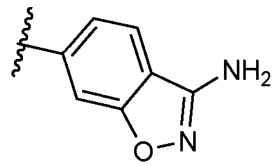

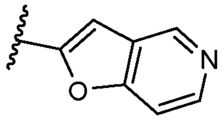 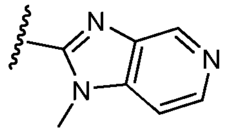 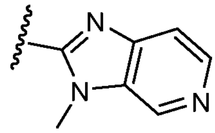 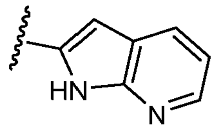

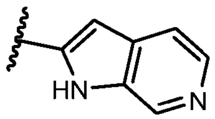 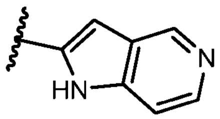 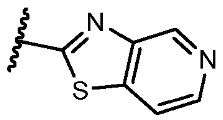 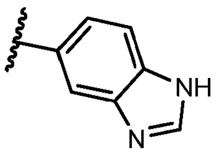 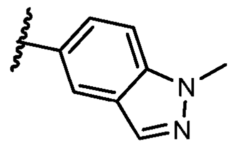 .

**8.** Verbindung nach Anspruch 6, wobei Ar Azaindol, Benzotriazol oder N-Methylbenzotriazol ist.

**9.** Verbindung nach einem vorhergehenden Anspruch, wobei $R^6$ H, Me oder -C(O)Me ist.

**10.** Verbindung nach einem vorhergehenden Anspruch, wobei $-L-(CR^cR^d)_n-$ ausgewählt ist aus: einer Bindung, $CH_2$, -NH-, $-NHCH_2-$, $-NH(CH_2)_2-$, $-NH(CH_2)_3-$, -N(Me)-, $-N(C(O)Me)CH_2-$, -NHC(O)-, $-NHC(O)CH_2-$, $-NHC(O)(CH_2)_2-$ oder $NHC(O)(CH_2)_3-$.

**11.** Verbindung nach einem vorhergehenden Anspruch, wobei $R^4$ ausgewählt ist aus: $=CH_2$, - CN, Halo, $C_{1-4}$-Alkyl, $C_{1-4}$-Haloalkyl, $-OR^{10}$, $-NR^{10}R^{11}$, Phenyl oder Napthalenyl, Pyridinyl, Pyrazinyl, Pyrazolyl, Imidazolyl, Dihydrobenzofuran, Benzodioxolanyl oder Isoindolinyl; wobei eine beliebige Gruppe, die cyclisch ist, unsubstituiert oder substituiert ist mit 1, 2 oder 3 $R^{12}$.

**12.** Verbindung nach einem vorhergehenden Anspruch, wobei $R^{12}$ unabhängig bei jedem Vorkommen ausgewählt ist aus: Halogen, $C_{1-4}$-Alkyl oder $-OR^{13}$, wobei optional $R^{12}$ unabhängig ausgewählt ist aus: Cl, Br, F, $CF_3$, OMe, OEt, OPh, CN, $SO_2Me$, Methyl, Pyridinyl oder Methylpyrazol.

**13.** Verbindung nach einem vorhergehenden Anspruch, wobei $R^{4a}$ H, OH oder F ist und $-L-(CR^cR^d)_n-R^4$ ausgewählt ist aus: $-CF_3$, -OH, $-NH_2$, $=CH_2$, -CN, -NHC(O)Me, Phenyl, Isoindolin, Benzyl, $-CH_2$-Phenyl, $-CH_2$-Pyridinyl, $-CH_2$-Pyrazolyl, $-CH_2$-Dihydrobenzofuran, $-CH_2$-Imidazolyl, $-CH_2$-Benzodioxolanyl, -NH-Pyrazinyl, $-NHCH_2Ph$, $-NHCH_2CH_2Ph$ und - $NHCH_2CH_2CH_2Ph$; wobei eine beliebige der obigen cyclischen Gruppen unsubstituiert oder substituiert ist mit 1, 2 oder 3 Gruppen ausgewählt aus: Cl, Br, F, $CF_3$, OMe, OEt, -O-Phenyl, -O-Benzyl, CN, $SO_2Me$, Methyl, Pyridinyl oder Methylpyrazol.

**14.** Verbindung nach einem vorhergehenden Anspruch, wobei $R^1$ ausgewählt ist aus substituiertem oder unsubstituiertem: Phenyl oder 5-, 6-gliedrigem Heteroaryl, wobei, wenn substituiert, $R^1$ substituiert ist mit 1, 2 oder 3 Gruppen ausgewählt aus: =O, CN, -OH oder $-O-C_{1-6}$-Alkyl, Halo und $C_{1-6}$-Alkyl.

**15.** Verbindung nach Anspruch 14, wobei $R^1$ ausgewählt ist aus: $-NMe_2$, -N(Me)i-Pr, -NH-Cyclopropyl, Cyclopropyl, Phenyl, Pyridinyl, Pyridinonyl, Pyrimidinyl, Imidazolyl, Pyrazolyl, Oxazolyl, Pyrollidinyl, Fluorpyrollidinyl, Azetidinyl, Piperidinyl, Piperazinyl, Azepanyl, Indolin, Tetrahydronapthalenyl oder

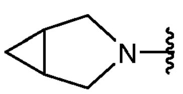 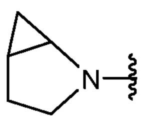 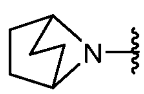 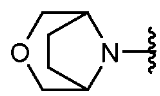 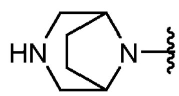 .

**16.** Verbindung nach Anspruch 14 oder Anspruch 15, wobei $R^1$ ausgewählt ist aus: Phenyl, Pyridinyl oder Pyrollidinyl, wobei $R^1$ unsubstituiert oder substituiert ist mit einer Gruppe ausgewählt aus: F, CN, -OH, $-OCF_3$, -OMe, Me, i-Pr oder $-CF_3$.

**17.** Verbindung nach Anspruch 1, wobei die Verbindung ausgewählt ist aus:

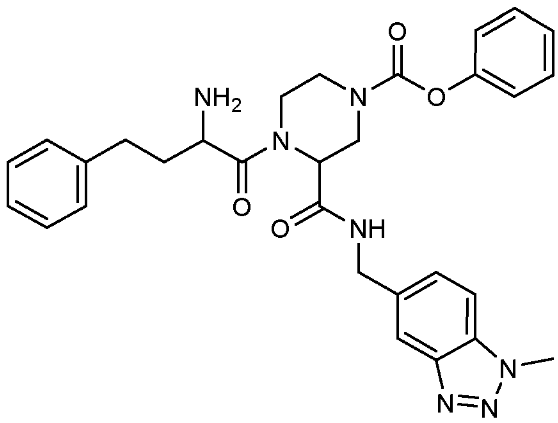

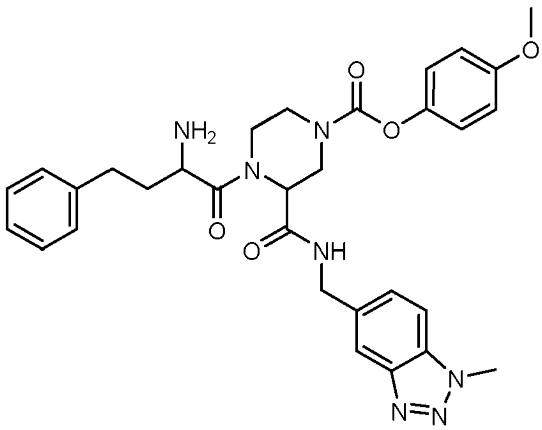

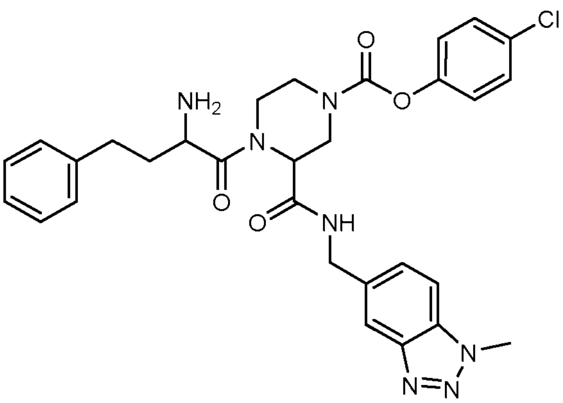

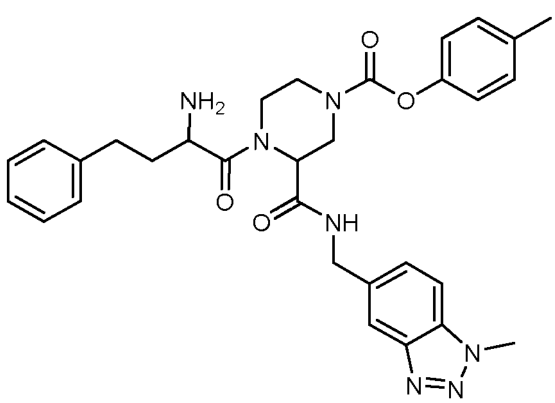

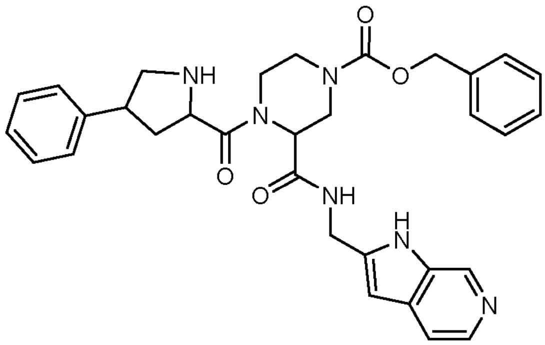

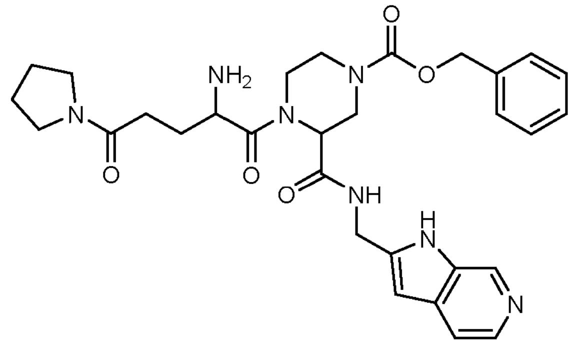

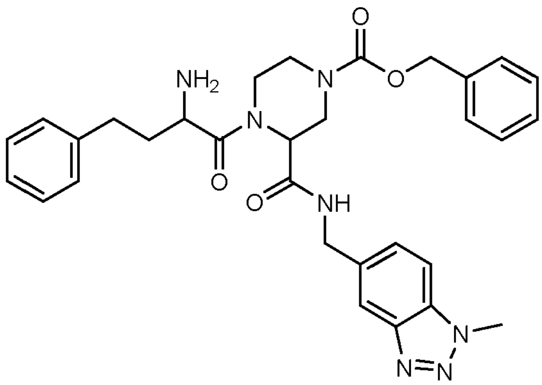

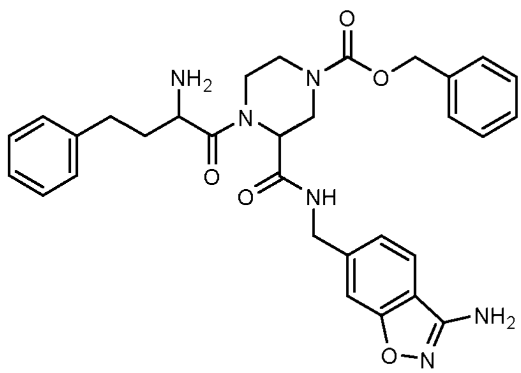

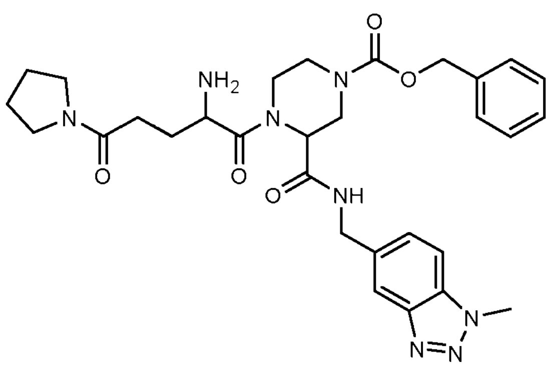

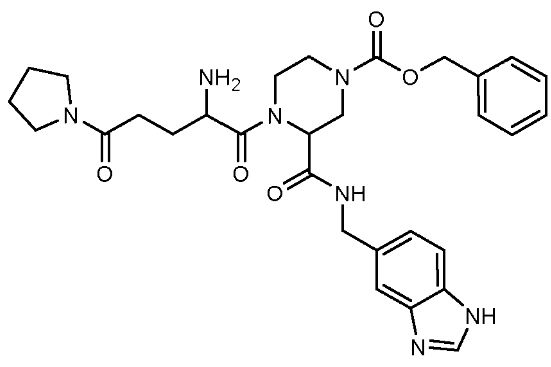

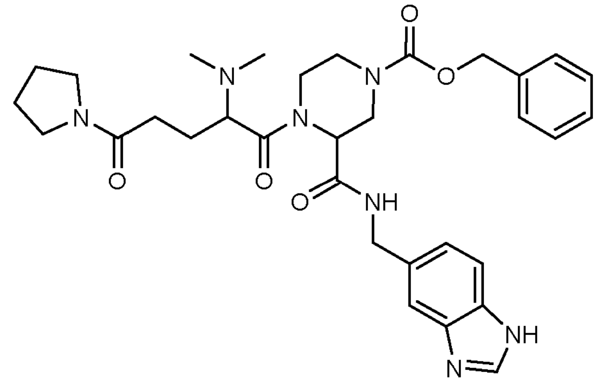

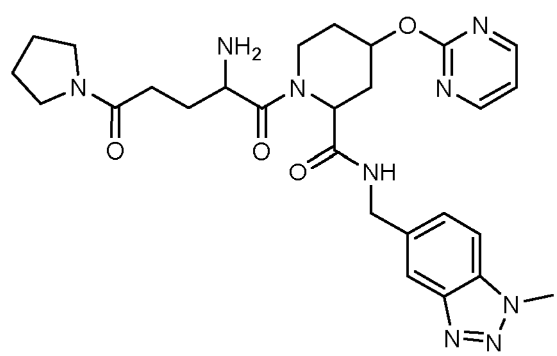

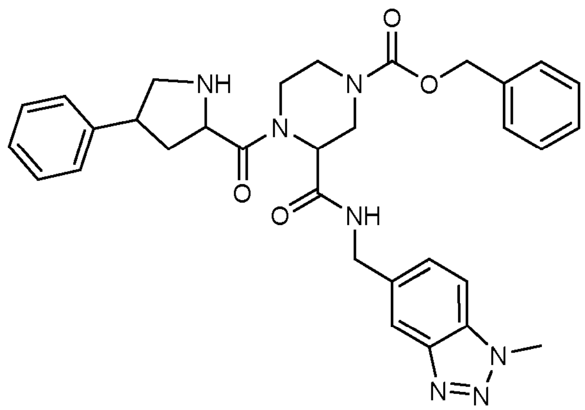

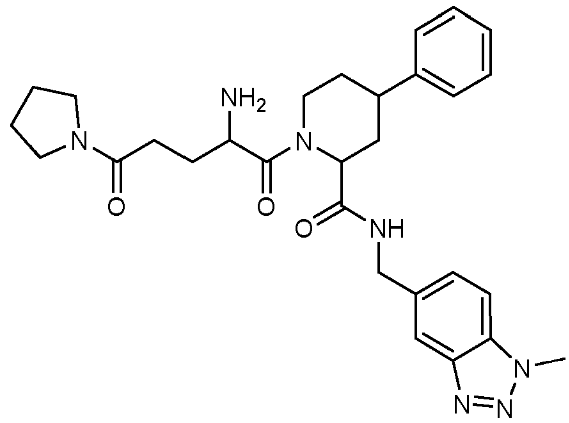

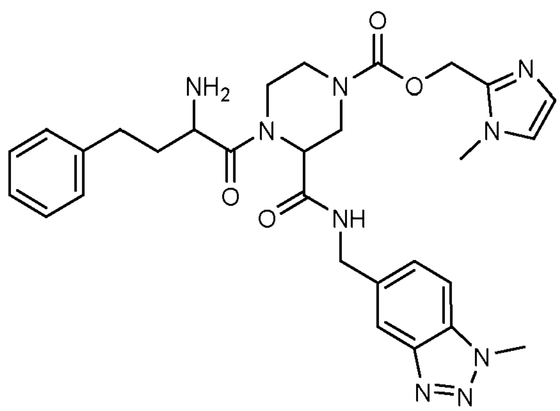

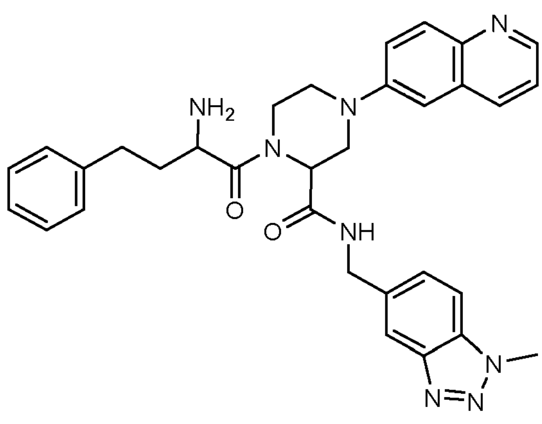

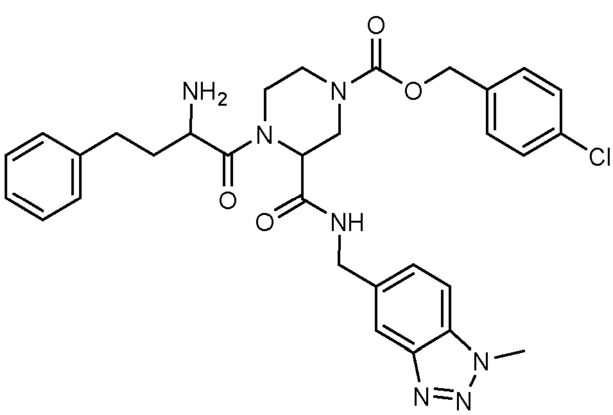

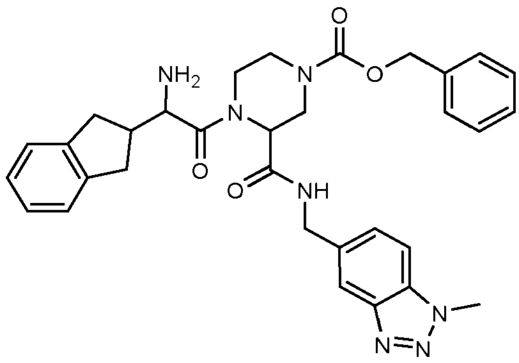

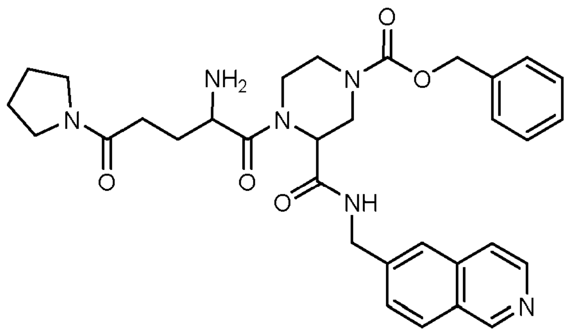

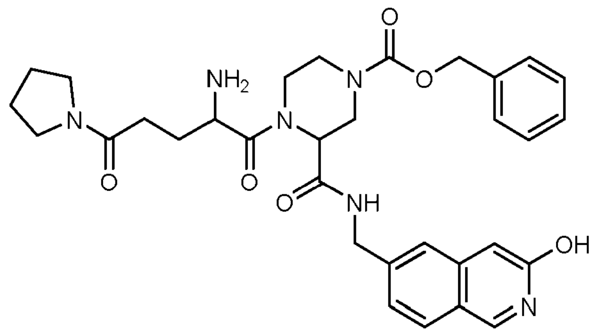

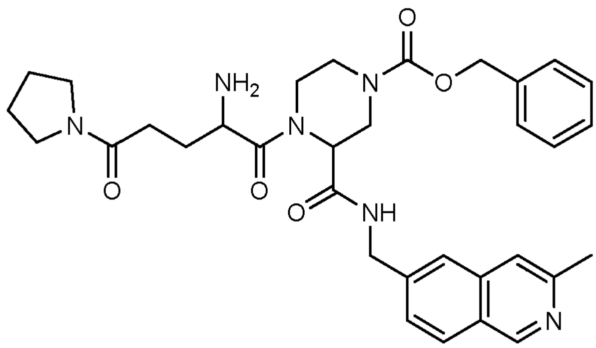

**18.** Verbindungen nach einem vorhergehenden Anspruch zur Verwendung als ein Medikament.

**19.** Verbindung nach einem der Ansprüche 1 bis 17 zur Verwendung bei der Behandlung und/oder Vorbeugung eines Zustands ausgewählt aus den folgenden oder als eine Co-Therapie bei einer Behandlung und/oder Vorbeugung eines Zustands ausgewählt aus: Thrombose, tiefer Venenthrombose, komplexer linksseitiger Ablation (Pulmonalvenenisolation; VT-Ablation), Reperfusionsverletzung, auch bekannt als Ischämie-Reperfusionsverletzung, Transkatheter-Aortenklappenersatz (TAVR), auch bekannt als Transkatheter-Aortenklappenimplantation (TAVI), Spinal- oder Epiduralanästhesie, Lumbaldiagnostikpunktion, Thoraxchirurgie, Bauchchirurgie, großer orthopädischer Chirurgie, Leberbiopsie, transurethraler Prostataresektion, Nierenbiopsie, Niereninsuffizienz, Lebererkrankungen, Endoskopie mit Biopsie, Prostata- oder Blasenbiopsie, elektrophysiologischer Studie oder Radiofrequenz-Katheterablation bei supraventrikulärer Tachykardie (beinhaltend linksseitige Ablation über eine einzelne trans-septale Punktion), Angiographie, Schrittmacher- oder implantierbarer Kardioverter-Defibrillator(ICD-)Implantation (sofern nicht komplexe anatomische Einstellung, z. B. angeborene Herzerkrankung), mechanischer Klappenimplantation, Klappenprothesenimplantation, Myokardinfarkt, Angina pectoris (beinhaltend instabile Angina), Reokklusionen und Restenosen nach Angioplastie oder Aortokoronar-Bypass, Schlaganfall, Patienten mit Vorhofflimmern, um ihr Schlaganfallrisiko zu reduzieren, Patienten mit Vorhofflimmern und chronischer Nierenerkrankung, transitorischen ischämischen Attacken, peripheren arteriellen Verschlussstörungen, tiefer venöser Thrombose, Lungenembolien, tiefer venöser mikrovaskulärer Erkrankung, Patienten, die extrakorporale Membranoxygenierung (ECMO) benötigen, Patienten, die extrakorporale Zirkulation benötigen, wie koronare Bypass-Transplantation (CABG), disseminierter intravaskulärer Koagulation (DIG), Atherosklerose, Arthritis, Thrombose bei Patienten mit Krebs, stiller Hirnischämie, Schlaganfall, neurotraumatischer Störung, neurologischen entzündlichen Störungen, medizinischen

Vorgängen, die Kontakt mit künstlichen Oberflächen umfassen, beinhaltend Nierendialyse, anderen Zuständen, bei denen Inhibition von FXIIa nützlich sein könnte, wie Alzheimer-Erkrankung, vaskulärer Demenz, Makuladegeneration, diabetischer Retinopathie, diabetischem Makulaödem, Hirnödem bei Schlaganfall, anderen Ursachen von Ödemen, erblichem Angioödem oder erworbenem Angioödem.

**20.** Verbindung zur Verwendung nach Anspruch 19, wobei der Zustand ein Zustand assoziiert mit Blutverdickung, Blutgerinnung oder Blutgerinnselbildung ist, zum Beispiel der Zustand Thrombose sein kann.

**21.** Verbindung nach einem der Ansprüche 1 bis 17 zur Verwendung als Antikoagulans.

**22.** Pharmazeutische Zusammensetzung, wobei die Zusammensetzung eine Verbindung nach einem der Ansprüche 1 bis 17 und pharmazeutisch verträgliche Hilfsstoffe umfasst.

**23.** Verbindung nach einem der Ansprüche 1 bis 17 zur Verwendung zum Vermeiden oder Mildern der Kontraindikationen von bestehenden gerinnungshemmenden Therapien, optional ausgewählt aus Dabigatran, Rivaroxaban, Apixaban, Warfarin, Edoxaban und Betrixaban.

## Revendications

**1.** Composé selon la formule (I) et sels pharmaceutiquement acceptables de celui-ci :

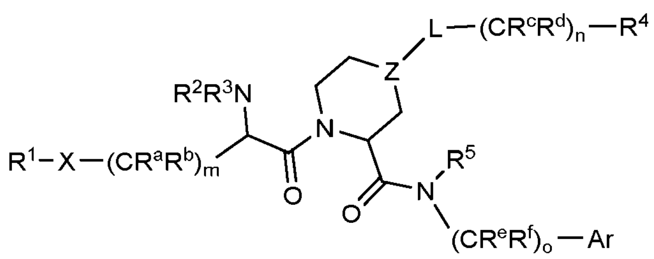

(I)

dans lequel

Z est N ou $CR^{4a}$ ;
X est une liaison, -C(O)NH-, -C(O)O- ou -C(O)- ;
L est choisi parmi : une liaison, -O-, -C(O)O-, $-NR^6-$, $-C(O)NR^7-$ et $-SO_2-$ ;
Ar est un système de cycle hétéroaromatique bicyclique à 9 à 10 chaînons substitué ou non substitué, ledit système de cycle hétéroaromatique bicyclique étant un système de cycle hydrocarboné aromatique avec au moins un hétéroatome dans le système de cycle choisi parmi O, N et S ou un système de cycle bicyclique qui n'est pas complètement aromatique mais qui contient un cycle aromatique et ledit au moins un hétéroatome est présent dans le cycle aromatique ou le cycle non aromatique, où, lorsqu'il est substitué, Ar est substitué par 1, 2 ou 3 substituants choisis parmi : un halogéno, un alkyle en $C_{1-6}$, $-OR^g$, $-NR^gR^h$ ou un alkyle en $C_{1-4}$ substitué par $-NR^gR^h$ ;
m est choisi parmi 0, 1, 2 ou 3 ;
n est choisi parmi 0, 1, 2, 3 ou 4 ;
o est choisi parmi 1 ou 2 ;
$R^1$ est choisi parmi : $-NR^8R^9$, un système de cycle carbocyclique à 5 à 10 chaînons ou un système de cycle hétérocyclique à 5 à 10 chaînons, chacun étant substitué ou non substitué ;
dans lequel, lorsqu'il est substitué, $R^1$ est substitué par 1, 2 ou 3 groupes choisis parmi : =O, CN, -OH ou -O-alkyle en $C_{1-6}$, un halogéno, un alkyle en $C_{1-6}$ et un halogénoalkyle en $C_{1-6}$ ;
$R^2$ est choisi parmi : H, un alkyle en $C_{1-6}$, un halogénoalkyle en $C_{1-6}$, un phényle, un benzyle, $-C(O)R^{2a}$ et $-S(O_2)R^{2a}$ ;
dans lequel $R^{2a}$ est choisi parmi : un alkyle en $C_{1-6}$, un phényle et un benzyle ;
$R^3$ est :

(a) H ou un alkyle en $C_{1-6}$ ; ou

(b) $R^3$ ensemble avec l'un de $R^a$ ou $R^b$ form une liaison, un groupe $-CH_2-$ ou $-CH_2CH_2-$résultant en un cycle hétérocycloalkyle de 4, 5 ou 6 chaînons comprenant le groupe $-CH_2-$ ou - $CH_2CH_2-$, l'atome N auquel $R^3$ est attaché, l'atome C auquel $R^a$ ou $R^b$ est attaché, et tout atome intermédiaire ; ou

(c) $R^3$ forme une liaison, un groupe $-CH_2-$ ou $-CH_2CH_2-$ avec un atome de $R^1$ lorsque $R^1$ est un système de cycle carbocyclique ou un système de cycle hétérocyclique ;

$R^4$ est choisi parmi : H, $=CH_2$, -CN, un halogéno, un alkyle en $C_{1-4}$, un halogénoalkyle en $C_{1-4}$, $-OR^{10}$, $-NR^{10}R^{11}$, un aryle à 6 à 10 chaînons, un hétéroaryle à 5 à 10 chaînons, ledit groupe aryle à 6 à 10 chaînons ou hétéroaryle à 5 à 10 chaînons est non substitué ou substitué par 1, 2 ou 3 $R^{12}$ ;
$R^{4a}$ est choisi parmi : H, -OH, un halogéno ou un alkyle en $C_{1-4}$ ;
$R^5$ est H ou un alkyle en $C_{1-6}$ ;
$R^6$ est H, un alkyle en $C_{1-6}$ ou -C(O)-alkyle en $C_{1-6}$ ;
$R^7$ est H ou un alkyle en $C_{1-6}$ ;
$R^8$ et $R^9$ sont indépendamment choisis, à chaque occurrence, parmi : H, un alkyle en $C_{1-6}$, un cycloalkyle en $C_{3-6}$, un phényle, un alkyle en $C_{1-4}$ substitué par $-OR^i$ ou un alkyle en $C_{1-4}$ substitué par un phényle, ou $R^8$ et $R^9$ pris ensemble avec l'atome auquel ils sont attachés forment un cycle hétérocycloalkyle à 3 à 8 chaînons, qui est non substitué ou substitué par : CN, un halogéno, un alkyle en $C_{1-6}$ ou $-OR^i$ ;
$R^{12}$ est indépendamment choisi, à chaque occurrence, parmi : un halogéno, un alkyle en $C_{1-4}$, un halogénoalkyle en $C_{1-4}$, $-OR^{13}$, -CN, $-C(O)R^{10}$, =O, $SO_2R^{10}$, un benzyle, un phényle, un hétéroaryle à 5 ou 6 chaînons non substitué ou un hétéroaryle à 5 ou 6 chaînons substitué par un méthyle ;
$R^{10}$ et $R^{11}$ sont indépendamment choisis, à chaque occurrence, parmi : H et un alkyle en $C_{1-4}$ ;
$R_{13}$ est choisi parmi : H, un alkyle en $C_{1-4}$, un halogénoalkyle en $C_{1-4}$, un phényle ou un benzyle ;
$R^a$ et $R^b$ sont indépendamment choisis, à chaque occurrence, parmi : H, un alkyle en $C_{1-4}$, $-OR^j$ ou l'un de $R^a$ ou $R^b$ ensemble avec $R^3$ forme une liaison, un groupe $-CH_2-$ ou$-CH_2CH_2-$résultant en un cycle hétérocycloalkyle à 4, 5 ou 6 chaînons comprenant le groupe $-CH_2-$ ou - $CH_2CH_2-$, l'atome N auquel $R^3$ est attaché, l'atome C auquel $R^a$ ou $R^b$ est attaché, et tout atome intermédiaire ; et
$R^c$, $R^d$, $R^e$, $R^f$, $R^g$, $R^h$, $R^i$ et $R^j$ sont indépendamment choisis, à chaque occurrence, parmi : H et un alkyle en $C_{1-4}$.

2. Composé de la revendication 1, dans lequel le composé est un composé de la formule (Ia) et des sels pharmaceutiquement acceptables de ceux-ci :

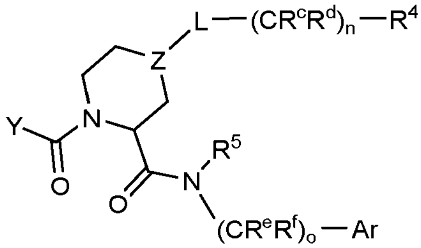

(Ia)

dans lequel

Y est choisi parmi :

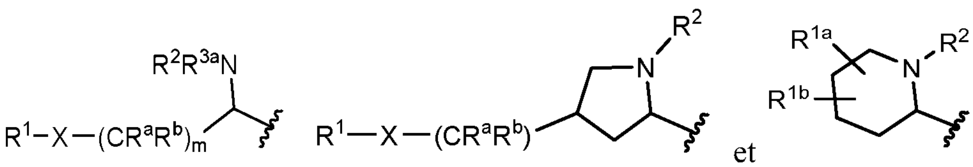

et

$R^{1a}$ et $R^{1b}$ pris ensemble forment : un cycle hétéroaromatique à 5 ou 6 chaînons ou un cycle phényle, chacun étant substitué ou non substitué ;
dans lequel, lorsque le cycle formé à partir de $R^{1a}$ et $R^{1b}$ est substitué, il est substitué par 1, 2 ou 3 groupes $R^z$, où $R^z$ est indépendamment choisi, à chaque occurrence, parmi : =O, CN, -OH ou -O-alkyle en $C_{1-6}$, un halogéno et un alkyle en $C_{1-6}$ ;
$R^{3a}$ est H ou un alkyle en $C_{1-6}$ ; et

m est choisi parmi 1, 2 ou 3.

**3.** Composé d'une quelconque revendication précédente, dans lequel L est choisi parmi une liaison, -O- ou -C(O)O-.

**4.** Composé d'une quelconque revendication précédente, dans lequel $R^2$ est H et/ou $R^3$ est H et/ou $R^5$ est H.

**5.** Composé d'une quelconque revendication précédente, dans lequel $R^1$ est choisi parmi : un phényle ou un système de cycle hétérocycloalkyle à 5 ou 6 chaînons, chacun étant substitué ou non substitué.

**6.** Composé d'une quelconque revendication précédente, dans lequel Ar est non substitué ou substitué par un méthyle, un chloro, -OMe, $-NH_2$ ou $-CH_2NH_2$.

**7.** Composé de la revendication 6, dans lequel Ar est choisi parmi :

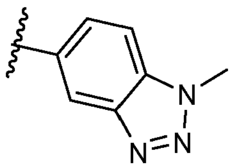
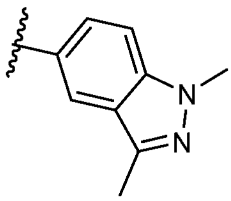
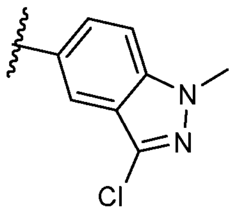
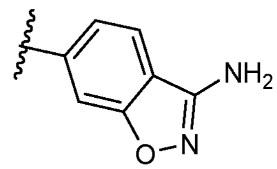
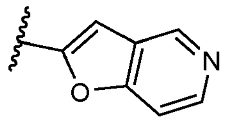
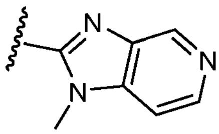
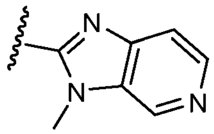
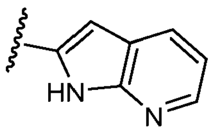
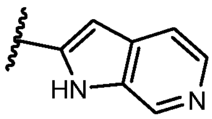
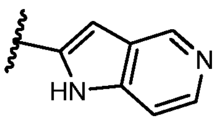
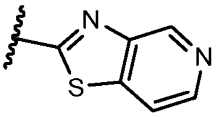
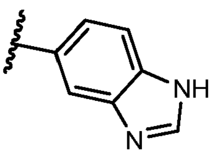
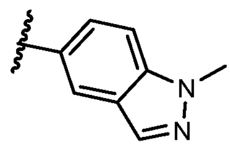
.

**8.** Composé de la revendication 6, dans lequel Ar est un azaindole, un benzotriazole ou un N-méthylbenzotriazole.

**9.** Composé d'une quelconque revendication précédente, dans lequel $R^6$ est H, Me ou - C(O)Me.

**10.** Composé d'une quelconque revendication précédente, dans lequel $-L-(CR^cR^d)_n-$ est choisi parmi : une liaison, $CH_2$, -NH-, $-NHCH_2-$, $-NH(CH_2)_2-$, $-NH(CH_2)_3-$, -N(Me)-, $-N(C(O)Me)CH_2-$, -NHC(O)-, $-NHC(O)CH_2-$, $-NHC(O)(CH_2)_2-$ ou $NHC(O)(CH_2)_3-$.

**11.** Composé d'une quelconque revendication précédente, dans lequel $R^4$ est choisi parmi : $=CH_2$, -CN, un halogéno, un alkyle en $C_{1-4}$, un halogénoalkyle en $C_{1-4}$, $-OR^{10}$, $-NR^{10}R^{11}$, un phényle ou un naphtalényle, un pyridinyle, un pyrazinyle, un pyrazolyle, un imidazolyle, un dihydrobenzofuranne, un benzodioxolanyle ou un isoindolinyle ; dans lequel tout groupe qui est cyclique est non substitué ou substitué par 1, 2 ou 3 $R^{12}$.

**12.** Composé d'une quelconque revendication précédente, dans lequel $R^{12}$ est indépendamment choisi, à chaque occurrence, parmi : un halogéno, un alkyle en $C_{1-4}$ ou $-OR^{13}$, éventuellement $R^{12}$ est indépendamment choisi parmi : Cl, Br, F, $CF_3$, OMe, OEt, OPh, CN, $SO_2Me$, un méthyle, un pyridinyle ou un méthylpyrazole.

**13.** Composé d'une quelconque revendication précédente, dans lequel $R^{4a}$ est H, OH ou F et $- L-(CR^cR^d)_n-R^4$ est choisi parmi : $-CF_3$, -OH, $-NH_2$, $=CH_2$, -CN, -NHC(O)Me, un phényle, un isoindoline, un benzyle, $-CH_2$phényle, $-CH_2$pyridinyle, $-CH_2$pyrazolyle, - $CH_2$dihydrobenzofuranne, $-CH_2$imidazolyle, $-CH_2$benzodioxolanyle, -NHpyrazinyle, $-NHCH_2Ph$, $-NHCH_2CH_2Ph$ et $-NHCH_2CH_2CH_2Ph$ ; dans lequel l'un quelconque des groupes cycliques cidessus est non substitué ou substitué par 1, 2 ou 3 groupes choisis parmi : Cl, Br, F, $CF_3$, OMe, OEt, -O-phényle, -O-benzyle, CN, $SO_2Me$, un méthyle, un pyridinyle ou un méthylpyrazole.

**14.** Composé d'une quelconque revendication précédente, dans lequel $R^1$ est choisi parmi un phényle ou un hétéroaryle à 5, 6 chaînons, chacun étant substitué ou non substitué ; dans lequel, lorsqu'il est substitué, $R^1$ est substitué par 1, 2 ou 3 groupes choisis parmi : =O, CN, -OH ou -O-alkyle en $C_{1-6}$, un halogéno et un alkyle en $C_{1-6}$.

**15.** Composé de la revendication 14, dans lequel $R^1$ est choisi parmi : $-NMe_2$, -N(Me)i-Pr, - NH-cyclopropyle, un cyclopropyle, un phényle, un pyridinyle, un pyridinonyle, un pyrimidinyle, un imidazolyle, un pyrazolyle, un oxazolyle, un pyrollidinyle, un fluoropyrollidinyle, un azétidinyle, un pipéridinyle, un pipérazinyle, un azépanyle, un indoline, un tétrahydronaptalényle, ou

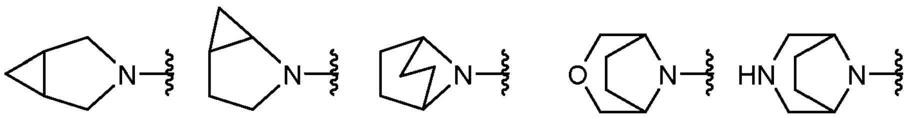

.

**16.** Composé selon la revendication 14 ou la revendication 15, dans lequel $R^1$ est choisi parmi : un phényle, un pyridinyle ou un pyrollidinyle, dans lequel $R^1$ est non substitué ou substitué par un groupe choisi parmi : F, CN, -OH, $-OCF_3$, -OMe, Me, i-Pr ou $-CF_3$.

**17.** Composé de la revendication 1, ledit composé étant choisi parmi :

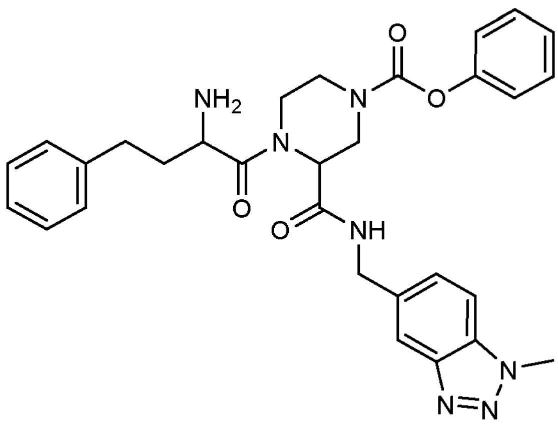

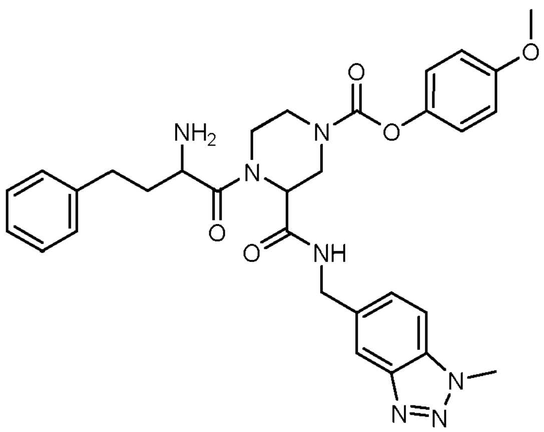

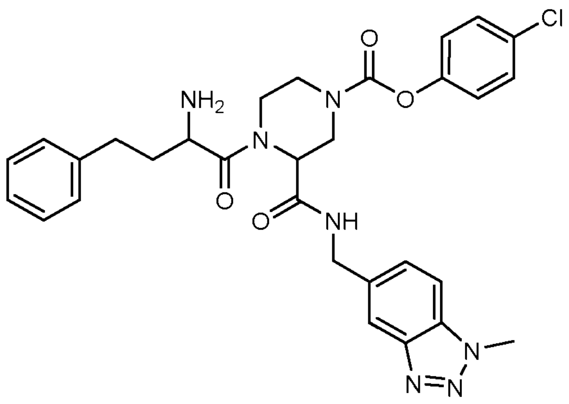

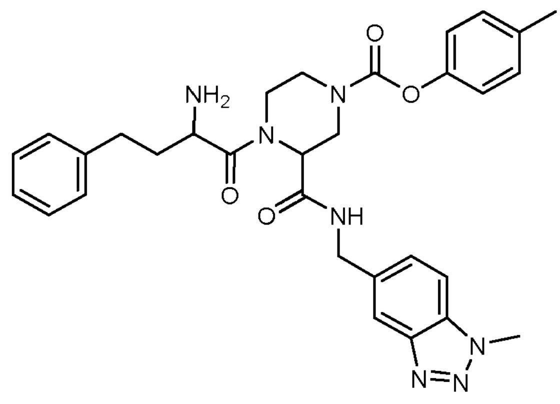

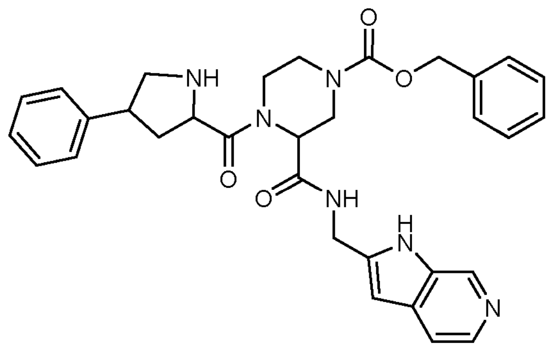

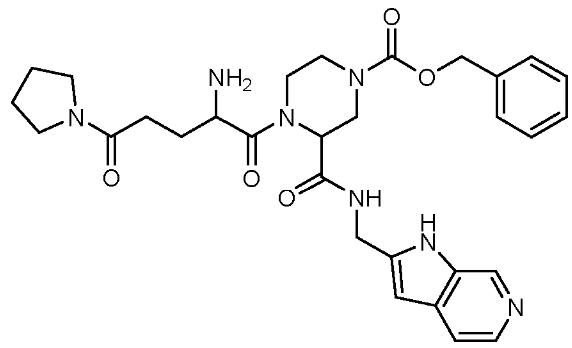

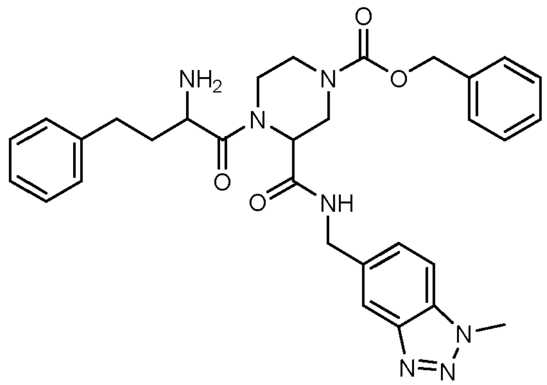

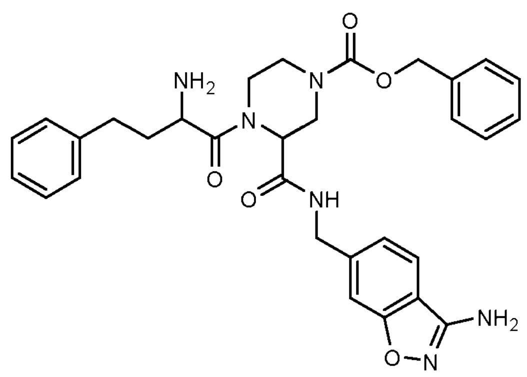

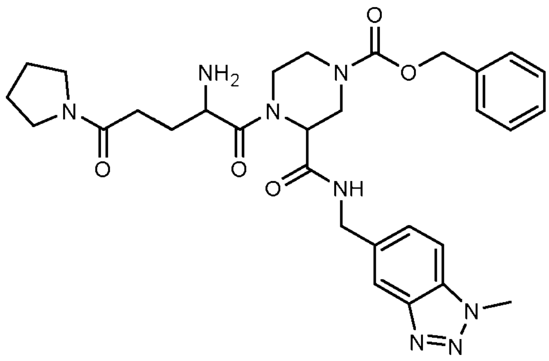

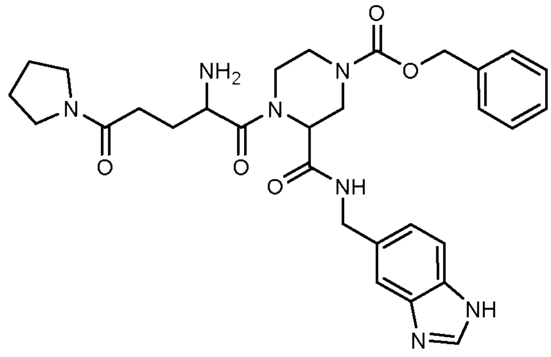

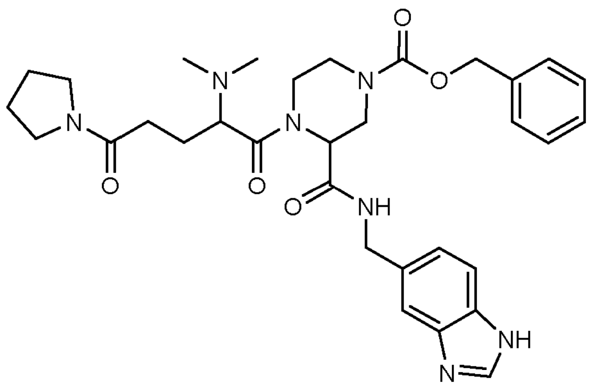

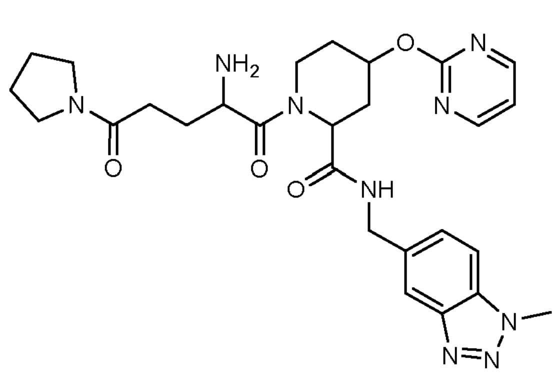

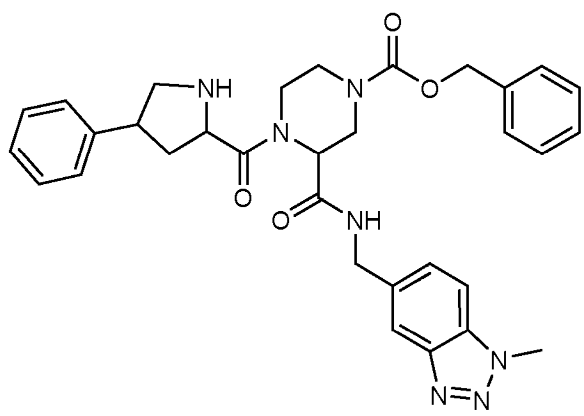

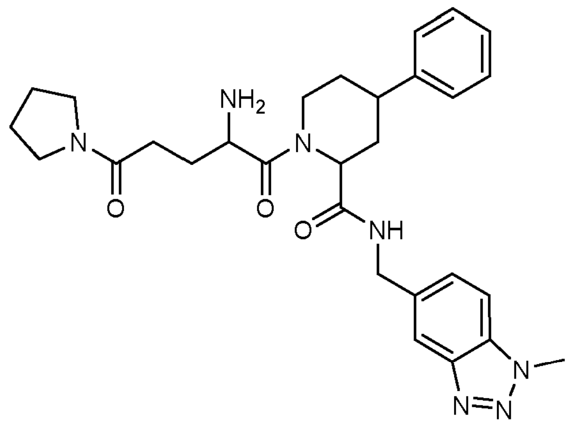

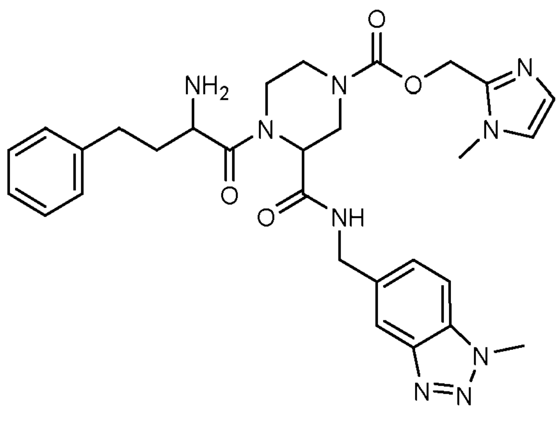

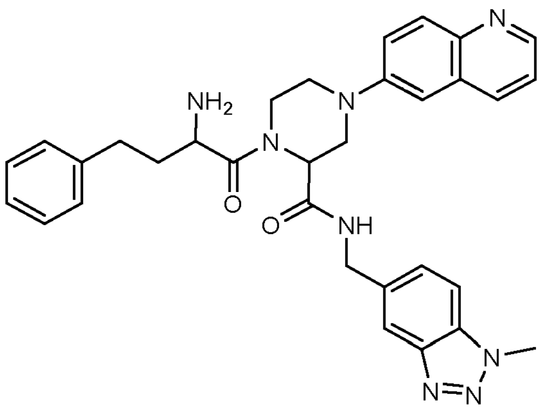

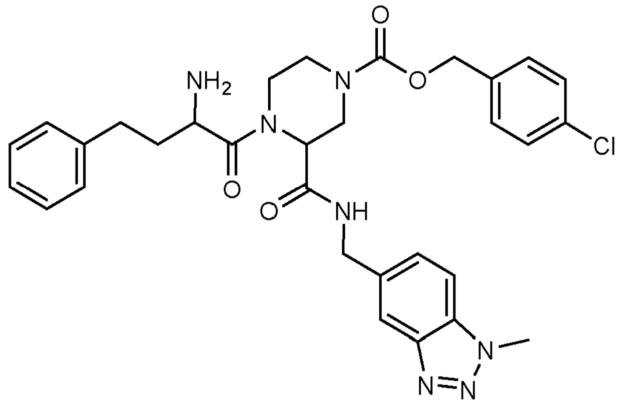

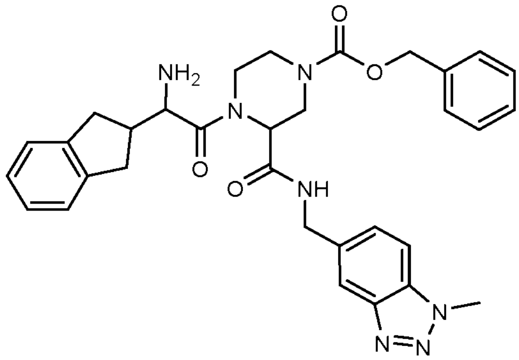

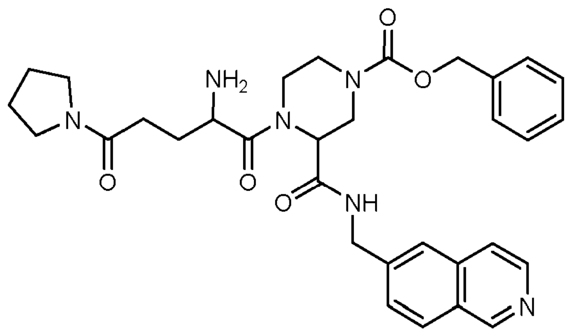

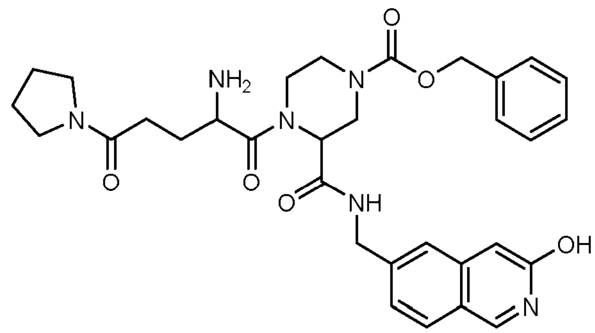

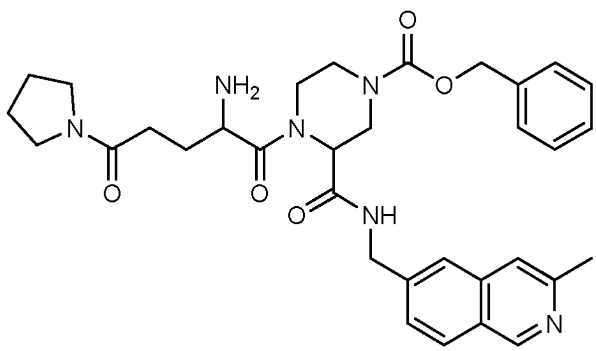

**18.** Composés d'une quelconque revendication précédente destinés à être utilisés en tant que médicament.

**19.** Composé de l'une quelconque des revendications 1 à 17, destiné à être utilisé dans le traitement et/ou la prévention d'une affection choisie parmi les suivantes ou en tant que co-thérapie dans le traitement et/ou la prévention d'une affection choisie parmi : la thrombose, la thrombose veineuse profonde, une ablation complexe du côté gauche (isolement des veines pulmonaires ; ablation de TV), une lésion de reperfusion, également connue sous le nom de lésion d'ischémie-reperfusion, le remplacement de la valve aortique par transcathéter (TAVR), également connu sous

le nom d'implantation de la valve aortique par transcathéter (TAVI), l'anesthésie rachidienne ou épidurale, une ponction lombaire diagnostique, une chirurgie thoracique, une chirurgie abdominale, une chirurgie orthopédique majeure, une biopsie hépatique, une résection transurétrale de la prostate, une biopsie rénale, une insuffisance rénale, des maladies du foie, une endoscopie avec biopsie, une biopsie de la prostate ou de la vessie, une étude électrophysiologique ou une ablation par radiofréquence par cathéter pour tachycardie supraventriculaire (y compris l'ablation du côté gauche par ponction trans-septale unique), une angiographie, une implantation d'un stimulateur cardiaque ou d'un défibrillateur automatique implantable (ICD) (sauf en cas de situation anatomique complexe, par exemple une cardiopathie congénitale), une implantation de valve mécanique, une implantation de valve prothétique, un infarctus du myocarde, une angine de poitrine (y compris une angine instable), des réocclusions et des resténoses après une angioplastie ou un pontage aortocoronarien, un accident vasculaire cérébral, des patients atteints de fibrillation auriculaire pour réduire leur risque d'accident vasculaire, des patients atteints de fibrillation auriculaire et de maladie rénale chronique, des attaques ischémiques transitoires, des troubles de l'occlusion artérielle périphérique, une thrombose veineuse profonde, des embolies pulmonaires, une maladie microvasculaire veineuse profonde, des patients nécessitant une oxygénation membranaire extracorporelle (OMEC), des patients nécessitant une circulation extracorporelle, telle que le pontage aortocoronarien (PAC), une coagulation intravasculaire disséminée (CID), l'athérosclérose, l'arthrite, la thrombose chez les patients atteints de cancer, l'ischémie cérébrale silencieuse, un accident vasculaire cérébral, un trouble neurotraumatique, des troubles inflammatoires neurologiques, des procédures médicales impliquant un contact avec des surfaces artificielles, y compris la dialyse rénale, autres affections pour lesquelles l'inhibition de FXIIa pourrait être bénéfique, telles que la maladie d'Alzheimer, la démence vasculaire, la dégénérescence maculaire, la rétinopathie diabétique, l'œdème maculaire diabétique, l'œdème cérébral dans les accidents vasculaires cérébraux, autres causes d'œdème, l'angio-oedème héréditaire ou l'angio-œdème acquis.

**20.** Composé destiné à être utilisé selon la revendication 19, ladite affection étant une affection associée à un épaississement du sang, à une coagulation sanguine ou à une formulation de caillot sanguin, ladite affection pouvant par exemple être la thrombose.

**21.** - Composé de l'une quelconque des revendications 1 à 17 destiné à être utilisé comme anticoagulant.

**22.** Composition pharmaceutique, dans laquelle la composition comprend un composé de l'une quelconque des revendications 1 à 17 et des excipients acceptables pharmaceutiquement.

**23.** Composé de l'une quelconque des revendications 1 à 17 destiné à être utilisé pour éviter ou atténuer les contre-indications de thérapies anticoagulantes existantes, éventuellement choisi parmi le dabigatran, le rivaroxaban, l'apixaban, la warfarine, l'edoxaban et le betrixaban.

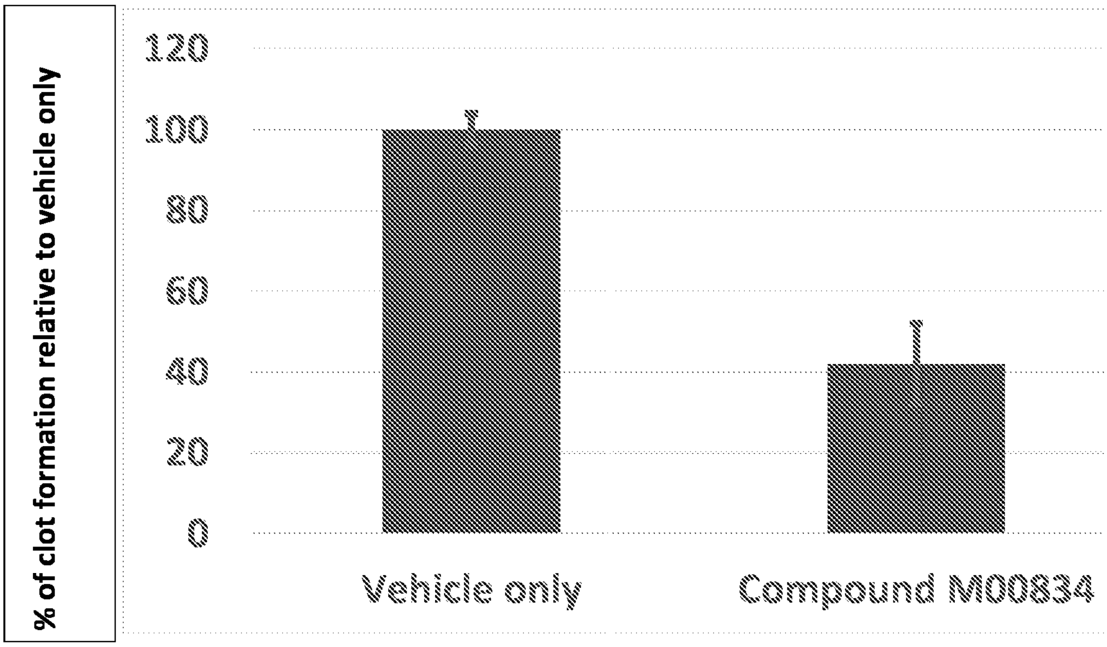
% of clot formation relative to vehicle only
120
100
80
60
40
20
0
Vehicle only
Compound M00834

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6087380 A
- US 7157456 B
- US 6413980 B
- US 7365205 B
- US 6376515 B
- WO 2012083436 A

### Non-patent literature cited in the description


- **HOLSTER IL** ; **VALKHOFF VE** ; **KUIPERS EJ** ; **TJWA ET**. New Oral Anticoagulants Increase Risk for Gastrointestinal Bleeding: A Systematic Review and Meta-analysis. *Gastroenterology*, July 2013, vol. 145 (1), 105-112
- **RENNE T** ; **POZGAJOVA M** ; **GRUNER S** ; **SCHUH K** ; **PAUER HU** ; **BURFEIND P** ; **GAILANI D** ; **NIESWANDT B.** Defective thrombus formation in mice lacking coagulation factor XII.. *J Exp Med*, 2005, vol. 202, 271-281
- **KLEINSCHNITZ C** ; **STOLL G** ; **BENDSZUS M** ; **SCHUH K** ; **PAUER HU** ; **BURFEIND P** ; **RENNE C** ; **GAILANI D** ; **NIESWANDT B** ; **RENNE T.** Targeting coagulation factor XII provides protection from pathological thrombosis in cerebral ischemia without interfering with hemostasis.. *J Exp Med*, 2006, vol. 203, 513-518
- **RENNE T** ; **NIESWANDT B** ; **GAILANI D.** The intrinsic pathway of coagulation is essential for thrombus stability in mice.. *Blood Cells Mol Dis*, 2006, vol. 36, 148-151
- **HAGEDORN I** ; **SCHMIDBAUER S** ; **PLEINES I** ; **KLEINSCHNITZ C** ; **KRONTHALER U** ; **STOLL G** ; **DICKNEITE G** ; **NIESWANDT B.** Factor XIIa inhibitor recombinant human albumin Infestin-4 abolishes occlusive arterial thrombus formation without affecting bleeding.. *Circulation*, 2010, vol. 121, 1510-1517
- **MATAFONOV A** ; **LEUNG PY** ; **GAILANI AE** ; **GRACH SL** ; **PUY C** ; **CHENG Q** ; **SUN MF** ; **MCCARTY OJ** ; **TUCKER EL** ; **KATAOKA H**. Factor XII inhibition reduces thrombus formation in a primate thrombosis model.. *Blood.*, 2014, vol. 123 (11), 1739-46
- **ZAKHAROVA et al.** *PLoS One.*, 17 February 2015, vol. 10 (2), e0116665
- **MAGNUS LARSSON et al.** A Factor XIIa Inhibitory Antibody Provides Thromboprotection in Extracorporeal Circulation Without Increasing Bleeding Risk. *Sci Transl Med*, 2014, vol. 6, 222-17
- **STAHL** ; **WERMUTH**. Handbook of Pharmaceutical Salts: Properties, Selection, and Use. Wiley-VCH, 2002
- **HALEBLIAN**. *J Pharm Sci*, August 1975, vol. 64 (8), 1269-1288
- **T.W. GREENE**. Protective Groups in Organic Synthesis. A. Wiley- Interscience Publication, 1981
- **P. J. KOCIENSKI**. Protecting groups. Georg Thieme Verlag, 1994
- **M. E. AULTON**. *Pharmaceuticals - The Science of Dosage Form Designs*, 1988